(19) Europäisches Patentamt
European Patent Office
Office européen des brevets

(11) **EP 4 056 128 B1**

(12) **EUROPEAN PATENT SPECIFICATION**

(45) Date of publication and mention
of the grant of the patent:
**09.10.2024 Bulletin 2024/41**

(21) Application number: **22171235.9**

(22) Date of filing: **23.01.2018**

(51) International Patent Classification (IPC):
*A61B 17/22* (2006.01)     *A61B 17/3203* (2006.01)
*A61M 1/00* (2006.01)     *A61B 17/00* (2006.01)

(52) Cooperative Patent Classification (CPC):
**A61B 17/32037; A61B 17/22; A61M 1/743;
A61M 1/84; A61M 1/85;** A61B 17/3203;
A61B 2017/22039; A61B 2017/22082;
A61B 2017/22084; A61B 2017/32035;
A61B 2217/005; A61B 2217/007; A61M 1/804

(54) **SYSTEMS FOR REMOVAL OF BLOOD AND THROMBOTIC MATERIAL**

SYSTEME ZUR ENTFERNUNG VON BLUT UND THROMBOTISCHEM MATERIAL

SYSTÈMES D'ÉLIMINATION DE DÉBRIS DE SANG ET DE THROMBUS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **23.01.2017 US 201762449572 P
04.10.2017 US 201762568240 P
13.11.2017 US 201762584986 P
22.01.2018 US 201815877200**

(43) Date of publication of application:
**14.09.2022 Bulletin 2022/37**

(62) Document number(s) of the earlier application(s) in
accordance with Art. 76 EPC:
**18741539.3 / 3 570 901**

(73) Proprietor: **Walk Vascular, LLC
Irvine, California 92614 (US)**

(72) Inventors:
 • **LOOK, David M.
 Newport Beach, 92660 (US)**
 • **CULBERT, Bradley S.
 Mission Viejo, 92692 (US)**

(74) Representative: **Lecomte & Partners
76-78, rue de Merl
2146 Luxembourg (LU)**

(56) References cited:
**US-A1- 2006 229 550     US-A1- 2015 327 875**

## Description

BACKGROUND OF THE INVENTION

Field of the Invention

The present disclosure pertains generally to medical devices and methods of their use. More particularly, the present invention pertains to aspiration and thrombectomy devices.

Description of the Related Art

Several devices and systems already exist to aid in the removal of thrombotic material (e.g., US 2015/327875 A1, US 2006/229550 A1).
Some simple aspiration tube type devices use vacuum syringes to extract thrombus into the syringe, simple flush-and-aspirate devices, more complex devices with rotating components the pull in, macerate and transport thrombotic material away from the distal tip using a mechanical auger, systems that use very high pressure to macerate the thrombus and create a venturi effect to flush the macerated material away.

All of the devices described above have limitations as a result of individual design characteristics. For example, simple aspiration catheters offer ease of use and rapid deployment but may become blocked or otherwise inoperable when faced with older, more organized thrombotic material. Such devices must be removed and cleared outside the body and then re-inserted into the vasculature, which lengthens the time needed for the procedure and increases the opportunity to kink the catheter shaft. Such kinks may reduce performance by decreasing the cross-sectional area of the catheter or may render the device inoperable.

Mechanical rotary devices use an auger to grab and carry the thrombus away from the target area. Some create transport force via vacuum bottles while others create differential pressure at the distal tip of the device with the auger acting as a low-pressure pump. These devices typically work slowly and offer the physician no feedback as to when the device should be advanced further into the lesion.

Flushing type devices include manual flush type devices in which the physician manipulates a hand-driven pump to provide flowing saline at the tip of the device to break up and aspirate the thrombus material, which may introduce performance variations based on the ability of the physician to consistently pump the device over the duration of the procedure. Flushing devices also include high pressure flushing devices that macerate the thrombus and then, using a vortex created by the high-pressure fluid, transport the emulsified thrombotic material to a collection bag. These devices are effective at removing all levels of thrombotic material, but the pressure created by the device is so great that its action against certain vessel walls may interrupt the heart muscle stimulation mechanism and create a bradycardia event in certain patients, sometimes requiring that a pacing lead be placed in the patient prior to use. Further, interacting with the thrombotic material outside of the catheter may allow loose material to escape the capture mechanism.

SUMMARY OF THE INVENTION

The Invention relates to a system for aspirating thrombus as defined in claim 1. Further embodiments of the invention are specified In the dependent claims. No methods of treatment or surgery are claimed.
In one embodiment of the present disclosure, a system for aspirating thrombus includes an aspiration catheter including an elongate shaft configured for placement within a blood vessel of a subject, a supply lumen and an aspiration lumen each extending within the shaft, the supply lumen having a proximal end and a distal end, and the aspiration lumen having a proximal end and an open distal end, and an opening at or near the distal end of the supply lumen, the opening configured to allow the injection of pressurized fluid into the aspiration lumen at or near the distal end of the aspiration lumen when the pressurized fluid is pumped through the supply lumen, a tubing set including tubing and having a distal end configured to couple to the aspiration lumen of the aspiration catheter and a proximal end configured to couple to a vacuum source, a tubing compression element configured to externally engage the tubing of the tubing set at a location between the proximal end of the tubing set and the distal end of the tubing set, and an activation interface configured to activate the tubing compression element to compress the tubing at the location between the proximal end of the tubing set and the distal end of the tubing set.

In another embodiment of the present disclosure, a system for aspirating thrombus includes an aspiration catheter including an elongate shaft configured for placement within a blood vessel of a subject, a supply lumen and an aspiration lumen each extending within the shaft, the supply lumen having a proximal end and a distal end, and the aspiration lumen having a proximal end and an open distal end, and an opening in a wall separating the supply lumen and the aspiration lumen, the opening at or adjacent the distal end of the supply lumen and in fluid communication with the interior of the aspiration lumen, the opening located proximally of the open distal end of the aspiration lumen, wherein the opening is configured to create a jet when pressurized fluid is pumped through the supply lumen of the aspiration catheter, a pressure sensor configured to be in fluid communication with the aspiration lumen of the aspiration catheter and to output a signal indicative of measured pressure, a tubing set including tubing and configured to extend proximally from the pressure sensor and to be in fluid communication with the aspiration lumen of the aspiration catheter, the tubing set having a proximal end configured to couple to a vacuum source, a tubing compression element configured to externally engage the tubing of the tubing set at a location between the proximal end of the tubing set and the pressure sensor, and an activation interface configured to activate the tubing compression element to compress the tubing at the location between the proximal end and the pressure sensor.

In yet another embodiment of the present disclosure, a system for aspirating thrombus includes an aspiration catheter including an elongate shaft configured for placement within a blood vessel of a subject, a supply lumen and an aspiration lumen each extending within the shaft, the supply lumen having a proximal end and a distal end, and the aspiration lumen having a proximal end and an open distal end, and an opening at or near the distal end of the supply lumen, the opening configured to allow the injection of pressurized fluid into the aspiration lumen at or near the distal end of the aspiration lumen when the pressurized fluid is pumped through the supply lumen, a pressure sensor configured to be in fluid communication with the aspiration lumen of the aspiration catheter and to output a signal indicative of measured pressure, a tubing set including tubing and configured to extend proximally from the pressure sensor and to be in fluid communication with the aspiration lumen of the aspiration catheter, the tubing set having a proximal end configured to couple to a vacuum source, a tubing compression element configured to externally engage the tubing of the tubing set at a location between the proximal end of the tubing set and the pressure sensor, and an activation interface configured to activate the tubing compression element to compress the tubing at the location between the proximal end and the pressure sensor.

In still another embodiment of the present disclosure, a system for aspirating thrombus includes an aspiration catheter including a supply lumen and an aspiration lumen, the supply lumen having a proximal end and a distal end, the aspiration lumen having a proximal end and an open distal end, an opening at or adjacent the distal end of the supply lumen, in fluid communication with the interior of the aspiration lumen, the opening located proximally of the open distal end of the aspiration lumen, wherein the opening is configured to create a jet when pressurized fluid is pumped through the supply lumen, a connector hydraulically coupled to the proximal end of the aspiration lumen, the connector having an interior cavity, a proximal end and a distal end, and a pressure sensor located within the interior cavity of the connector, and a measurement device configured to receive signals from the pressure sensor.

In yet another embodiment of the present disclosure, a system for aspirating thrombus includes an aspiration catheter including a supply lumen and an aspiration lumen, the supply lumen having a proximal end and a distal end, the aspiration lumen having a proximal end and an open distal end, an opening at or adjacent the distal end of the supply lumen, in fluid communication with the interior of the aspiration lumen, the opening located proximally of the open distal end of the aspiration lumen, wherein the opening is configured to create a jet when pressurized fluid is pumped through the supply lumen, a connector hydraulically coupled to the proximal end of the aspiration lumen, the connector having an interior cavity, a proximal end and a distal end, and wherein the opening includes a slit in a wall of a tubular structure which encloses the supply lumen.

In still another embodiment of the present disclosure, a method for removing thrombus from a patient includes providing an aspiration catheter including a supply lumen and an aspiration lumen, the supply lumen having a proximal end and a distal end, the aspiration lumen having a proximal end and an open distal end, an opening at or adjacent the distal end of the supply lumen, in fluid communication with the interior of the aspiration lumen, the opening located proximally of the open distal end of the aspiration lumen, wherein the opening is configured to create a jet when pressurized fluid is pumped through the supply lumen, a connector hydraulically coupled to the proximal end of the aspiration lumen, the connector having an interior cavity, a proximal end and a distal end, and a pressure sensor coupled to the proximal end of the supply lumen, coupling or causing to couple the supply lumen of the aspiration catheter to a fluid source, coupling or causing to couple the aspiration lumen of the aspiration catheter to a vacuum source, coupling or causing to couple a pump for injecting fluid from the fluid source through the supply lumen and through the opening into the aspiration lumen, providing a control unit configured to adjust the settings on the pump, and setting the pump with the control unit such that an input pressure of the supply lumen is between about 650 pounds per square inch and about 1200 pounds per square inch.

In yet another embodiment of the present disclosure, a system for aspirating thrombus includes an aspiration catheter including a supply lumen and an aspiration lumen, the supply lumen having a proximal end and a distal end, the aspiration lumen having a proximal end and an open distal end, an opening at or adjacent the distal end of the supply lumen, in fluid communication with the interior of the aspiration lumen, the opening located proximally of the open distal end of the aspiration lumen, wherein the opening is configured to create a jet when pressurized fluid is pumped through the supply lumen, and a connector hydraulically coupled to the proximal end of the aspiration lumen, the connector having an interior cavity having an inner surface, a proximal end and a distal end, wherein the connector includes a first sideport communicating with the interior cavity of the connector and in fluid communication with the aspiration lumen of the aspiration catheter, and wherein the first sideport is the nearest significant interruption of the inner surface to the distal end of the connector.

In still another embodiment of the present disclosure, a method for removing thrombus from a patient includes providing an aspiration catheter including a supply lumen and an aspiration lumen, the supply lumen having a proximal end and a distal end, the aspiration lumen having a proximal end and an open distal end, an opening at or adjacent the distal end of the supply lumen, in fluid communication with the interior of the aspiration lumen, the opening located proximally of the open distal end of the aspiration lumen, wherein the opening is configured to create a jet when pressurized fluid is pumped through the supply lumen, and a connector hydraulically coupled to the proximal end of the aspiration lumen, the connector having an interior cavity, a proximal end and a distal end, placing the distal end of a guiding catheter into a blood vessel, the guiding catheter having an inner lumen configured for placement of the aspiration catheter, placing the aspiration catheter through the inner lumen of the guiding catheter and into the blood vessel such that a distal end of the aspiration catheter is adjacent a thrombus, coupling or causing to couple the supply lumen of the aspiration catheter to a fluid source, coupling or causing to couple the aspiration lumen of the aspiration catheter to a vacuum source, coupling or causing to couple a first pump for injecting fluid from the fluid source through the supply lumen and through the opening into the aspiration lumen, causing an injection of fluid from the fluid source through the supply lumen of the aspiration catheter via the first pump with the vacuum source actively coupled to the aspiration lumen, determining that aspiration of the thrombus through the aspiration lumen of the aspiration catheter is not occurring at a desired thrombus aspiration rate, and injecting an injectate through the inner lumen of the guiding catheter and into the blood vessel to increase the thrombus aspiration rate.

In yet another embodiment of the present disclosure, a method for removing thrombus from a patient includes providing an aspiration catheter including a supply lumen and an aspiration lumen, the supply lumen having a proximal end and a distal end, the aspiration lumen having a proximal end and an open distal end, an opening at or adjacent the distal end of the supply lumen, in fluid communication with the interior of the aspiration lumen, the opening located proximally of the open distal end of the aspiration lumen, wherein the opening is configured to create a jet when pressurized fluid is pumped through the supply lumen, and a connector hydraulically coupled to the proximal end of the aspiration lumen, the connector having an interior cavity, a proximal end and a distal end, placing the distal end of a guiding catheter into a blood vessel, the guiding catheter having an inner lumen configured for placement of the aspiration catheter, placing the aspiration catheter through the inner lumen of the guiding catheter and into the blood vessel such that a distal end of the aspiration catheter is adjacent a thrombus, coupling or causing to couple the supply lumen of the aspiration catheter to a fluid source, coupling or causing to couple the supply lumen of the aspiration catheter to a fluid source, coupling or causing to couple a first pump for injecting fluid from the fluid source through the supply lumen and through the opening into the aspiration lumen, causing an injection of fluid from the fluid source through the supply lumen of the aspiration catheter via the first pump with the vacuum source actively coupled to the aspiration lumen, determining that aspiration of the thrombus through the aspiration lumen of the aspiration catheter is not occurring at a desired thrombus aspiration rate, and rotating the guiding catheter within the blood vessel to increase the thrombus aspiration rate.

In still another embodiment of the present disclosure, a method for removing thrombus from a patient includes providing an aspiration catheter including a supply lumen and an aspiration lumen, the supply lumen having a proximal end and a distal end, the aspiration lumen having a proximal end and an open distal end, an opening at or adjacent the distal end of the supply lumen, in fluid communication with the interior of the aspiration lumen, the opening located proximally of the open distal end of the aspiration lumen, wherein the opening is configured to create a jet when pressurized fluid is pumped through the supply lumen, and a connector hydraulically coupled to the proximal end of the aspiration lumen, the connector having an interior cavity, a proximal end and a distal end, placing the aspiration catheter into a blood vessel such that a distal end of the aspiration catheter is adjacent a thrombus, coupling or causing to couple the supply lumen of the aspiration catheter to a fluid source, coupling a first port of a four-way stopcock to the aspiration lumen of the aspiration catheter, coupling a second port of the four-way stopcock to a pressure sensor, the pressure sensor configured to send a signal to a controller, coupling a third port of the four-way stopcock to a vacuum source, coupling or causing to couple a pump between the fluid source and the aspiration lumen, causing an injection of fluid from the fluid source through the supply lumen of the aspiration catheter via the pump with the vacuum source actively coupled to the aspiration lumen, wherein the four-way stopcock is in a first state such that the pressure sensor, the aspiration lumen, and the vacuum source are all in fluid communication, wherein the controller is configured to stop the pump when the pressure sensor sends signals indicative of the pressure sensor not being in fluid communication with the vacuum source, and adjusting the four-way stopcock to a second state such that the pressure sensor remains in fluid communication with the vacuum source, but each of the pressure sensor and the vacuum source is no longer in fluid communication with the aspiration lumen, such that the controller maintains operation of the pump while the aspiration lumen is not in fluid communication with the vacuum source.

In yet another embodiment of the present disclosure, a first connector configured for removable connection proximal to an aspiration catheter, the aspiration catheter including a supply lumen and an aspiration lumen, the supply lumen having a proximal end and a distal end, the aspiration lumen having a proximal end and an open distal end, an opening at or adjacent the distal end of the supply lumen in fluid communication with the interior of the aspiration lumen, the opening located proximally of the open distal end of the aspiration lumen, wherein the opening is configured to create a jet when pressurized fluid is pumped through the supply lumen, and a second connector hydraulically coupled to the proximal end of the aspiration lumen, the second connector having an interior cavity, the first connector including a body having an interior, a distal end including a connection configured to sealably couple to the proximal end of the aspiration lumen of the aspiration catheter, a proximal end including an openable and closable seal configured for sealing over a guidewire, an aspiration port in fluid communication with an interior of the body and configured to couple to a vacuum source, and a pressure sensor in fluid communication with the interior of the body.

In still another embodiment of the present disclosure, a system for aspirating thrombus includes an aspiration catheter including a supply lumen and an aspiration lumen, the supply lumen having a proximal end, a distal end and a wall, the aspiration lumen having a proximal end and an open distal end, an orifice at or adjacent the distal end of the supply lumen, in fluid communication with the interior of the aspiration lumen, the orifice located proximally of the open distal end of the aspiration lumen, wherein the orifice is configured to create a jet when pressurized fluid is pumped through the supply lumen when a distal end of the aspiration catheter is immersed within an aqueous environment, and a first connector hydraulically coupled to the proximal end of the aspiration lumen, and a pressure sensor having an internal passageway and including a distal connector configured to hydraulically couple to the first connector, a proximal connector configured to couple to a vacuum source, and a valve disposed between the distal connector and the proximal connector, the valve having an open state and a closed state.

In yet another embodiment of the present disclosure, a method for removing thrombus from a patient includes providing an aspiration catheter including a supply lumen and an aspiration lumen, the supply lumen having a proximal end, a distal end and a wall, the aspiration lumen having a proximal end and an open distal end, an orifice at or adjacent the distal end of the supply lumen, in fluid communication with the interior of the aspiration lumen, the orifice located proximally of the open distal end of the aspiration lumen, wherein the orifice is configured to create a jet when pressurized fluid is pumped through the supply lumen when a distal end of the aspiration catheter is immersed within an aqueous environment, and a first connector hydraulically coupled to the proximal end of the aspiration lumen, providing a pressure sensor having an internal passageway and including a distal connector configured to hydraulically couple to the first connector, a proximal connector configured to couple to a vacuum source, and a valve disposed between the distal connector and the proximal connector, the valve having an open state and a closed state, coupling the distal connector of the pressure sensor to the first connector of the aspiration catheter, coupling the proximal connector of the pressure sensor to a vacuum source, coupling the supply lumen of the aspiration catheter to a pump having control circuitry, the control circuitry capable of receiving a signal from the pressure sensor, inserting at least a distal portion of the aspiration catheter into the vasculature of a subject near or adjacent a thrombus, and changing the valve from one of the open state and closed state to the other of the open state and closed state such that a change in pressure may be detected by the control circuitry.

BRIEF DESCRIPTION OF THE DRAWINGS

FIG. 1 is a diagrammatic view of a system for aspirating thrombus according to an embodiment of the present disclosure.

FIG. 2 is a diagrammatic view showing more detail of the proximal portion of the system for aspirating thrombus of FIG. 1.

FIG. 3 is a diagrammatic view of the distal end portion of the system for aspirating thrombus of FIG. 1.

FIG. 4 is a plan view of disposable components of a system for aspirating thrombus according to an embodiment of the present disclosure.

FIG. 5 is a detailed view of detail 5 of FIG. 4.

FIG. 6 is a detailed view of detail 6 of FIG. 4.

FIG. 7 is a detailed view of detail 7 of FIG. 4.

FIG. 8 is a detailed view of detail 8 of FIG. 4.

FIG. 9 is a plan view of a distal end of an aspiration catheter of the system for aspirating thrombus of FIG. 4.

FIG. 10 is a sectional view of FIG. 9 taken through line 10-10, as viewed within a blood vessel.

FIG. 11 is a detailed view of detail 11 of FIG. 10.

FIG. 12 is perspective view of a pump base according to an embodiment of the present disclosure.

FIG. 13 illustrates a piston of the system for aspirating thrombus being coupled to a saddle of a piston pump.

FIG. 14 is a cross-sectional view of the distal tip of the aspiration catheter of FIG. 9.

FIG 15 is a view a cassette for coupling to a pump base.

FIG. 16 is a sectional view of the cassette of FIG. 15.

FIG. 17 is a partially exploded view of the pump base of FIG. 12.

FIG. 18 is a graph of a pressure vs. time relationship of a piston pump.

FIG. 19 is a plan view of a piston and a cassette of a piston pump according to an embodiment of the present disclosure.

FIG. 20 is a graph of a pressure vs. time relationship of a piston pump.

FIG. 21 is a plan view of disposable components of a system for aspirating thrombus according to an embodiment of the present disclosure.

FIG. 22 is a detailed view of a catheter of the system for aspirating thrombus of FIG. 21.

FIG. 23 is a detailed view of a tubing set of the system for aspirating thrombus of FIG. 21.

FIG. 24 is an exploded view of a saline pump drive unit according to an embodiment of the present disclosure.

FIG. 25 is an exploded view of a disposable piston pump head of the saline pump unit of FIG. 24.

FIG. 26 is a sectional view of an aspiration catheter of a system for aspirating thrombus within a blood vessel according to an embodiment of the present disclosure.

FIG. 27 is a sectional view of a catheter within a blood vessel delivering a drug to a target site.

FIG. 28 is a sectioned perspective view of an aspiration catheter according to an embodiment of the present disclosure.

FIG. 29 is a sectioned perspective view of an aspiration catheter according to an embodiment of the present disclosure.

FIG. 30 is a sectioned perspective view of an aspiration catheter according to an embodiment of the present disclosure.

FIG. 31 is a sectioned perspective view of an aspiration catheter according to an embodiment of the present disclosure.

FIG. 32 is a sectioned perspective view of the aspiration catheter of FIG. 28 with a significant negative pressure applied on the aspiration lumen.

FIG. 33 is a sectioned perspective view of the aspiration catheter of FIG. 29 with a significant negative pressure applied on the aspiration lumen.

FIG. 34 is a sectioned perspective view of the aspiration catheter of FIG. 30 with a significant negative pressure applied on the aspiration lumen.

FIG. 35 is a sectioned perspective view of the aspiration catheter of FIG. 31 with a significant negative pressure applied on the aspiration lumen.

FIG. 36 is a sectioned perspective view of the aspiration catheter of FIG. 28 with little or no negative pressure applied on the aspiration lumen.

FIG. 37 is a sectioned perspective view of the aspiration catheter of FIG. 29 with little or no negative pressure applied on the aspiration lumen.

FIG. 38 is a sectioned perspective view of the aspiration catheter of FIG. 30 with little or no negative pressure applied on the aspiration lumen.

FIG. 39 is a sectioned perspective view of the aspiration catheter of FIG. 31 with little or no negative pressure applied on the aspiration lumen.

FIG. 40 is a sectioned perspective view of the aspiration catheter of FIG. 28 with a particular negative pressure applied on the aspiration lumen.

FIG. 41 is a sectioned perspective view of the aspiration catheter of FIG. 30 with a particular negative pressure applied on the aspiration lumen.

FIG. 42 is a sectioned perspective view of an aspiration catheter according to an embodiment of the present disclosure.

FIG. 43 is a sectioned perspective view of an aspiration catheter according to an embodiment of the present disclosure.

FIG. 44A is an end view of an aspiration catheter according to an embodiment of the present disclosure.

FIG. 44B is a longitudinal sectional view of an aspiration catheter according to an embodiment of the present disclosure.

FIG. 45A is an end view of an aspiration catheter according to an embodiment of the present disclosure.

FIG. 45B is a longitudinal sectional view of an aspiration catheter according to an embodiment of the present disclosure.

FIG. 46A is a longitudinal sectional view of an aspiration catheter in a first state according to an embodiment of the present disclosure.

FIG. 46B is a longitudinal sectional view of the aspiration catheter of FIG 46A in a second state according to an embodiment of the present disclosure.

FIG. 47 is a sectional view of a spray pattern of an aspiration catheter according to an embodiment of the present disclosure.

FIG. 48 is a sectional view of a spray pattern of an aspiration catheter according to an embodiment of the present disclosure.

FIG. 49 is a partial cutaway view of a spray pattern of an aspiration catheter according to an embodiment of the present disclosure.

FIG. 50 is a partial cutaway view of a spray pattern of an aspiration catheter according to an embodiment of the present disclosure.

FIG. 51 is a partial cutaway view of a spray pattern of an aspiration catheter according to an embodiment of the present disclosure.

FIG. 52 is a sectional view of a spray pattern of an aspiration catheter according to an embodiment of the present disclosure.

FIGS. 53-55 are sectional views of a thrombus/clot being treated by an aspiration catheter according to an embodiment of the present disclosure.

FIG. 56 is a sectional view an aspiration system including an aspiration catheter and a curved mandrel tool, according to an embodiment of the present disclosure.

FIG. 57 is a sectional view of the aspiration system of FIG. 56 in a deflected state.

FIG. 58 is an elevation view of an aspiration system according to an embodiment of the present disclosure.

FIG. 59A is a sectional view of an aspiration system including an aspiration catheter and a spinning wire, according to an embodiment of the present disclosure.

FIG. 59B is an elevation view of a rotating device for rotating the spinning wire of the embodiment of FIG. 59A.

FIG. 60 is a sectional view of a system for removing intracranial thrombus or intracranial hematoma through a window, aperture, or hole in the cranium of a patient.

FIG. 61 is a plan view of a system for aspirating thrombus according to an embodiment of the present disclosure.

FIG. 62 is a sectional view of the system for aspirating thrombus of FIG. 61.

FIG. 63 is diagrammatic representation of a method for aspirating thrombus, according to an embodiment of the present disclosure.

FIG. 64 is a plan view of disposable components of a system for aspirating thrombus according to an embodiment of the present disclosure.

FIG. 65 is a sectional view of a distal end of the aspiration catheter of the system for aspirating thrombus of FIG. 64.

FIG. 66 is a detail view of a y-connector of the aspiration catheter of the system for aspirating thrombus of FIG. 64.

FIG. 67 is a partially sectional view of the aspiration catheter of the system for aspirating thrombus of FIG. 64 in use.

FIG. 68 is a detail view of an orifice of the aspiration catheter of FIG. 67.

FIG. 69 is an enlarged view of the y-connector of FIG. 66 in use.

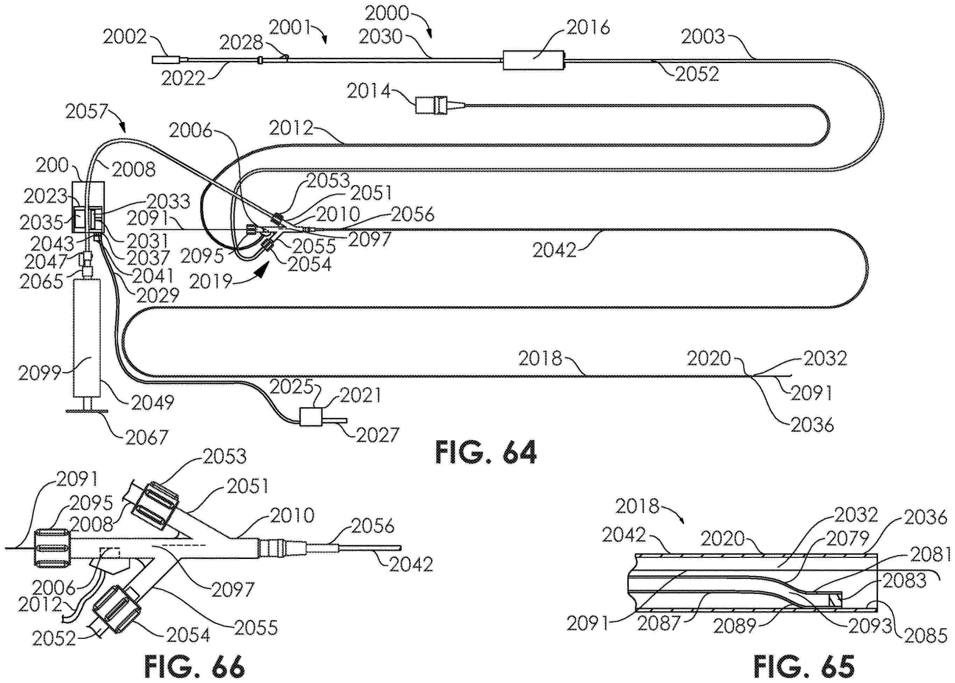

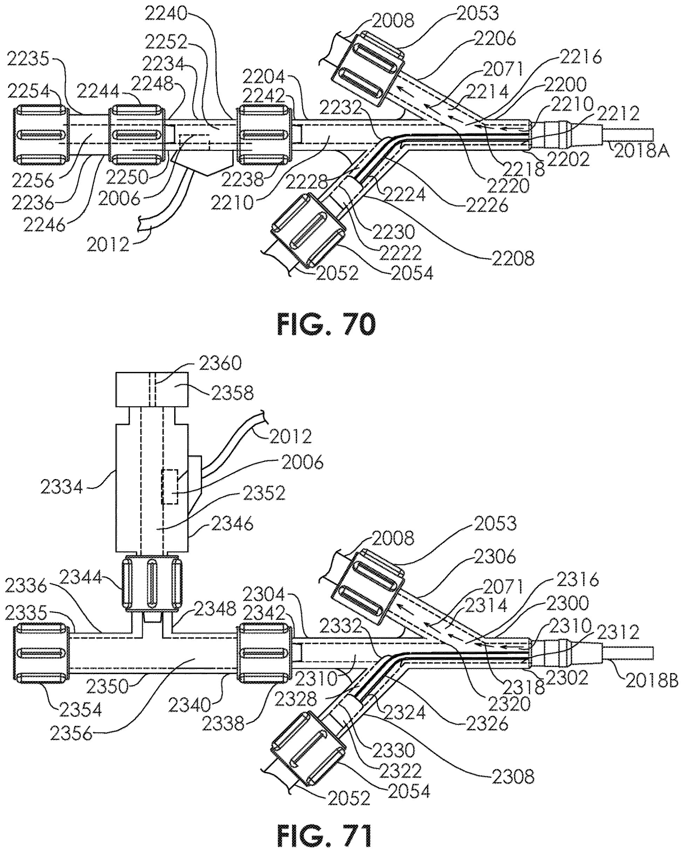

[0090] FIG. 70 is a plan view of an alternate connector configuration for the aspiration catheter of FIG. 64, according to an embodiment of the present disclosure.

FIG. 71 is a plan view of an alternate connector configuration for the aspiration catheter of FIG. 64, according to an embodiment of the present disclosure.

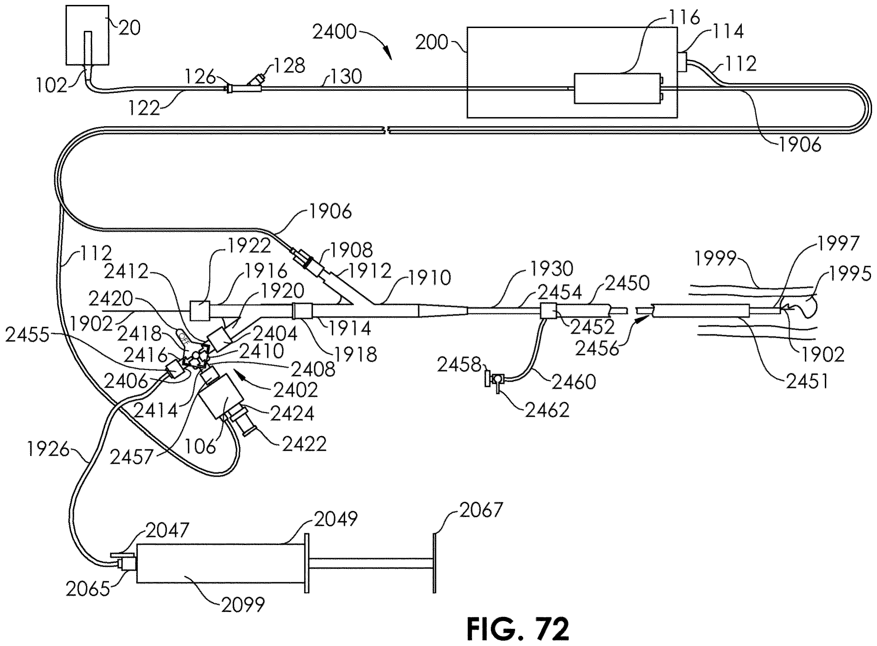

FIG. 72 is a plan view of an aspiration system having an aspiration catheter with a valve in a first state, according to an embodiment of the present disclosure.

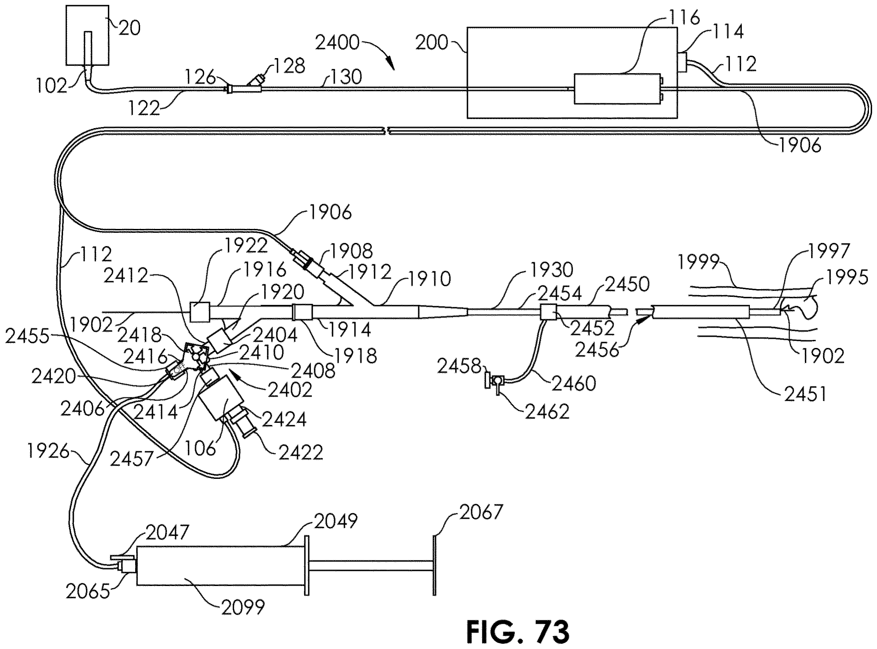

FIG. 73 is a plan view of the aspiration system of FIG. 72 with the aspiration catheter with a valve in a second state, according to an embodiment of the present disclosure.

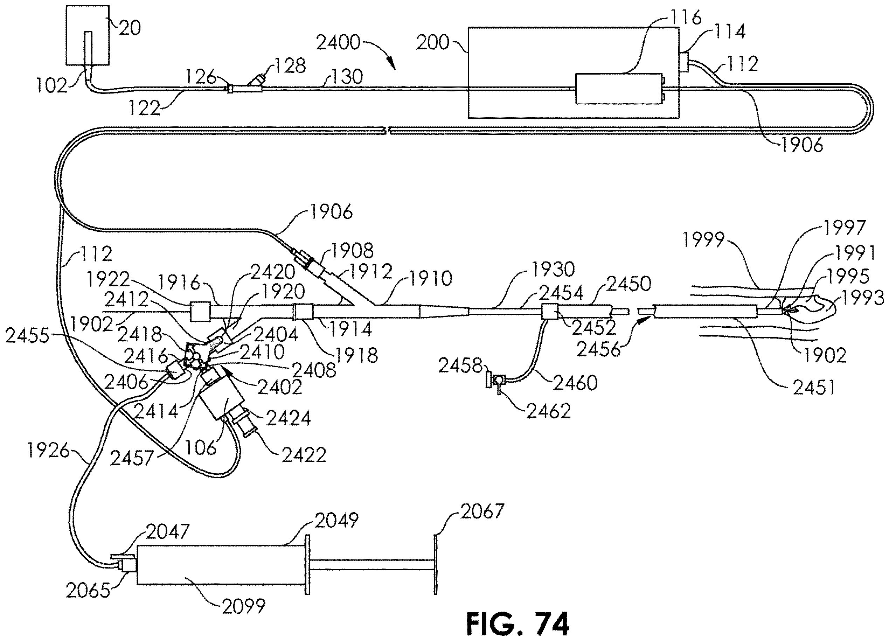

FIG. 74 is a plan view of the aspiration system of FIG. 72 with the aspiration catheter with a valve in a third state, according to an embodiment of the present disclosure.

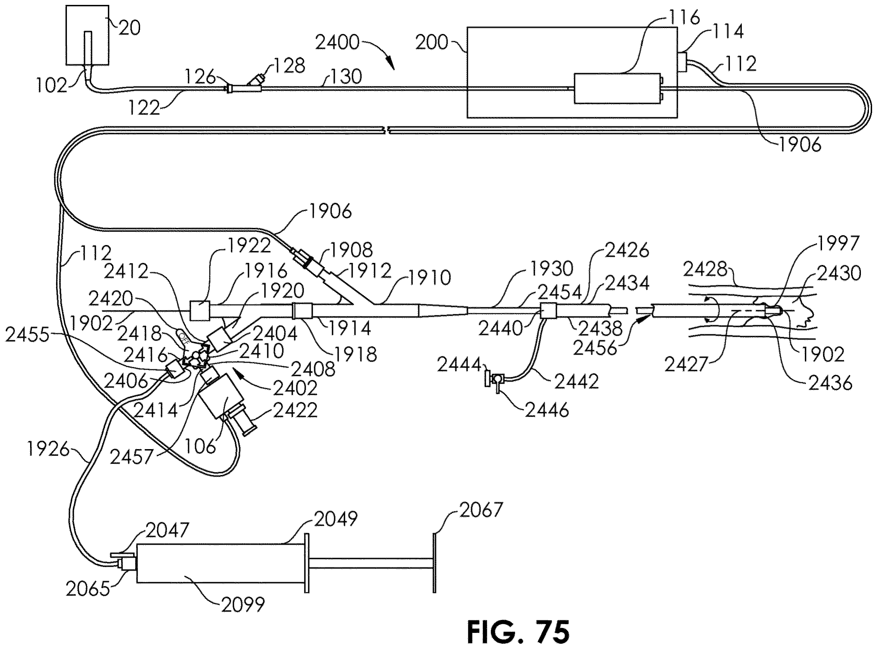

FIG. 75 is a perspective view of an aspiration system including an aspiration catheter and a guiding catheter, according to an embodiment of the present disclosure.

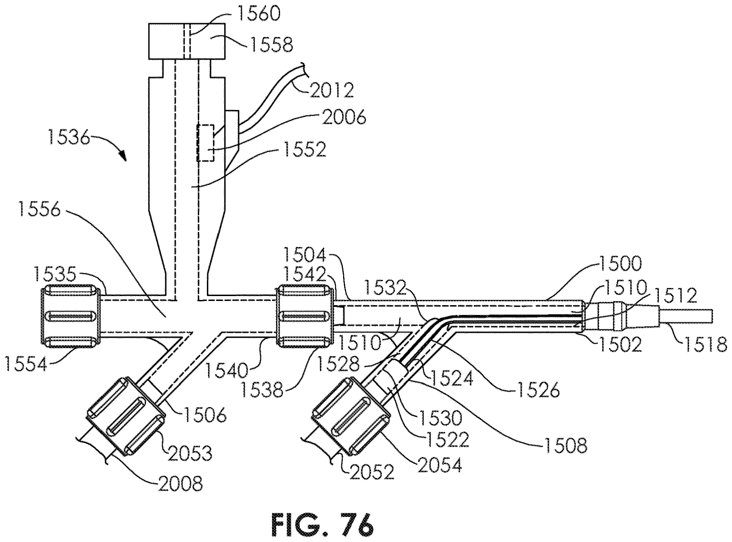

FIG. 76 is a plan view of an alternate connector configuration for an aspiration catheter, according to an embodiment of the present disclosure.

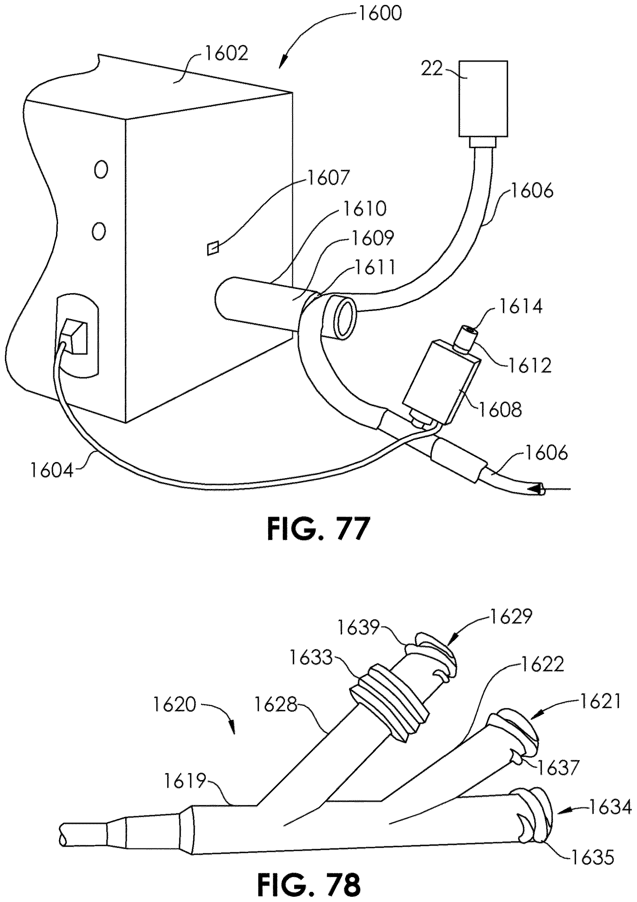

FIG. 78 is a perspective view of a y-connector of an aspiration catheter of an aspiration system according to an embodiment of the present disclosure.

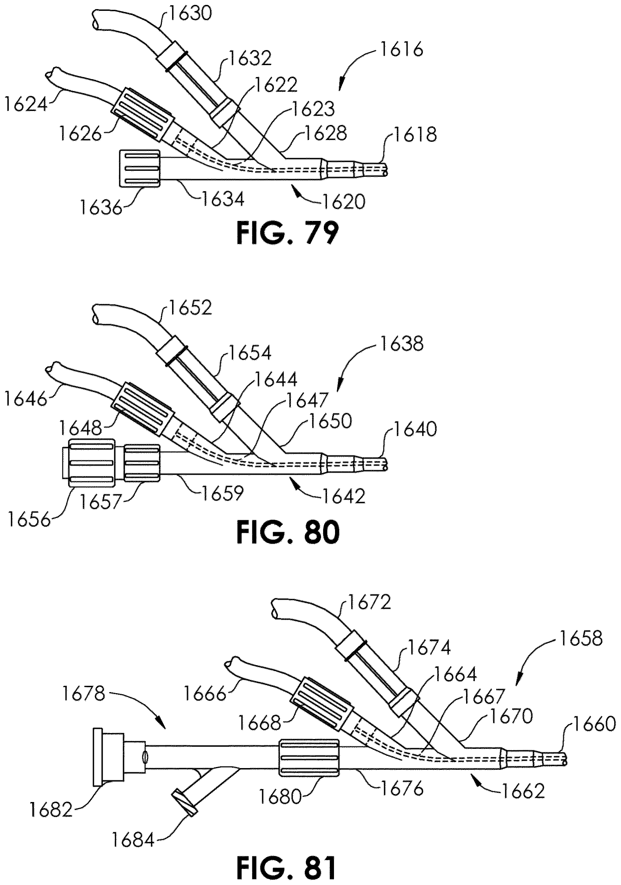

FIG. 79 is a plan view of a connector configuration of an aspiration system according to an embodiment of the present disclosure.

FIG. 80 is a plan view of a connector configuration of an aspiration system according to an embodiment of the present disclosure.

FIG. 81 is a plan view of a connector configuration of an aspiration system according to an embodiment of the present disclosure.

DETAILED DESCRIPTION OF THE ILLUSTRATED EMBODFMENTS

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5). [0105] As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The drawings, which are not necessarily to scale, depict illustrative embodiments and are not intended to limit the scope of the invention.

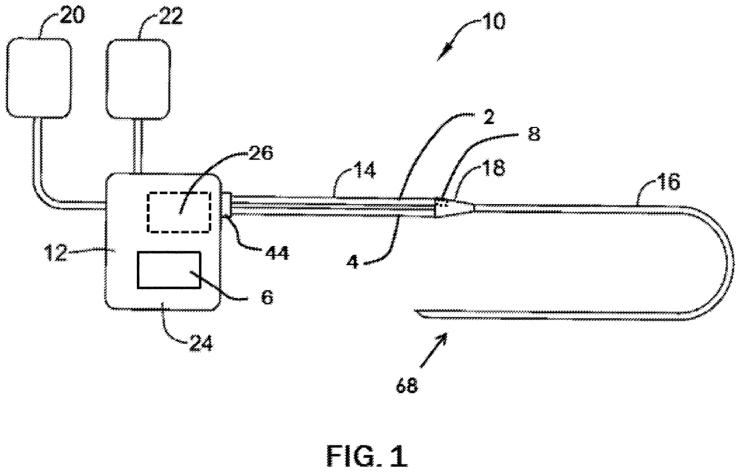

FIG. 1 is a diagrammatic figure depicting an assisted aspiration system 10. The aspiration system 10 includes a remote hand piece 12 that contains a fluid pump 26 and an operator control interface 6. In one contemplated embodiment, the system 10 is a single use disposable unit. The aspiration system 10 may also include extension tubing 14, which contains a fluid irrigation lumen 2(or high pressure injection lumen) and an aspiration lumen 4, and which allows independent manipulation of a catheter 16 without requiring repositioning of the hand piece 12 during a procedure performed with the aspiration system 10. Extension tubing 14 may also act as a pressure accumulator. High pressure fluid flow from the pump 26, which may comprise a displacement pump, pulses with each stroke of the pump 26, creating a sinusoidal pressure map with distinct variations between the peaks and valleys of each sine wave. Extension tubing 14 may be matched to the pump 26 to expand and contract in unison with each pump pulse to reduce the variation in pressure caused by the pump pulses to produce a smooth or smoother fluid flow at tip of catheter 16. Any tubing having suitable compliance characteristics may be used. The extension tubing 14 may be permanently attached to the pump 26 or it may be attached to the pump 26 by a connector 44. The connector 44 is preferably configured to ensure that the extension tubing 14 cannot be attached to the pump 26 incorrectly.

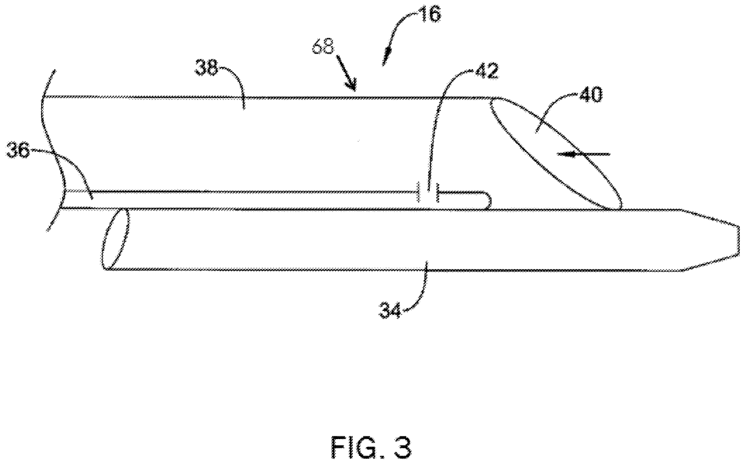

An interface connector 18 joins the extension tubing 14 and the catheter 16 together. In one contemplated embodiment, the interface connector 18 may contain a filter assembly 8 between high pressure fluid injection lumen 2 of the extension tubing 14 and a high-pressure injection lumen 36 of the catheter 16 (FIG. 3). The catheter 16 and the extension tubing 14 may be permanently joined by the interface connector 18. Alternatively, the interface connector 18 may contain a standardized connection so that a selected catheter 16 may be attached to the extension tubing 14.

Attached to the hand piece 12 are a fluid source 20 and a vacuum source 22. A standard hospital saline bag may be used as fluid source 20; such bags are readily available to the physician and provide the necessary volume to perform the procedure. Vacuum bottles may provide the vacuum source 22, or the vacuum source 22 may be provided by a vacuum canister, syringe, a vacuum pump or other suitable vacuum sources.

In one contemplated embodiment, the catheter 16 has a variable stiffness ranging from stiffer at the proximal end to more flexible at the distal end. The variation in the stiffness of the catheter 16 may be achieved with a single tube with no radial bonds between two adjacent tubing pieces. For example, the shaft of the catheter 16 may be made from a single length of metal tube that has a spiral cut down the length of the tube to provide shaft flexibility. Variable stiffness may be created by varying the pitch of the spiral cut through different lengths of the metal tube. For example, the pitch of the spiral cut may be greater (where the turns of the spiral cut are closer together) at the distal end of the device to provide greater flexibility. Conversely, the pitch of the spiral cut at the proximal end may be lower (where the turns of the spiral cut are further apart) to provide increased stiffness. In some embodiments, a single jacket may cover the length of the metal tube to provide for a vacuum tight catheter shaft. An inner layer or lining of a lubricious material, such as a fluoropolymer including PTFE and an outer layer or jacket of PEBAX may together encapsulate or sandwich the spiral -cut metal tube. The spiral-cut tube can be encapsulated in a manner such that it stops short of the distal end of the catheter 16, so that a more flexible tip is provided. Other features of catheter 16 are described with reference to FIG. 3.

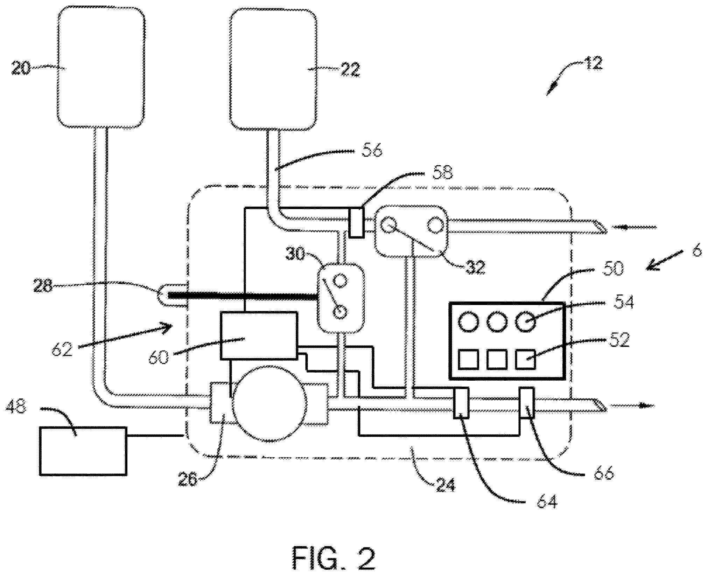

FIG. 2 is a diagrammatic view showing more detail of the hand piece 12 and the proximal portion of assisted catheter aspiration system 10. The hand piece 12 includes a control box 24 where the power and control systems are disposed. The pump 26 may in some embodiments be a motor driven displacement pump that has a constant output. The pump displacement relationship to the catheter volume, along with the location of the orifice 42 (exit) of the catheter high pressure lumen 36 within the aspiration lumen 38 (FIG. 3), ensures that no energy is transferred to the patient from the saline pump as substantially all pressurized fluid is evacuated by the aspiration lumen. A prime button 28 is mechanically connected to a prime valve 30. When preparing the device for use, it is advantageous to evacuate all air from the pressurized fluid system to reduce the possibility of air embolization. By depressing the prime button 28, the user connects the fluid source 20 to the vacuum source 22 via the pump 26. This forcefully pulls fluid (for example 0.9 % NaCl solution, or "saline", or "normal saline", or heparinized saline) through the entire pump system, removing all air and positively priming the system for safe operation. A pressure/vacuum valve 32 is used to turn the vacuum on and off synchronously with the fluid pressure system. One contemplated valve 32 is a ported one-way valve. Such a valve is advantageous with respect to manual or electronic valve systems because it acts as a tamper proof safety feature by mechanically and automatically combining the operations of the two primary systems. By having pressure/vacuum valve 32, the possibility of turning the vacuum on without also activating the fluid system is eliminated.

The operator control interface 6 is powered by a power system 48 (such as a battery or an electrical line), and may comprise an electronic control board 50, which may be operated by a user by use of one or more switches 52 and one or more indicator lamps 54. The control board 50 also monitors and controls several device safety functions, which include over pressure detection, air bubble detection, and vacuum charge. A pressure sensor 64 monitors pressure (i.e. injection pressure), and senses the presence of air bubbles. Alternatively, or in conjunction, an optical device 66 may be used to sense air bubbles. In one contemplated embodiment, the pump pressure is proportional to the electric current needed to produce that pressure. Consequently, if the electric current required by pump 26 exceeds a preset limit, the control board 50 will disable the pump 26 by cutting power to it. Air bubble detection may also be monitored by monitoring the electrical current required to drive the pump 26 at any particular moment. In order for a displacement pump 26 to reach high fluid pressures, there should be little or no air (which is highly compressible) present in the pump 26 or connecting system (including the catheter 16 and the extension tubing 14). The fluid volume is small enough that any air in the system will result in no pressure being generated at the pump head. The control board monitors the pump current for any abrupt downward change that may indicate that air has entered the system. If the rate of drop is faster than a preset limit, the control board 50 will disable the pump 26 by cutting power to it until the problem is corrected. Likewise, a block in the high-pressure lumen 36 (FIG. 3), which may be due to the entry of organized or fibrous thrombus, or a solid embolus, may be detected by monitoring the electrical current running the pump 26. In normal use, the current fluxuations of the pump 26 are relatively high. For example, the pump 26 may be configured so that there is a variation of 200 milliAmps or greater in the current during normal operation, so that when current fluxuations drop below 200 milliAmps, air is identified, and the system shuts down. Alternatively, current fluxuations in the range of, for example, 50 milliAmps to 75 milliAmps may be used to identify that air is in the system. Additionally, an increase in the current or current fluxuations may indicate the presence of clot or thrombus within the high-pressure lumen 36. For example, a current of greater than 600 milliAmps may indicate that thrombus it partially or completely blocking the high-pressure lumen 36, or even the aspiration lumen 38 (FIG. 3).

A vacuum line 56, connected to the vacuum source 22, may be connected to a pressure sensor 58. If the vacuum of the vacuum source 22 is low (i.e. the absolute value pressure has decreased) or if a leak is detected in the vacuum line 56, the control board 50 disables the pump 26 until the problem is corrected. The pressure sensor 58 may also be part of a safety circuit 60 that will not allow the pump 26 to run if a vacuum is not present. Thereby, a comprehensive safety system 62, including the safety circuit 60, the pressure sensor 64 and/or the optical device 66, and the pressure sensor 58, requires both pump pressure and vacuum pressure for the system to run. If a problem exists (for example, if there is either a unacceptably low pump pressure or an absence of significant vacuum), the control board 50 will not allow the user to operate the aspiration system 10 until all problems are corrected. This will keep air from being injected into a patient, and will assure that the aspiration system 10 is not operated at incorrect parameters. Alternatively, in lieu of a direct connection (e.g., electrical, optical), the pressure sensor 58 can be configured to send a wireless signal to the control board 50, or any other component (e.g., antenna) coupled to or in communication with the control board 50, to remotely control operation of the pump 26. The remote control may be possible, whether the pump is within the sterile filed or outside the sterile field.

FIG. 3 is a diagrammatic view of the distal end portion 68 of the assisted catheter aspiration system 10, showing more details of the catheter 16. The catheter 16 in some embodiments is a single-operator exchange catheter and includes a short guidewire lumen 34 attached to the distal end of the device. The guidewire lumen 34 can be between about 1 and about 30 cm in length, or between about 5 and about 25 cm in length, or between about 5 and about 20 cm in length, or approximately 13.5 cm in length. In other embodiments, a full-length guidewire lumen (extending the length of the catheter 16) may be used. For example, a catheter 16 sized to be used on peripheral blood vessels, including peripheral arteries, may incorporate a full-length guidewire lumen. In some embodiments, the aspiration itself may also serve as a guidewire lumen. An aspiration lumen 38 includes a distal opening 40 which allows a vacuum (for example, from vacuum source 22) to draw thrombotic material into the aspiration lumen 38. A high-pressure lumen 36 includes a distal orifice 42 that is set proximally of distal opening 40 by a set amount. For example, distal orifice 42 can be set proximally of distal opening 40 by about 0.508 mm (0.020 inches), or by 0.508 mm ± 0.076 mm (0.020 inches t 0.003inches) or by another desired amount. The orifice 42 is configured to spray across the aspiration lumen to macerate and/or dilute the thrombotic material for transport to vacuum source 22, for example, by lowering the effective viscosity of the thrombotic material. The axial placement of the fluid orifice 42 is such that the spray pattern interaction with the opposing lumen wall preferably produces a spray mist and not a swirl pattern that could force embolic material out from the distal opening 40. The spray pattern may be present at least when a distal end of the catheter 16 is within an aqueous environment, such as a body lumen, including a blood vessel. The aqueous environment may be at body temperature, for example between about 35.0°C and about 40.0°C, or between about 36.0°C and about 38.0°C. The system may be configured so that the irrigation fluid leaves the pump at a pressure of between about 3.447 megapascal (500 pounds per square inch) and about 10.342 megapascal (1500 pounds per square inch). In some embodiments, after a pressure head loss along the high-pressure lumen 36, the irrigation fluid leaves orifice 42 at between about 4.137 megapascal (600 pounds per square inch) and about 8.274 megapascal (1200 pounds per square inch), or between about 4.816 megapascal (650 pounds per square inch) and about 5.861 megapascal (850 pounds per square inch).

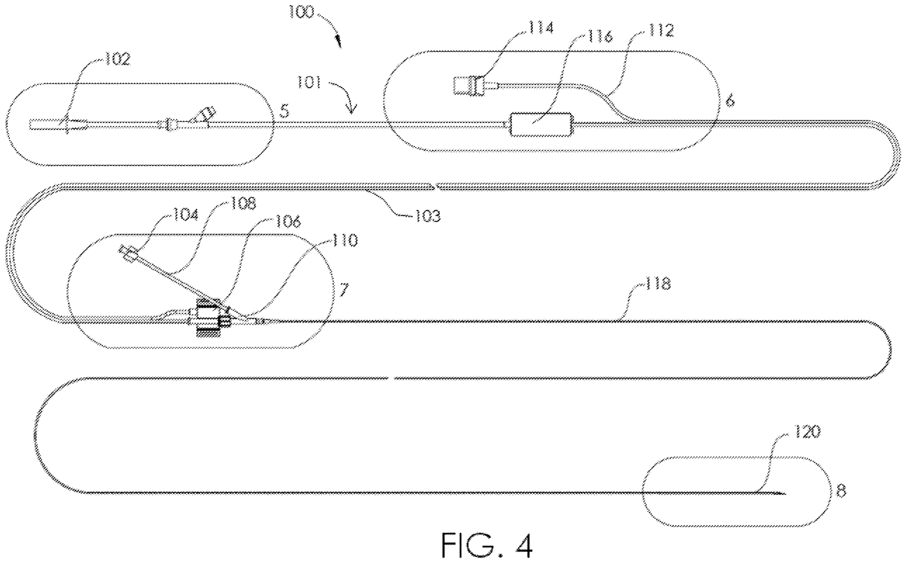

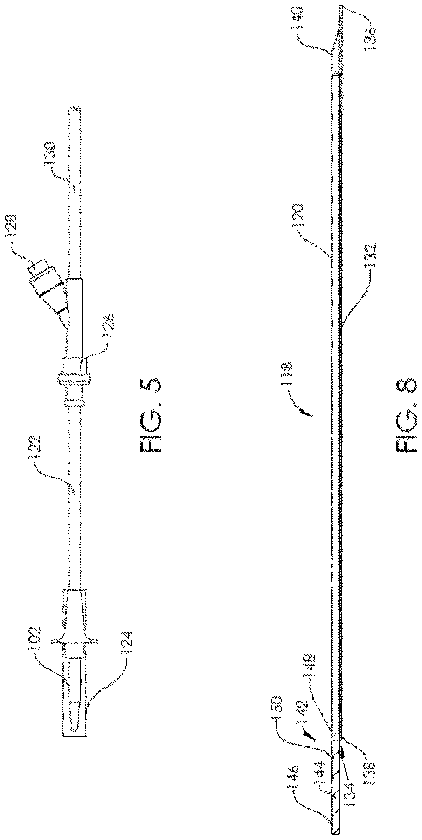

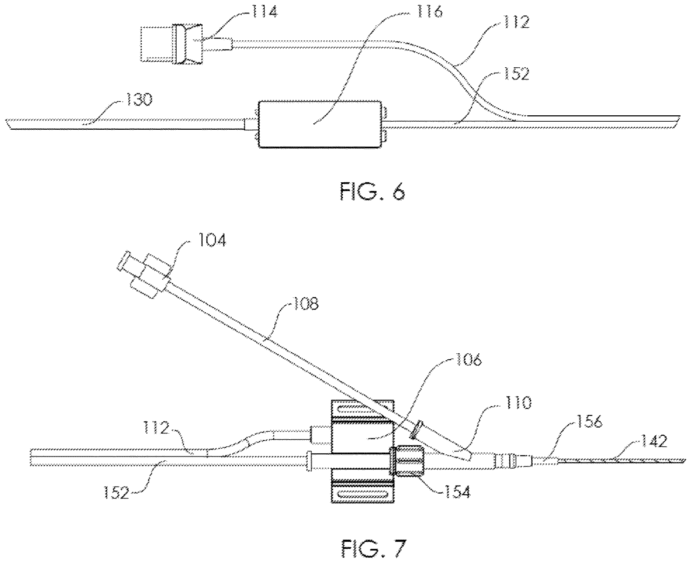

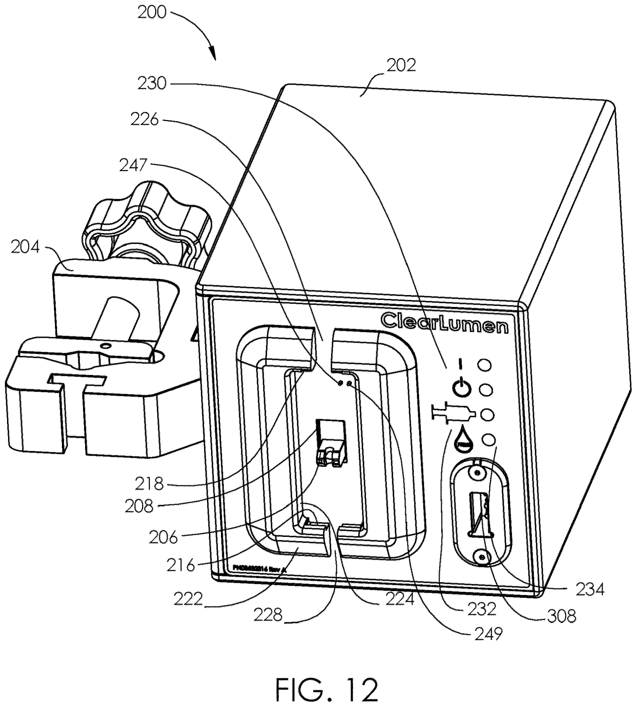

FIG. 4 illustrates a system for aspirating thrombus 100 according to an embodiment of the present disclosure. The system for aspirating thrombus 100 depicted in FIG. 4 represents disposable components 101, comprising a tubing set 103 and an aspiration catheter 118, which are configured to attach to a vacuum source 22, a fluid source 20 (FIGS. 1 and 2), a pressure monitor (not shown), and a pump base 200 (FIG. 12). The system for aspirating thrombus 100 is also configured to be used with a guidewire. Beginning with the components of the tubing set 103, a spike 102 (shown in more detail in FIG. 5) is configured to couple to a fluid source 20 such as a saline bag. The saline bag may have a volume of saline equal to about 1000 ml or about 500 ml. The saline may comprise normal saline, and may be heparinized, or may contain one or more therapeutic agents. Other fluids may be used in place of normal saline or a saline mixture, including lactated Ringer's solution, hypertonic saline, or even solutions containing blood products. The saline, or other fluid, may be at room temperature, or may be warmed or cooled (e.g., to permanently or temporarily increase or decrease activity). A connector 104 (shown in more detail in FIG. 7), for example a luer connector, is configured to couple to a vacuum source 22. The vacuum source 22 may be a vacuum bottle or canister having a volume of between 20 ml and 500 ml. The vacuum source 22 may instead be a 60 ml syringe whose plunger is pulled back after coupling to the connector 104. This may be a lockable plunger, which is locked in order to maintain the evacuated plunger position. In some cases, the vacuum source 22 may be a 20 ml syringe or a 30 ml syringe. An exemplary syringe with a lockable plunger is the VacLok® syringe sold by Merit Medical Systems, Inc. of South Jordan, UT, USA. The vacuum source 22 may also be a vacuum pump, with or without a collection container. A pressure transducer 106 capable of measuring vacuum (including positive pressure sensors that are configured to measure positive pressure, but are capable of measuring negative pressure) is coupled to a vacuum line 108 via a y-connector 110. Signals from the pressure transducer 106 travel along a cable 112 (FIG. 7), which also supplies voltage to the pressure transducer 106. A connector 114 (also shown in FIG. 6) couples the cable 112 to a pressure monitor or to the pump base 200. A cassette 116 is a disposable component attachable to the pump base 200 (FIG. 12) for allowing pressurized injection of a liquid injectate (such as saline). The cassette 116 is described in more detail in relation to FIG. 6. The aspiration catheter 118 having a distal end 120 is shown in more detail in FIG. 8.

Turning to FIG. 5, the spike 102 communicates with extension tubing 122. Liquid injectate is pumped downstream at the piston pump, which pulls more liquid injectate (for example from a saline bag) through a check valve 126 and through a supply tube 130. An injection port 128 may be used for injecting other materials into the system, or for removing air or priming the system. The spike 102 may be packaged with a removable protective spike cover 124.

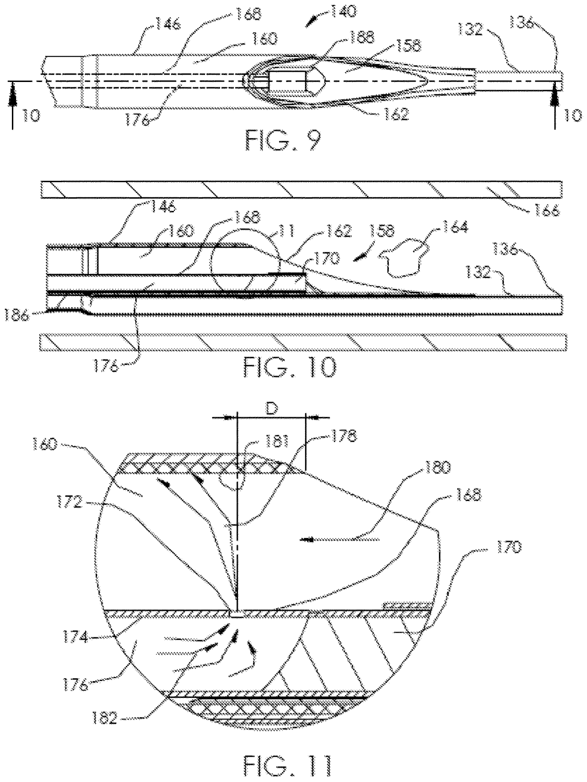

The cassette 116, as seen in FIG. 6, pulls liquid injectate from the supply tube 130, and pressurizes (in conjunction with the pump base 200) an injection tube 152. More detail of the cassette 116 will be described along with the description of the entire piston pump. FIG. 7 shows more detail of the pressure transducer 106 for measuring the vacuum. The pressure transducer 106 connects to the y-connector 110 with a luer fitting 154. The injection tube 152 and the vacuum line 108 communicate to lumens of a catheter shaft 142. For example, the injection tube 152 may be fluidly connected to a distal supply tube 168 (FIGS. 9-11), for example a polyimide or stainless steel or nitinol tube having high strength thin walls. This distal supply tube 168 may reside within the catheter shaft 142, with the annulus between forming an aspiration lumen 160 (FIGS. 9-11). A strain relief 156 protects the catheter shaft 142 from kinking and other damage. In any cases in which luer fittings 154 are used (at any of the connections), a custom luer with an added o-ring may be used in order to allow the connection to withstand elevated pressures. In some embodiments, a bespoke connector may be utilized, to increase high pressure endurance. In some embodiments, pressures as high as 6.89 megapascal (1,200 pounds per square inch) or greater may be achieved without leakage or without causing decoupling of the catheter.

Turning to FIG. 8, the aspiration catheter 118 is illustrated as a single-operator exchange catheter and includes a guidewire tube 132 attached to the distal end 120 on one side of the aspiration catheter 118. The guidewire tube 132 can be between about 1 and about 30 cm in length, or between about 5 and about 25 cm in length, or between about 5 and about 20 cm in length, or approximately 13.5 cm in length. The guidewire tube 132 has a distal end 136 and a proximal end 138, and a single guidewire lumen 134 passing between the two ends 136, 138. The guidewire lumen 134 may be configured to be compatible with a 0.014" guidewire, a 0.018" guidewire, or a number of other guidewire diameters. A lumen inner diameter may be about 0.406 mm (0.016 inches) for compatibility with a 0.014" guidewire. The guidewire tube 132 may be constructed of a number of materials, including nylon, polyethylene, PEBAX®, polyester, PET, or may be constructed from composite or coextruded materials. For example an inner layer may comprise high density polyethylene or FEP, PTFE, ETFE, or other materials for high lubricity, and an outer layer may include PEBAX, nylon or other materials, for combination mechanical strength and flexibility. A tie layer may be used between the inner and outer layers, for example linear low density polyethylene. The catheter 118 may include a composite catheter shaft 142 having an inner support structure 144 covered with a polymer jacket 146. The inner support structure 144 may be a tubular braid or one or more helical coils, for example, made with stainless steel flat or round wires. The inner support structure 144 may also be spiral cut hypodermic tubing, for example made from 304 stainless steel or nickel - titanium. The spiral cut hypodermic tubing may have a pitch measuring about 4 to 6 millimeters, or about 5 millimeters at the proximal end for increased stiffness, transitioning to a pitch of about 0.75 to 1 mm or about 0.87 mm, at the distal end 150 of the inner support structure 144. In between these two different pitch sections, may be intermediate pitch sections, for example, a section having a pitch of between about 2 mm and about 5 mm, and another section having a pitch of about 1 mm to about 2.5 mm. The inner support structure 144 may end at a transition zone 148, so that the polymer jacket 146 alone extends to the distal end 136 of the aspiration catheter 118. A catheter tip portion 140 is described in more detail in relation to FIGS. 9-11.

FIGS. 9-11 show an open distal end 158 of an aspiration lumen 160 for aspirating thrombus. A skive 162 may be formed in the polymer jacket 146, to aid entry of thrombus 164 that is aspirated into the aspiration lumen 160 (in the direction of arrow 180) by the combination of the vacuum created by the vacuum source 22. The skive 162 also minimizes the chances of the open distal end 158 being sucked against a blood vessel wall 166. A distal supply tube 168 has a closed distal end 170, for example, it may be occluded during manufacture using adhesive, epoxy, hot melt adhesive or an interference member. Alternatively, the distal supply tube 168 may be closed off by melting a portion of it. The distal supply tube 168 has a lumen 176 extending its length and an orifice 172 formed through its wall 174 at a location adjacent and proximal to the closed distal end 170. The orifice 172 may have a diameter between about 0.0508 mm (0.002 inches) and about 0.1016 mm (0.004 inches), or about 0.0787 mm (0.0031 inches). The inner diameter of the distal supply tube 168 may be between about 0.3048 mm (0.012 inches) and about 0.4826 mm (0.019 inches), or between about 0.3556 mm (0.014 inches and about 0.4318 mm (0.017 inches) or about 0.3937 mm (0.0155 inches). The lumen 176 of the distal supply tube 168 is a continuation of an overall flow path emanating from the fluid source 20 including the extension tubing 122, the supply tube 130, the interior of the cassette 116, and the injection tube 152. In some embodiments, the lumen 176 of the distal supply tube 168 may taper, for example, from an inner diameter of about 0.3937 mm (0.0155 inches) at a proximal portion to an inner diameter of about 0.2974 mm (0.011 inches) at a distal portion. In some embodiments, the equivalent of a taper may be achieved by bonding different diameter tubing to each other, resulting in a stepped-down tubing inner diameter. In some embodiments, different diameter tapered tubing may be bonded to each other, for a combination of tapering and step-down of diameter. As described in conjunction with the piston pump, a pump output pressure wave of about 4.137 megapascal (600 pounds per square inch) to about 5.516 megapascal (800 pounds per square inch) causes a liquid injectate to flow through the flow path, including a distal supply tube 168 (arrows 182), and causes a fluid jet 178 to exit the orifice 172 at a high velocity. The fluid jet 178, in absence of flow through the aspiration lumen 160 (for example if there is no vacuum), would impinge upon an inner wall 181 of the aspiration lumen 160 directly adjacent the orifice 172. Depending on the amount of vacuum present, the fluid jet, may curve as shown. The fluid jet 178 serves to macerate thrombus 164 that enters the aspiration lumen 160, and dilutes it. The flow rate of the liquid injectate (e.g. saline) and the amount of vacuum are controlled so that about 50% to about 95% of the volume of the mixture of the saline and blood flowing through the proximal aspiration lumen 160 is blood. Or about 90% of the volume is blood. In other embodiments, the flow rate of the liquid injectate (e.g. saline) and the amount of vacuum are controlled so that about 50% to about 70% of the volume of the mixture of the saline and blood flowing through the proximal aspiration lumen 160 is blood. Or, about 60% of the volume is blood. This maceration and dilution assures that there is continuous flow through the aspiration lumen 160 so that it will not clog. The fluid jet 178 is configured to be contained within the aspiration lumen 160, and to not exit into a blood vessel or other body lumen.

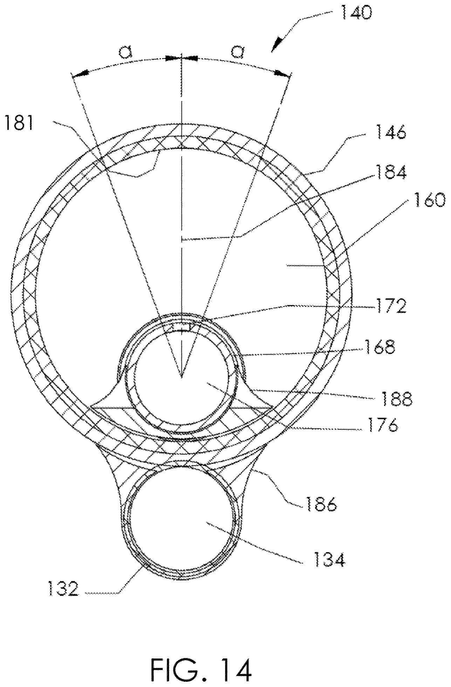

The axial center of the orifice 172 is about 0.3302 mm (0.013 inches) to about 0.8382 mm (0.033 inches), or about 0.4064 mm (0.016 inches) to about 0.6604 mm (0.026 inches) proximal to the most proximal portion of the open distal end 158, as illustrated by distance D in FIG. 11. FIG. 14 is a cross-section of the catheter tip portion 140 at the axial center of the orifice 172. The orifice 172 it is oriented approximately along a vertical midline 184 of the aspiration lumen 160, or within a range of t a, there where angle a is about 20°. The angle a, may be varied in different embodiments between about 1° and about 45°, or between about 20° and about 35°. The guidewire tube 132 may be secured to the polymer jacket 146 with attachment materials 186, such as adhesive, epoxy, hot melt or other materials. The guidewire tube 132 may be secured along its entire length, or at discrete locations along its length, in order to maximize flexibility. The distal supply tube 168 may be secured within the aspiration lumen 160 with attachment materials 188, such as adhesive, epoxy, hot melt or other materials. The polymer jacket 146 may comprise a number of different materials, including PEBAX, nylon, or polyurethane. In some embodiments, the polymer jacket may be partially melt bonded to the distal supply tube 162 and/or the guidewire tube 132, in order to minimize the wall thickness of the assembly.

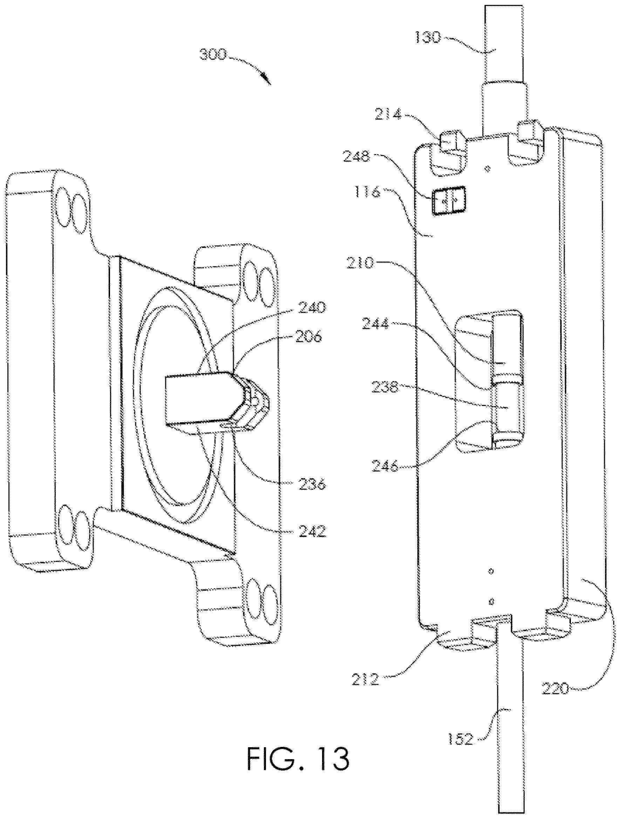

FIG. 12 illustrates a pump base 200 for coupling the cassette 116 of the system for aspiration of thrombus 100. A housing 202 is attached to an IV pole clamp 204, and contains the control circuitry and the motor for operating a piston pump system 300 (FIG. 13) which comprises the combined pump base 200 and the cassette 116. By action of a motor and cam within the pump base 200, a saddle 206 is cyclically actuated (up and down) within a window 208 to move a piston 210 within the cassette 116 (FIG. 13). Pegs 212 of the cassette 116 insert into cavities 216 in the pump base 200. Biased snaps 214 lock into one or more grooves 218 in the pump base 200. Either the cavities 216 or the grooves 218, may have one or more switches which sense the presence of the cassette 116. For example, the cassette for one particular model may have a first number (or combination) of pegs 212 or biased snaps 214, which another particular model may have a different number (or combination) of pegs 212 or biased snaps 214, which is recognized by the system. A smooth surface 224 of an elastomeric frame 222 engages edges 220 of the cassette 116, for enhanced protection. An upper space 226 is configured to engage, or closely match the supply tube 130 and a lower space 228 is configured to engage, or closely match the injection tube 152. The saddle 206 has a semi-cylindrical cavity 236 which snaps over a cylindrical engagement surface 238 on the piston 210. The saddle also has an upper edge 240 and a lower edge 242 for axially engaging a first abutment 244 and a second abutment 246, respectively, of the piston 210. A user interface 230 on the pump base 200 has one or more buttons 232 and one or more indicators 234, which allow the user to operate and assess the operation of the system 100. For example, the buttons may include a start button to begin pumping, a stop button to stop pumping, a prime button to prime the system with a fluid injectate and purge out air, or a temporary pause button. Other data entry keys are also possible. The cassette 116 may include one or more interface components 248. For example, a resistor, whose value the pump base 200 is able to measure via contacts 247, 249 when the cassette 116 is attached to the pump base 200. This allows the pump base 200 to determine the appropriate parameter for operating a specific model of the system 100. For example, a first resistor having a first resistance may be used with a first model and a second resistor having a second resistance may be used with another model. Alternatively, the interface component 248 may incorporate an RFID chip, such as a read RFID chip or a read/write RFID chip. This may allow specific data (pump operating pressures, RPM of motor output, etc.) to be recorded within the pump base 200 or to connected hardware and identified for each patient.

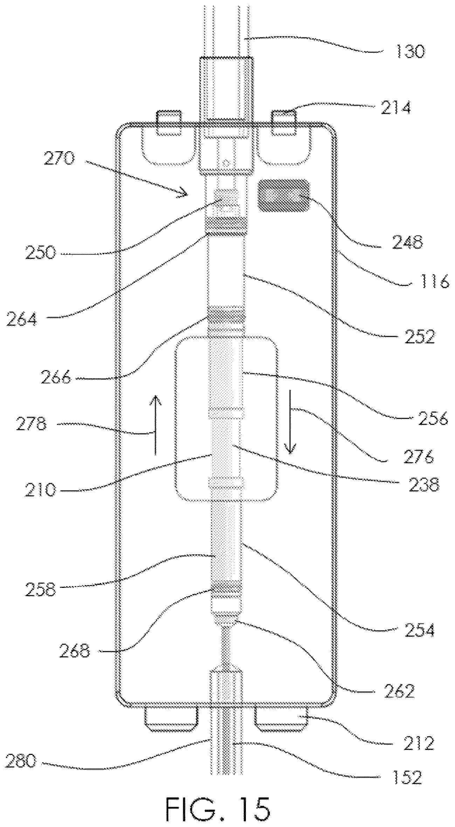

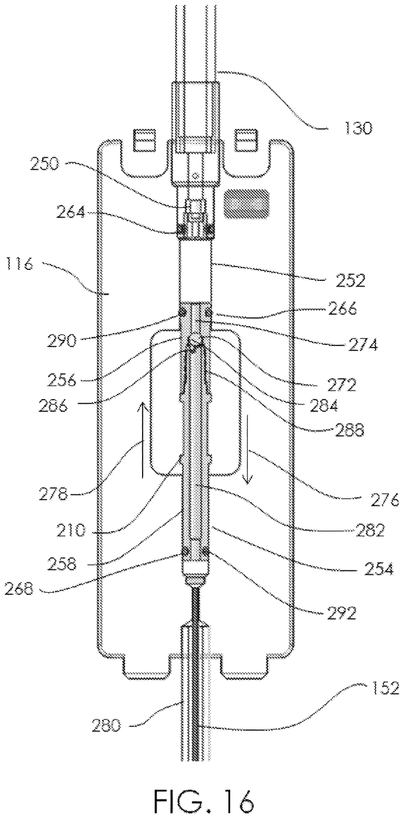

FIG. 15 and 16 illustrate the cassette 116 with most of its internal components visible. FIG. 16 is a sectional view of the cassette 116. The cassette 116 comprises an internal supply cylinder 252 and an internal injection cylinder 254, which are cylindrical cavities extending within the cassette 116. The piston 210 includes a supply side shaft 256 and an injection side shaft 258, the supply side shaft 256 including an o-ring 266 for sealably interfacing with the supply cylinder 252 and the injection side shaft 258 including an o-ring 268 for sealably interfacing with the injection cylinder 254. Each of the o-rings 266, 268 are within a cylindrical groove 290, 292 around each respective shaft portion 256, 258. An internal ball valve 272 (FIG. 16) stops injectate (saline) from flowing through an internal channel 274 in the supply side shaft 256 of the piston 210 when the piston 210 moves in a first direction 276, but the internal ball valve 272 allows injectate to flow through the internal channel 274 and through an internal channel 282 in the injection side shaft 258 when the piston 210 moves in a second direction 278. The ball valve 272 is axially held between a spherical annular recess 284 in the interior of the supply side shaft 256 and a recess having thru channels 286 in the injection side shaft 258. The supply side shaft 256 and the injection side shaft 258 may be held together with a threaded connection 288. When the piston 210 moves in the first direction 276, the injection side shaft 258 of the piston 210 and o-ring 268 force injectate through the injection tube 152. A protective tube 280 is shown over the injection tube 152. In FIG. 15, the injection side shaft 258 is shown at the bottom of an injection pulse. Injectate is filtered through an in-line filter 262, which may be a 40 to 50 micron filter, having an approximate thickness of 0.762 mm (0.030 inches). The in-line filter 262 is configured to keep particulate out of the injectate. Even though injectate is circulated through the aspiration catheter 118, and not into the blood vessel, the filtering provided by the in-line filter 262 is an extra safety step. However, this step helps assure that particulate does not block the small orifice 172 (FIG. 11). When the piston 210 moves in the second direction 278, the supply side shaft 256 of the piston 210 and the o-ring 266 sealably move together within the supply cylinder 252, but the ball valve 272 allows the injectate to pass through the internal channels 274, 282 of the piston 210 and fill the injection cylinder 254. The injectate is able to enter from the supply tube 130 through a check valve assembly 270 comprising an o-ring 264 and a check valve 250. The check valve 250 allows injectate to enter the interior of the cassette 116 from the supply tube 130, but not to move from the cassette 116 to the supply tube 130. The check valve 250 may be configured so that air, due at least in part to its low viscosity, will not be able to cause the check valve 250 to move (open), thus not allowing air to progress through the system. In some embodiments, the piston 210 may be a single piece (monolithic) design with a bore into which a check-valve is press-fit or bonded. A check valve compatible with this assembly may be supplied by the Lee Company of Westbrook, CT, USA.

The volume of injectate injected per cycle may range from about 0.02 ml to about 41 ml, or from about 0.04 ml to about 2.0 ml, or about 0.06 ml to about 0.08 ml, or about 0.07 ml. The usable volume (volume that can be injected) of the injection cylinder 254 may be configured to be less than the usable volume (volume that can be filled from) of the supply cylinder 252, in order to assure sufficient filling of the injection cylinder 254. For example, the usable volume of the injection cylinder 254 may be about 0.05 ml to about 0.12 ml, and the usable volume of the supply cylinder 252 may be about 0.07 ml to about 0.16 ml. A usable volume ratio Ru of between about 1.15 and about 2.00, or between about 1.25 and about 1.85, or about 1.40 is contemplated, where:

$$Ru = Vscu/Vicu,$$

wherein:

Vscu = Usable volume of the supply cylinder 252, and

Vicu = Usable volume of the injection cylinder 254.

A mean flow rate of between about 5 ml/minute and about 100 ml/minute. In some embodiments for use in coronary applications, 20 ml/minute may be desired. In some embodiments for use in peripheral applications, 50 ml/minute may be desired.

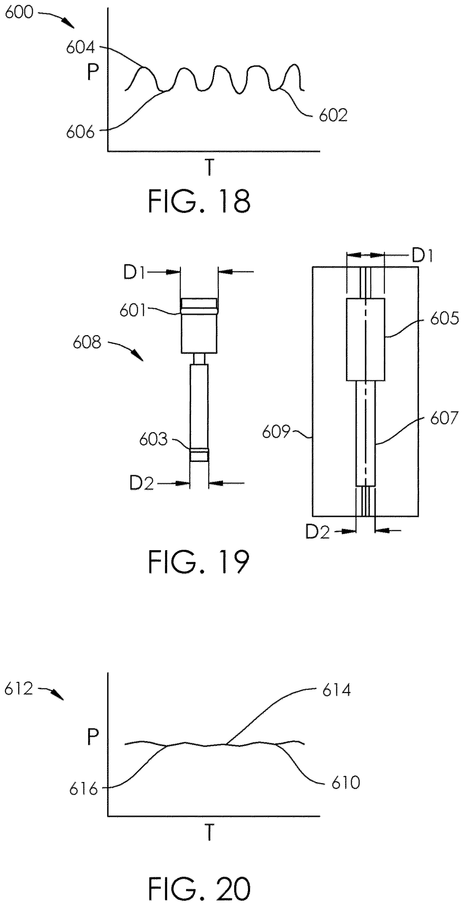

FIG. 18 illustrates a graph 600 of a pressure (P) vs. time (T) curve 602 of a piston pump. Peaks 604 and valley 606 of the curve 602 can be dependent upon the design of the piston and cylinders of the piston pump, particularly of the usable volume ratio Ru. Turning to FIG. 19, a piston 608 is illustrated having a first diameter Di and a second diameter D2 measured at the compressed o-rings 601, 603 (when placed within cylinders 605 and 607 of a cassette 609). The diameters of the cylinders 605, 607 are thus also defined as diameters Di and D2. When the diameters Di, D2, and the lengths of the cylinders 605, 607 are adjusted such that the usable volume ratio Ru is optimized as previously described, a curve 610 as illustrated in FIG. 20 may be produced. The curve 610 has less-defined peaks 614 and valleys 616, and thus produces less variation of flow amplitude, and a more balanced injection.

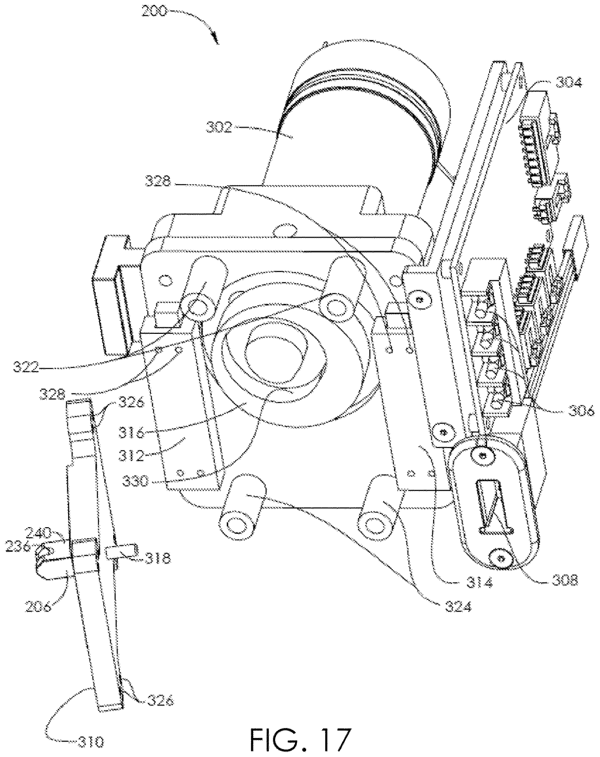

The partially exploded pump base 200 in FIG. 17 illustrates the internal mechanisms for linear (up and down) actuation of the saddle 206, which is attached to a saddle stage 310. A motor 302 is controlled by a circuit board 304 and operated by the user interface 230 (FIG. 12), whose indicators 234 are lit by LEDs 306. The motor 302 turns a cam 316, in which includes a path 330. The saddle stage 310 has a pin 318 extending from its back side. The pin 318 may be press fit, bonded or screwed in place within the saddle stage 310. The saddle stage 310 is secured with screws to two slides 312, 314 through holes 326, 328, such that rotary motion of the cam 316 causes the pin 318 to track along the path 330 of the cam 316, thus causing the saddle stage 310 attached to the slides 312, 314 to slide upward and downward in cyclic motion. The shape of the cam determines the amount of acceleration and deceleration in the motion. Upper posts 322 and lower posts 324 serve as guides and/or stops of the saddle stage 310. The connector 114 of the pressure transducer 106 for measuring vacuum may be plugged into socket 308 (also shown in FIG. 12), and pressure related signals may be processed by the circuit board 304. The entire pump base 200 is reusable.

The inner contour diameter of the cam 316 may be sized and/or shaped to control the stroke length of the piston 210 and the amount of pulsatility (i.e., the difference between the high and low pressure). In some cases, decreasing the stroke length decreases the amount of pulsatility. In applications within the heart, such as coronary artery applications, lowering the amount of pulsatility can reduce the incidence of bradycardia. To compensate for a lower stroke length, and to maintain a sufficient total flow rate, the speed of the rotation of the cam (i.e. rotations per minute), can be increased, for example by increasing motor output speed, either by gearing or by increased applied voltage.

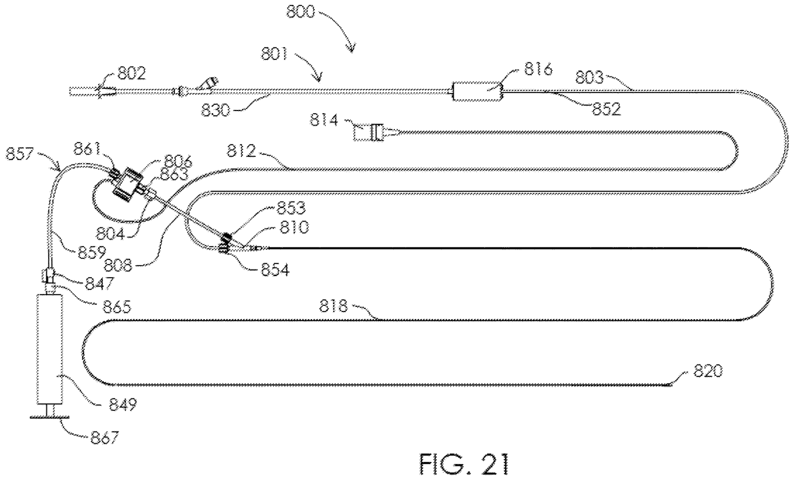

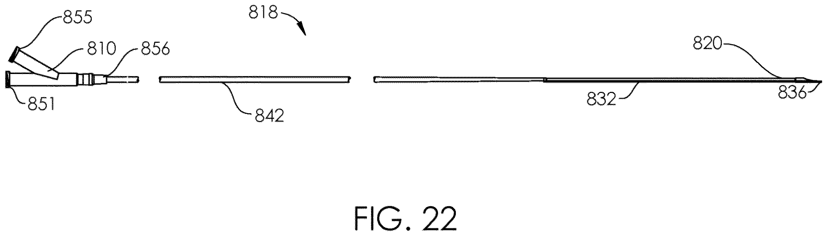

Another embodiment of a system for aspirating thrombus 800 is illustrated in FIG. 21. The system for aspirating thrombus 800 includes, three major components: the pump base 200 of FIG. 12, an aspiration catheter 818, and a tubing set 803. The aspiration catheter 818 and the tubing set 803 represent disposable components 801, and the pump base 200 is a reusable component. It is not necessary to sterilize the pump base 200 as it is kept in a non-sterile field or area during use. The aspiration catheter 818 and the tubing set 803 may each be supplied sterile, after sterilization by ethylene oxide gas, electron beam, gamma, or other sterilization methods. The aspiration catheter 818 may be packaged and supplied separately from the tubing set 803, or the aspiration catheter 818 and the tubing set 803 may be package together and supplied together. Alternatively, the aspiration catheter 818 and tubing set may be packaged separately, but supplied together (i.e., bundled). As shown in FIGS. 21 and 22. The aspiration catheter 818 and tubing set 803 share many of the same features as the aspiration catheter 118 and tubing set 103 of FIG. 4, but are configured to allow easier separation from each other, and additional procedural adaptability. The aspiration catheter 818 has a distal end 820 comprising a guidewire tube 832 having a distal tip 836, and a proximal end 819 comprising a y- connector 810. The catheter shaft 842 of the aspiration catheter 818 is connected to the y- connector 810 via a protective strain relief 856. In other embodiments, the catheter shaft 842 may be attached to the y-connector 810 with a luer fitting. The y-connector 810 may comprise a first female luer 851 which communicates with a catheter supply lumen (as in the catheter 118 of FIGS. 4, 8-11), and a second female luer 855 which communicates with a catheter aspiration lumen (as in catheter 118 of FIGS. 4, 8-11).

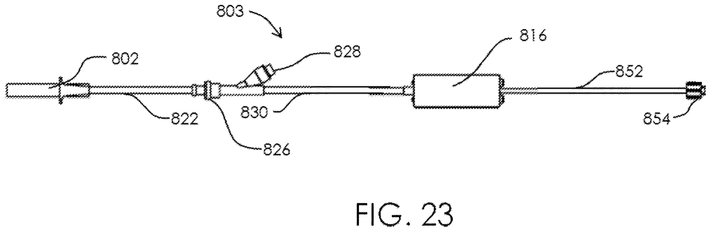

Turning to FIG. 23, the tubing set 803 is shown in more detail. A spike 802 for coupling to a fluid source 20 (FIG. 1) allows fluid to enter through extension tubing 822 and a check valve 826, and into supply tube 830. An optional injection port 828 allows injection of materials or removal of air, as described in relation to previous embodiments. A cassette 816 is used in conjunction with the pump base 200, and is similar in structure and function to the cassette 116 in FIGS. 15-16. Fluid is pumped into injection tube 852 from cassette 816. A male luer 854 is configured to attach to the female luer 851 of the y- connector 810.

Returning to FIG. 21, accessories 857 are illustrated that are intended for applying a vacuum source 22, including a syringe 849 having a plunger 867, to the catheter 818. The syringe 849 is attached to syringe extension tubing 859 via the luer 865 of the syringe 849. A stopcock 847 may be used to hold maintain the vacuum, or the plunger 867 may be a locking variety of plunger. A luer 861 of the syringe extension tubing 859 is connected to an pressure transducer 806, the pressure transducer 806 having a male luer 863 for connection to a connector (e.g., female luer) 804 of vacuum line 808. A male luer 853 at the end of the vacuum line 808 may be detachably secured to the female luer 855 of the y-connector 810 of the aspiration catheter 818. Signals from the pressure transducer 806 are carried through cable 812 to a connector 814. The connector 814 is plugged into the socket 308 (FIG. 12) of the pump base 200. Pressure related signals may be processed by the circuit board 304 of the pump base 200. The pressure transducer 806 may be power from the pump base 200, via cable 812. The accessories 857 may also be supplied sterile to the user.

In use, the pump base 200 resides outside the sterile field. Because operation of the pump base 200 may be controlled by the presence or absence of a pressure, a user who is working in the sterile field may turn the pump on or off without touching the non- sterile pump base 200. For example, the pump may be started by placing a vacuum on the system (e.g., pulling the plunger 867 of the syringe 849). The pump may in turn be stopped by removing the vacuum on the system (unlocking the plunger 867 of the syringe 849 and allowing to release, or opening the stopcock 847). The syringe 849 or the combination syringe 849 and stopcock 847 may act as a sterile on/off button of the pump vase 200. Alternatively, the aspiration catheter 818 may be initially used without the pump base 200, with only aspiration being applied to the aspiration lumen. If in certain cases, if the aspiration lumen becomes clogged, the distal end 820 of the aspiration catheter 818 may be backed off of the thrombus, and the pump base 200 and tubing set 803 may be coupled to the aspiration catheter 818, to then operate with forced saline injection, for increased aspiration, and clear the aspiration lumen. This will also help stop any thrombus that is blocking the aspiration lumen from being inadvertently delivered into the blood vessel of the patient.

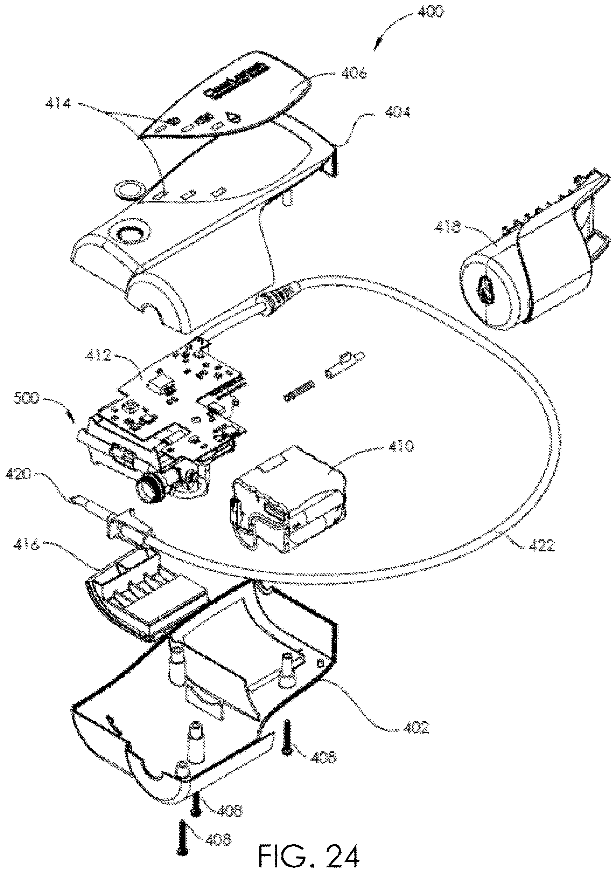

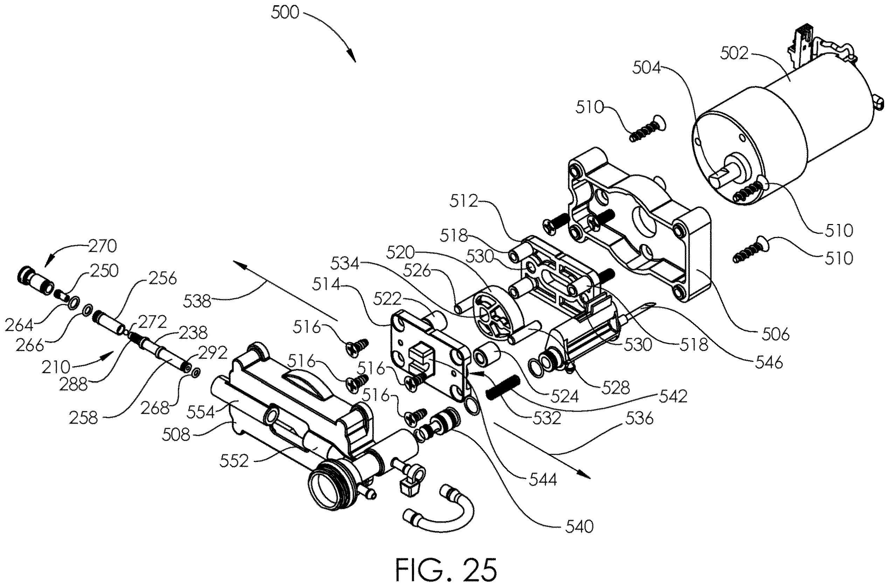

FIGS. 24 and 25 illustrate a saline pump drive unit 400 having a completely disposable pump head 500. The saline pump drive unit 400 is configured to be usable with the catheters 16, 118 described herein, or other embodiments of aspiration systems comprising fluid injection. In FIG. 24, a bottom case 402 and a top case 404 having a label 406 are secured together with screws 408. Contained within the bottom case 402 and top case 404 are a battery pack 410 and an electronic control module 412. A battery cover 416 holds the battery pack 410 in place. In some embodiments, the battery pack 410 may supply a voltage of 18 Volts DC, but systems utilizing other voltages are possible. A user interface 414 enables operation of the saline pump drive unit. A vacuum bottle sleeve 418 may be used when a vacuum bottle is incorporated as the vacuum source 22. A spike 420 is connectable to a fluid source 20, and fluid injectate passes from the fluid source 20 through extension tubing 422 to a disposable piston pump head 500. Saline may be primed through the system by an automatic priming ("self-priming") system described herein in relation to prior embodiments, or may be primed by gravity from a saline bag that is located (for example on an IV pole) above the rest of the system. A valve on the lowest portion of the system may be opened in order to prime the entire system.

As illustrated in FIG. 25, the disposable piston pump head 500 is configured to couple to a motor shaft 504 of a motor 502, that is powered by the battery pack 410 of the saline pump drive unit 400. A motor plate 506 and a main body 508 of the disposable piston pump head 500 are secured to each other with screws 510, and hold the internal components of the disposable piston pump head 500. First and second follower plates 512, 514 are held together with screws 516 and bosses 518 extending from the first follower plate 512. The first and second follower plates 512, 514 rotatably hold a cam 520. The cam may be asymmetric (as illustrated) or alternatively may be symmetric. The asymmetry may be incorporated in order to control the amount of noise in the pump, the contours serving to customize the shape of the pressure wave, and of the function of the pump. First and second bushings 522, 524 are rotatably held on first and second pins 526, 528. The pins 526, 528 insert into cylindrical cavities 530, 532 in each of the follower plates 512, 514.

In use, a user attaches the disposable piston pump head 500 to the motor 502 of the saline pump drive unit 400 by bringing the motor plate 506 close to the motor shaft 504 so that a d-shaped hole 534 in the cam 520 can be pressed over the d-shaped motor shaft 504. Alternatively, the d-shapes may be other non-circular shapes, including, but not limited to elliptical, oval, or rectangular. In operation the motor 502 turns the motor shaft 504, which in turn turns the cam 520. The cam 520 turns, forcing the bushings 522, 524 to push the first and second follower plates 512, 514 back and forth in a first direction 536 and a second direction 538. A saddle 544 is carried on the second follower plate 514, and a piston 210 may be coupled to the saddle 544 in the same manner as described herein with other embodiments. A supply cylinder 552 and an injection cylinder 554 in the main body 508 are analogous to the supply cylinder 252 and injection cylinder 254 of the cassette 116 of the system 100. The piston 210 of the cassette 116 may be used in the disposable piston pump head 500. The labelled components related to the piston 210 in FIG. 25 are similar to those described in relation to the piston 210 in FIGS. 15 and 16. The outer diameter of the cam 520 may be sized and/or shaped to control the stroke length of the piston 210 and the amount of pulsatility (i.e., the difference between the high and low pressure). In some cases, decreasing the stroke length decreases the amount of pulsatility. In applications within the heart, such as coronary artery applications, lowering the amount of pulsatility can reduce the incidence of bradycardia. To compensate for a lower stroke length, and to maintain a sufficient total flow rate, the speed of the rotation of the cam (i.e. rotations per minute), can be increased, for example by increasing motor output speed, either by gearing or by increased applied voltage. In some embodiments, it may be desired to control the pulsatility in order to tailor the size of the pieces of thrombus that are being cut by the fluid jet 178 (FIG. 11). A pulse frequency of 250 pulses per minute (4.167 Hz) or more can be effective in insuring that the pieces of thrombus cut by the fluid jet are relatively small, and that the feed of these pieces through the aspiration lumen 160 during aspiration is adequate such that clogging does not tend to occur. A vacuum spike 546 is used for coupling to the vacuum source 22, for example a vacuum bottle held within the vacuum bottle sleeve 418. A vacuum switch valve 540, which is activated against the bias of a spring 542, may be used to allow pump activation. For example, the electronic control module 412 may be configured to initiate the operation of the motor 502 automatically when the vacuum switch valve 540 sends a signal corresponding to movement of the vacuum switch valve 540, which occurs when a significant vacuum is achieved. This control may be instead of or in addition to control from a vacuum pressure transducer, such as pressure transducer 106. The turning on of the vacuum may thus be used to simultaneously turn on the motor 502, so that a single input begins the operation of the saline pump drive unit 400. Additionally, a vacuum source 22 may be controlled by the electronic control module 412 (for example, by opening or closing a solenoid), when a minimum injectate pressure is measured by an additional pressure transducer. For example, when a pressure of about 0.62 megapascal (90 pounds per square inch) or greater is measured, the vacuum may be activated or communicated to the system. An advantage of the saline pump drive unit 400 is that the user is required only to assemble a single component onto the shaft 504 of the motor 502.

As previously described, the systems according to any of the embodiments of the present disclosure may be configured such that active flow of saline (or other) injectate is not possible without concurrent vacuum being applied for aspiration. Also, the systems may be configured such aspiration is not possible without saline (or other) injectate flow. The systems according to any of the embodiments of the present disclosure may be configured such that current driving the pump (for example the current driving the motor 302, 502) is monitored, or by any alternative monitoring method, such that when a change in condition occurs, for example, air in the injection system, or clogs in any of the catheter lumens or extension tubes, or leaks within the system, the system shuts down, in order to avoid events such as injection of air into the blood vessels, or catheter or system failure.

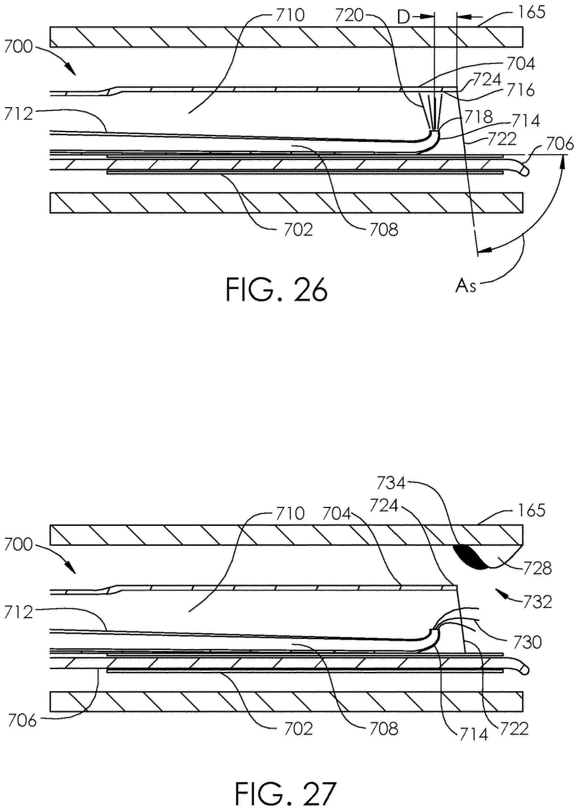

FIG. 26 illustrates an aspiration catheter 700 inserted within a blood vessel 165. The aspiration catheter 700 includes a guidewire lumen 702 secured to the distal end 704 of the aspiration catheter 700 which allows the aspiration catheter 700 to be tracked over a guidewire 706. A supply lumen 708 is secured within an aspiration lumen 710. The supply lumen 708 extends through a tapering tube 712. In some embodiments, the tapering tube 712 may be constructed of polyimide. In some embodiments, the tapering tube 712 may have a luminal inner diameter that tapers from its proximal end to its distal end. For example, in some embodiments, the luminal inner diameter may taper from about 0.3937 mm (0.0155 inches) to about 0.2794 mm (0.011 inches). The supply lumen 708 extends generally parallel to the aspiration lumen 710, however a distal end 714 of the tapering tube 712 curves towards an interior wall surface 716 of the aspiration lumen 710, thus allowing an open end 718 of the supply lumen 708 to act as an orifice for applying a spray pattern 720. The open end 718 of the supply lumen 708 may further promote a jet or spray effect by having an internal diameter that is less than about 0.203 mm (0.008 inches). In some embodiments, the open end 718 of the supply lumen 708 may have an internal diameter that is between about 0.076 mm (0.003 inches) and about 0.102 mm (0.004 inches). The center of the open end 718 orifice may in some embodiments be about 0.3302 mm (0.013 inches) to about 0.4826 mm (0.019 inches) proximal to the most proximal portion 724 of the open distal end 722 of the aspiration lumen 710, as illustrated by distance D in FIG. 26. The most distal portion 726 of the open distal end 722 of the aspiration lumen 710 is slightly distal of the most proximal portion 724 in the embodiment illustrated, and thus has an angled skive, but the skive angle Asis not severe. A skive angle Asof between about 75° and about 89°, or between about 80° and about 85° may be used, in order to allow a large portion of thrombus being pulled into the open distal end 722 of the aspiration lumen 710 to be struck by high velocity exiting jet (e.g. saline) flow, as illustrated with the spray pattern 720.

FIG. 27 illustrates the catheter 700 of FIG. 26 being utilized to deliver a drug 730 to a target site 732 within a blood vessel 165. The target site 732 may include an atherosclerotic lesion 728 and/or a thrombus 734. Whereas the aspiration of thrombus, as in FIG. 26, involves actively applying a vacuum (e.g., from a vacuum source) on the aspiration lumen 710, the drug delivery illustrated in FIG. 27, though utilizing the same catheter 700, allows the metering of a fine, precision volume flow rate of drug 730 to be delivered into the vessel. This is achieved by having significantly less vacuum applied to the aspiration lumen 710, or no vacuum applied to the aspiration lumen. The precision metering in small, controlled volumes, provides efficient use of typically expensive drugs, with minimal wasted drug. In addition, the relatively small volume, or dead space, of the supply lumen 708, because of its relatively small diameter, assures that upon stopping the infusion of a drug 730, very little volume of inadvertent injection is even possible.

In some embodiments, the drug 730 may be delivered at body temperature. In other embodiments, the drug 730 may be warmed, and delivered at an elevated temperature, for example, to increase the activity and effectiveness of a drug. This may be done, for example, to get a more effective dose, with a smaller volume of drug. In other embodiments, the drug 730 may be cooled and delivered at a reduced temperature (i.e., in relation to the body temperature). The drug 730 may be cooled to control the activity level, or to delay the activity of the drug (e.g., so that it is active downstream, at a location that is not reachable by the catheter 700). In some cases, the drug 730 may be cooled in order to apply a conjunctive therapeutic cooling effect on the tissue being treated. In some cases, the therapeutic cooling effect may be achieved from cooled saline or other aqueous non-drug media alone.

Some of the drugs 730 which may be delivered include thrombolytic agents (clot busting drugs), such as streptokinase, tissue plasminogen activator (t-PA), recombinant or genetically-engineered tissue plasminogen activator, tenecteplase (TNK), urokinase, staphylokinase, and reteplase. Alternatively, stem cells or "cocktails" containing stem cells may be delivered. In some cases, glycoprotein inhibitos (GPI's) may be injected through the supply lumen 708 of the aspiration catheter 700. Saline or other aqueous solutions may be delivered alone for selective dilution of blood at the target site 732. In some applications, a solution may be used which is capable of exhibiting a phase change, for example, when its pressure or temperature is changed. In these applications, a liquid may be injected that becomes a gas when exiting from a small orifice, for example at the open end 718 of the supply lumen 708. Alternatively, a gas may be injected that becomes a liquid when being force through a small orifice, such as the open end 718 of the supply lumen 708. In any of the applications in which drugs 730 or other materials are injected intravascularly through the catheter 700, the injection of the drugs 730 or other materials may occur before, during, after, or instead of an aspiration procedure. Returning to the aspiration catheter 818 of FIGS 21-22, if, during an aspiration procedure, it is desired to deliver drugs down the supply lumen and into the vessel, the tubing set 803 may be removed from the aspiration catheter 818 by disconnecting the male luer 854 of the tubing set 803 from the female luer 851 of the aspiration catheter 818, and the drug may be injected directly into the supply lumen at the female luer 851, for example, by a syringe or metering system, including a syringe/syringe pump combination. By also removing the vacuum source from the female luer 855 of the aspiration catheter 818, when aspiration lumen now serves as an overflow, so that the fluid being delivered into the patient (e.g., intravascularly) is maintained at a controlled rate. The volume of the supply lumen is relatively very small, so only a small volume of drug is needed to fill the supply lumen, and thus reach the distal top of the aspiration catheter 818. This, at the end of the procedure, very little drug is wasted, or needs to be disposed, allowing for a very cost-effective procedure.

In the embodiments described herein, a sterile fluid path is provided extending all the way from the fluid source 20 to the distal opening 40/open distal end 158 of the catheter 16, 118. In both the embodiments of the system 100 of FIGS. 4-17, the system 800 of FIGS. 21-23, and the embodiments of FIGS. 24-25, a disposable catheter and disposable pump set are configured to be supplied sterile, and coupled to a non-sterile (reusable) pump base 200 or pump motor 502. These combinations allow for reusability of the more expensive components, and for reusability (and maximized sterility) of the less expensive components, thus maximizing cost containment and patient safety at the same time.

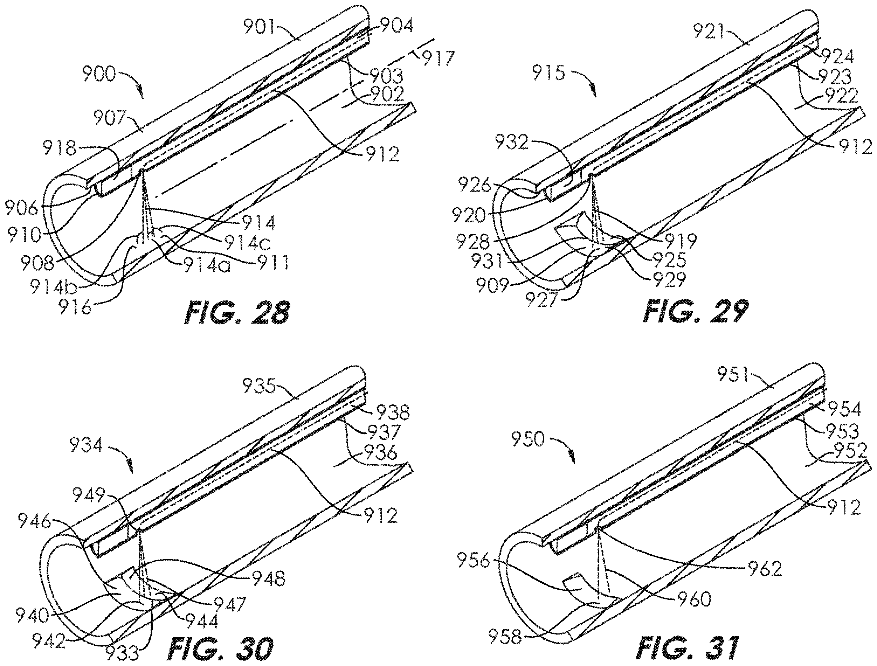

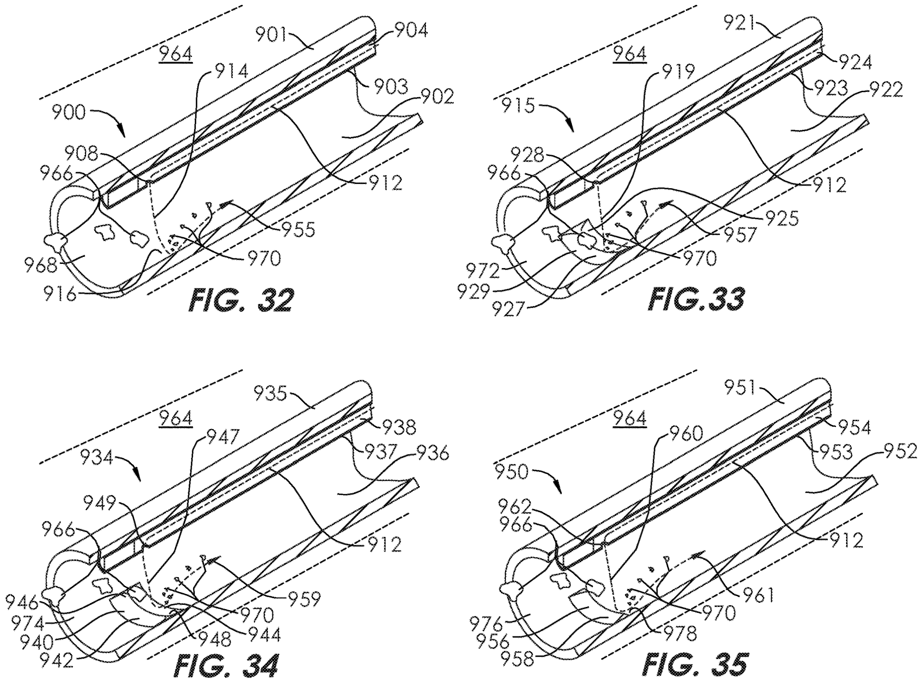

FIG. 28 illustrates an aspiration catheter 900 including a shaft 901 having an aspiration lumen 902 and a supply tube 903 having a supply lumen 904 (high pressure lumen). The supply tube 903 is secured to an inner wall 906 of the shaft 901, for example, by adhesive, epoxy, mechanical securement, or thermal bonding or tacking. The supply lumen 904 is configured to carry pressurized fluid 912, which may include saline, lytic (thrombolytic) agents, contrast agents, or other agents. In use, the pressurized fluid 912 exits in a spray pattern 914 from an orifice 908 adjacent the distal end 910 of the supply lumen 904, impinging against an interior wall surface 916 of the aspiration lumen 902. The agent or agents may be undiluted or may be diluted (e.g., with saline). A jet spray impact 911 against the interior wall surface 916 may form a distal component and/or a proximal component, as described in further detail in FIGS. 32, 36, and 40. The distal component or proximal component may be substantially distally-oriented or substantially proximally-oriented, in part or in whole, because of factors such as: the particular level of positive pressure of the pressurized fluid 912 within the supply lumen 904, or because of the particular geometry of the orifice 908, or because of the particular level of negative pressure on the aspiration lumen 902, or because of the particular geometry of the interior wall surface 916, separately, or in any type of combination. A pump, syringe, or other source of pressurization may be coupled to the proximal end of the supply lumen 904, to allow pressurization or pulsation of the supply lumen 904. In some embodiments, the pump base 200 (FIG. 12) may be used to supply and pressurize the supply lumen 904 with the fluid 912. The supply tube 903 includes a plug 918 which blocks the end of the supply lumen 904, forcing pressurized fluid 912 through the orifice 908 and into the aspiration lumen 902, and, when operated to supply sufficient pressure, against the interior wall surface 916.

The spray pattern 914 may be directed by the orifice 908 toward the interior wall surface 916 perpendicularly (i.e., at a 90° angle) 914a in relation to the longitudinal axis 917 of the aspiration catheter 900 and/or may impact the interior wall surface 916 at an oblique angle that is distally-oriented 914b or an oblique angle that is proximally- oriented 914c. The spray pattern 914 may comprise two or three of these elements 914a, 914b, 914c together.

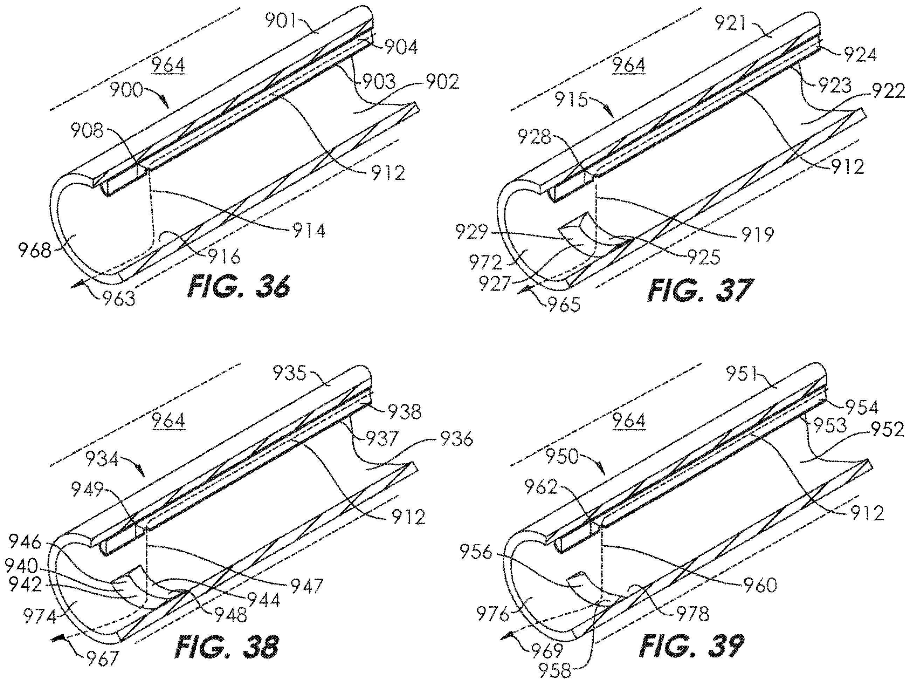

An alternative embodiment of an aspiration catheter 915 is illustrated in FIG. 29, and includes a shaft 921 having an aspiration lumen 922 and a supply tube 923 having a supply lumen 924 (high pressure lumen). The supply tube 923 is secured to an inner wall 926 of the shaft 921. The supply lumen 924 is configured to carry pressurized fluid 912, which may include saline, lytic (thrombolytic) agents, contrast agents, or other agents. The agent or agents may be undiluted or may be diluted (e.g., with saline). The pressurized fluid 912 exits in a spray pattern 919 from an orifice 928 adjacent the distal end 920 of the supply lumen 924 and impinges against an interior wall surface 909 of the aspiration lumen 922. The interior wall surface 909 includes an additional element 929 (e.g., deflection element) which is configured for deflecting at least a portion of the spray pattern 919 either proximally or distally. The deflection element 929 includes a forward ramp 927 and a reverse ramp 925 which converge at a dividing line 931. The forward ramp 927 is configured to deflect at least a portion of the spray pattern 919 distally and the reverse ramp 925 is configured to deflect at least a portion of the spray pattern 919 proximally. A jet spray impact against the interior wall surface 909 may include a distal component and/or a proximal component, as described in further detail in FIGS. 33 and 37. In other embodiments, the interior wall surface 909 may simply be a deformation of a portion of the inner wall 926 itself. The deformation may take the place of the deflection element 929 and thus act as the deflection element 929. The deformation may an angulation or formation of the distal end 907 of the aspiration catheter 900 that causes the inner wall 926 to have, for example, one or more ramps or angled, or curvilinear surfaces.

A distal component or proximal component may be substantially distally- oriented or substantially proximally-oriented in part or in whole because of factors such as: the particular level of positive pressure of the pressurized fluid 912 within the supply lumen 924, or because of the particular geometry of the orifice 928, or because of the particular level of negative pressure on the aspiration lumen 922, or because of the particular geometry of the interior wall surface 909, separately, or in any type of combination. A pump, syringe, or other source of pressurization may be coupled to the proximal end of the supply lumen 924, to allow pressurization or pulsation of the supply lumen 924. The supply tube 923 includes a plug 932 which blocks the distal end 920 of the supply lumen 924, forcing pressurized fluid 912 through the orifice 928 and into the aspiration lumen 922 and, when operated to supply sufficient pressure, against the interior wall surface 909 comprising ramps 925, 927. In some embodiments, a portion of the spray pattern 919 that strikes the forward ramp 927 is deflected distally. In some embodiments, a portion of the spray pattern 919 that strikes the reverse ramp 925 is deflected proximally. In some embodiments, the specific amount of negative pressure being applied on the aspiration lumen 922 (e.g., by a vacuum source) controls how much of the spray pattern 919 impinges upon each of the ramps 925, 927.

In the aspiration catheter 915 of FIG. 29, the ramps 925, 927 of the element 929 extend from the dividing line 931 in a linear fashion, wherein the effective inner radius of the aspiration lumen changes linearly in relation to the longitudinal location along the ramp 925, 927. In contrast, FIG. 30 illustrates an aspiration catheter 934 having non-linear ramps 942, 944 (e.g., curvilinear) extending between a dividing line 933. The aspiration catheter 934 includes a shaft 935 having an aspiration lumen 936 and a supply tube 937 having a supply lumen 938 (high pressure lumen). The aspiration catheter 934 further includes a deflection element 940 with ramps 942, 944 that each include a concave contour 946, 948, such that the effective inner radius of the aspiration lumen changes non-linearly in relation to the longitudinal location along the ramp 942, 944. In some embodiments, the deflection element 940 may be configured for directing and/or deflecting a spray pattern 947 (emanating from orifice 949) that is narrow and/or that comprises a jet. In other embodiments, the deflection element 929 of the aspiration catheter 915 of FIG. 29 may be configured for directing and/or deflecting a spray pattern 919 that is wider or which significantly diverges or spreads.

FIG. 31 illustrates an aspiration catheter 950 which includes a shaft 951 having an aspiration lumen 952 and a supply tube 953 having a supply lumen 954 (high pressure lumen). The aspiration catheter 950 further includes a deflection element 956 with a single distally-oriented ramp 958 which is configured to deflect at least a portion of a spray pattern 960 emanating from an orifice 962 in a substantially distal direction.

FIG. 32 illustrates the aspiration catheter 900 of FIG. 28 in use within a blood vessel 964 as part of an aspiration system 10 or system for aspirating thrombus 100, 800. FIG. 32 illustrates the aspiration catheter 900 in a first mode of operation configured to cause substantial aspiration of thrombi 966. A venturi effect is created by the spray pattern 914, which may comprise a jet. Suction is thus created at the distal opening 968 of the aspiration lumen 902 causing the thrombi 966 to be aspirated into the aspiration lumen 902. In addition, an aspiration pressure (negative pressure) may be applied at a proximal end of the aspiration lumen 902 (e.g., with a vacuum source, such as a syringe, vacuum chamber or vacuum pump), thus maintaining the flow of the thrombi 966 through the aspiration lumen 902. The impingement of the spray pattern 914 of the pressurized fluid 912 against the interior wall surface 916 of the aspiration lumen 902, opposite the orifice 908, may also macerate the thrombi 966 into smaller pieces 970 which can help to lower the effective viscosity of the composite fluid flowing through the aspiration lumen 902. By applying a significant vacuum/aspiration pressure on the proximal end of the aspiration lumen 902, the removal of thrombi 966 and any smaller pieces 970 of thrombi 966 can be optimized. The spray pattern 914 is at least partially diverted into a substantially proximally-oriented flow 955 after impingement upon the interior wall surface 916.

FIG. 33 illustrates the aspiration catheter 915 of FIG. 29 in use within a blood vessel 964 as part of an aspiration system 10 or system for aspirating thrombus 100, 800. FIG. 33 illustrates the aspiration catheter 915 in a first mode of operation configured to cause substantial aspiration of thrombi 966. A venturi effect is created by the spray pattern 919, which may comprise a jet. Suction is thus created at the distal opening 972 of the aspiration lumen 922 causing the thrombi 966 to be aspirated into the aspiration lumen 922. In addition, an aspiration pressure (negative pressure) may be applied at a proximal end of the aspiration lumen 922 (e.g., with a vacuum source, such as a syringe, vacuum chamber or vacuum pump), thus maintaining the flow of the thrombi 966 through the aspiration lumen 922. The impingement of the spray pattern 919 of the pressurized fluid 912 against the reverse ramp 925 of the deflection element 929, opposite the orifice 928, may also macerate the thrombi 966 into smaller pieces 970 which can help to lower the effective viscosity of the composite fluid flowing through the aspiration lumen 902. By applying a significant vacuum/aspiration pressure on the proximal end of the aspiration lumen 922, the removal of thrombi 966 and any smaller pieces 970 of thrombi 966 can be optimized. The spray pattern 919 is at least partially diverted into a substantially proximally-oriented flow 957 after impingement upon the reverse ramp 925 of the deflection element 929.

FIG. 34 illustrates the aspiration catheter 934 of FIG. 30 in use within a blood vessel 964 as part of an aspiration system 10 or system for aspirating thrombus 100, 800. FIG. 34 illustrates the aspiration catheter 934 in a first mode of operation configured to cause substantial aspiration of thrombi 966. A venturi effect is created by the spray pattern 947, which may comprise a jet. Suction is thus created at the distal opening 974 of the aspiration lumen 936 causing the thrombi 966 to be aspirated into the aspiration lumen 936. In addition, an aspiration pressure (negative pressure) may be applied at a proximal end of the aspiration lumen 936 (e.g., with a vacuum source, such as a syringe, vacuum chamber or vacuum pump), thus maintaining the flow of the thrombi 966 through the aspiration lumen 936. The impingement of the spray pattern 947 of the pressurized fluid 912 against the reverse ramp 944 of the deflection element 940, opposite the orifice 949, may also macerate the thrombi 966 into smaller pieces 970 which can help to lower the effective viscosity of the composite fluid flowing through the aspiration lumen 936. By applying a significant vacuum/aspiration pressure on the proximal end of the aspiration lumen 936, the removal of thrombi 966 and any smaller pieces 970 of thrombi 966 can be optimized. The spray pattern 947 is at least partially diverted into a substantially proximally-oriented flow 959 after impingement upon the reverse ramp 944 of the deflection element 940.

FIG. 35 illustrates the aspiration catheter 950 of FIG. 31 in use within a blood vessel 964 as part of an aspiration system 10 or system for aspirating thrombus 100, 800. FIG. 35 illustrates the aspiration catheter 950 in a first mode of operation configured to cause substantial aspiration of thrombi 966. A venturi effect is created by the spray pattern 960, which may comprise a jet. Suction is thus created at the distal opening 976 of the aspiration lumen 952 causing the thrombi 966 to be aspirated into the aspiration lumen 952. In addition, an aspiration pressure (negative pressure) may be applied at a proximal end of the aspiration lumen 952 (e.g., with a vacuum source, such as a syringe, vacuum chamber or vacuum pump), thus maintaining the flow of the thrombi 966 through the aspiration lumen 952. The impingement of the spray pattern 960 of the pressurized fluid 912 against the interior wall surface 978 which is proximal to the deflection element 956, opposite the orifice 962, may also macerate the thrombi 966 into smaller pieces 970 which can help to lower the effective viscosity of the composite fluid flowing through the aspiration lumen 952. By applying a significant vacuum/aspiration pressure on the proximal end of the aspiration lumen 952, the removal of thrombi 966 and any smaller pieces 970 of thrombi 966 can be optimized. The spray pattern 960 is at least partially diverted into a substantially proximally-oriented flow 961 after impingement upon the interior wall surface 978 which is proximal to the deflection element 956.

FIG. 36 illustrates the aspiration catheter 900 of FIG. 28 in use within a blood vessel 964 as part of an aspiration system 10 or system for aspirating thrombus 100, 800. FIG. 36 illustrates the aspiration catheter 900 in a second mode of operation configured to deliver a fluid (such as a fluid comprising an agent) distally out the distal opening 968 of the aspiration lumen 902. The impingement of the spray pattern 914 of the pressurized fluid 912 against the interior wall surface 916 of the aspiration lumen 902, opposite the orifice 908, at least partially diverts the spray pattern 914 into a substantially distally- oriented flow 963. In addition, an aspiration pressure (negative pressure) may be reduced, completely stopped, or simply not applied at a proximal end of the aspiration lumen 902, thus allowing at least some of the spray pattern 914 to transform into the substantially distally-oriented flow 963 after impingement upon the interior wall surface 916. In some embodiments, the orifice 908 and/or the interior wall surface 916 may be configured such that in some conditions, the substantially distally-oriented flow 963 may itself be a jet. The agent may comprise a lytic agent, such as a thrombolytic agent, or may comprise a contrast agent. The substantially distally-oriented flow 963 may comprise 50% or more of the spray pattern 914 (upon deflection), or 60% or more, or 70% or more, or 80% or more, or 90% or more, or even 100%.

FIG. 37 illustrates the aspiration catheter 915 of FIG. 29 in use within a blood vessel 964 as part of an aspiration system 10 or system for aspirating thrombus 100, 800. FIG. 37 illustrates the aspiration catheter 915 in a second mode of operation configured to deliver a fluid (such as a fluid comprising an agent) distally out the distal opening 972 of the aspiration lumen 922. The impingement of the spray pattern 919 of the pressurized fluid 912 against the forward ramp 927 of the deflection element 929, opposite the orifice 928, at least partially diverts the spray pattern 919 into a substantially distally-oriented flow 965. In addition, an aspiration pressure (negative pressure) may be reduced, completely stopped, or simply not applied at a proximal end of the aspiration lumen 922, thus allowing at least some of the spray pattern 919 to transform into the substantially distally-oriented flow 965 after impingement upon the forward ramp 927 of the deflection element 929. In some embodiments, the orifice 928 and/or the forward ramp 927 of the deflection element 929 may be configured such that in some conditions, the substantially distally-oriented flow 965 may itself be a jet. The agent may comprise a lytic agent, such as a thrombolytic agent, or may comprise a contrast agent.

FIG. 38 illustrates the aspiration catheter 934 of FIG. 30 in use within a blood vessel 964 as part of an aspiration system 10 or system for aspirating thrombus 100, 800. FIG. 38 illustrates the aspiration catheter 934 in a second mode of operation configured to deliver a fluid (such as a fluid comprising an agent) distally out the distal opening 974 of the aspiration lumen 936. The impingement of the spray pattern 947 of the pressurized fluid 912 against the forward ramp 942 of the deflection element 940, opposite the orifice 949, at least partially diverts the spray pattern 947 into a substantially distally-oriented flow 967. In addition, an aspiration pressure (negative pressure) may be reduced, completely stopped, or simply not applied at a proximal end of the aspiration lumen 936, thus allowing at least some of the spray pattern 947 to transform into the substantially distally-oriented flow 967 after impingement upon the forward ramp 942 of the deflection element 940. In some embodiments, the orifice 949 and/or the forward ramp 942 of the deflection element 940 may be configured such that in some conditions, the substantially distally-oriented flow 967 may itself be a jet. The agent may comprise a lytic agent, such as a thrombolytic agent, or may comprise a contrast agent.

FIG. 39 illustrates the aspiration catheter 950 of FIG. 31 in use within a blood vessel 964 as part of an aspiration system 10 or system for aspirating thrombus 100, 800. FIG. 39 illustrates the aspiration catheter 950 in a second mode of operation configured to deliver a fluid (such as a fluid comprising an agent) distally out the distal opening 976 of the aspiration lumen 952. The impingement of the spray pattern 960 of the pressurized fluid 912 against the distally-oriented ramp 958 of the deflection element 956, opposite the orifice 962, at least partially diverts the spray pattern 960 into a substantially distally- oriented flow 969. In addition, an aspiration pressure (negative pressure) may be reduced, completely stopped, or simply not applied at a proximal end of the aspiration lumen 952, thus allowing at least some of the spray pattern 960 to transform into the substantially distally-oriented flow 969 after impingement upon the distally-oriented ramp 958 of the deflection element 956. In some embodiments, the orifice 962 and/or the distally-oriented ramp 958 of the deflection element 956 may be configured such that in some conditions, the substantially distally-oriented flow 969 may itself be a jet. The agent may comprise a lytic agent, such as a thrombolytic agent, or may comprise a contrast agent.

The delivery of an agent comprising a drug using the second mode of operation described in FIGS. 36-39 in relation to aspiration catheters 900, 915, 934, 950 may be achieved in a precise manner which allows for correct dosage, without wasting often- expensive drugs. The small inner diameter of transverse internal dimension of the supply lumen 904, 924, 938, 954 not only allows for precision and small volume introduction of the agent, but also avoids unwanted loss of agent when it is desired to suddenly stop injection. This is a significant improvement over standard, gravity -fed injection systems. In addition, the use of the pump base 200 (FIG. 12) to pressurize the supply lumen 904, 924, 938, 954 to deliver the agent adds additional precision, control, and lack of waste. This decreases the cost of a procedure, increases the accuracy of the drug treatment (or, for example, contrast delivery), and may also speed up the procedure, because of fewer errors to correct or steps to repeat. This in itself may be another element for saving cost. Though the word "aspiration" is used in defining the aspiration lumen 902, 922, 936, 952 and the aspiration catheters 900, 915, 934, 950, it should be apparent that a user may choose to use the aspiration catheters 900, 915, 934, 950 in the second mode only, as described in relation to FIGS. 36-39, and may in some cases choose to do so without any aspiration whatsoever.

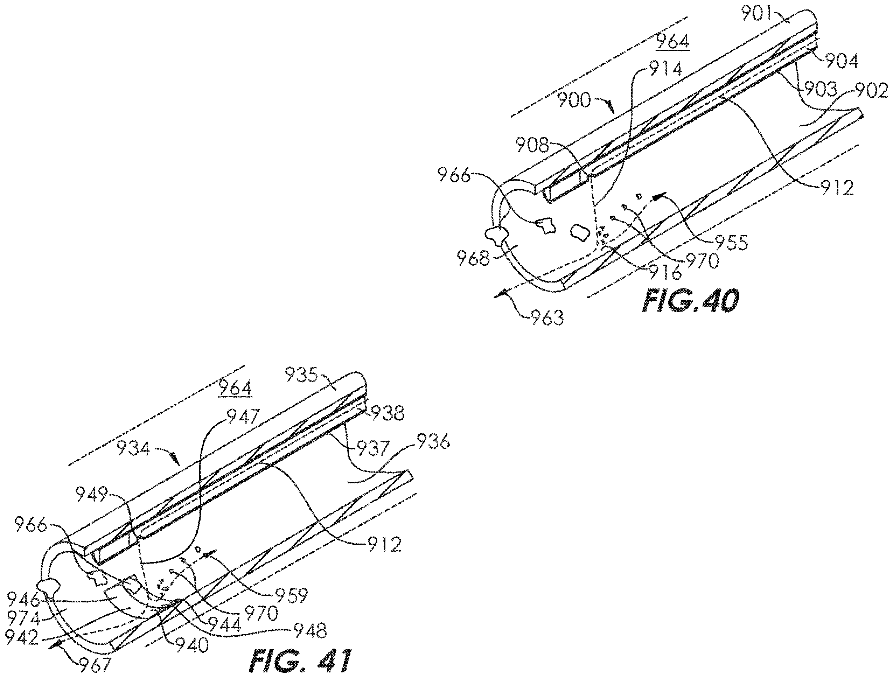

FIG. 40 illustrates the aspiration catheter 900 of FIG. 28 in use within a blood vessel 964 as part of an aspiration system 10 or system for aspirating thrombus 100, 800. FIG. 40 illustrates the aspiration catheter 900 in a third mode of operation configured to deliver a fluid (such as a fluid comprising an agent) distally out the distal opening 968 of the aspiration lumen 902 while also causing at least some aspiration of thrombi 966. The impingement of the spray pattern 914 of the pressurized fluid 912 against the interior wall surface 916 of the aspiration lumen 902, opposite the orifice 908, at least partially splits the spray pattern 914 into a substantially distally-oriented flow 963 and a substantially proximally-oriented flow 955. An aspiration pressure (negative pressure) may be applied, adjusted, increased, or reduced at a proximal end of the aspiration lumen 902, thus allowing at least some of the spray pattern 914 to transform into the substantially distally- oriented flow 963 after impingement upon the interior wall surface 916 and at least some of the spray pattern 914 to transform into the substantially proximally-oriented flow 955 after impingement upon the interior wall surface 916. In some embodiments, the orifice 908 and/or the interior wall surface 916 may be configured such that in some conditions, the substantially distally-oriented flow 963 may itself be a jet. The agent may comprise a lytic agent, such as a thrombolytic agent, or may comprise a contrast agent.

FIG. 41 illustrates the aspiration catheter 934 of FIG. 30 in use within a blood vessel 964 as part of an aspiration system 10 or system for aspirating thrombus 100, 800. FIG. 41 illustrates the aspiration catheter 934 in a third mode of operation configured to deliver a fluid (such as a fluid comprising an agent) distally out the distal opening 974 of the aspiration lumen 936 while also causing at least some aspiration of thrombi 966. The impingement of the spray pattern 947 of the pressurized fluid 912 against the ramps 942, 944 of the deflection element 940, opposite the orifice 949, at least partially splits the spray pattern 947 into a substantially distally-oriented flow 967 and a substantially proximally-oriented flow 959. An aspiration pressure (negative pressure) may be applied, adjusted, increased, or reduced at a proximal end of the aspiration lumen 936, thus allowing at least some of the spray pattern 947 to transform into the substantially distally- oriented flow 967 after impingement upon the forward ramp 942 of the deflection element 940 and at least some of the spray pattern 947 to transform into the substantially proximally-oriented flow 959 after impingement upon the reverse ramp 944 of the deflection element 940. In some embodiments, the orifice 949 and/or the forward ramp 942 of the deflection element 940 may be configured such that in some conditions, the substantially distally-oriented flow 967 may itself be a jet. The agent may comprise a lytic agent, such as a thrombolytic agent, or may comprise a contrast agent.

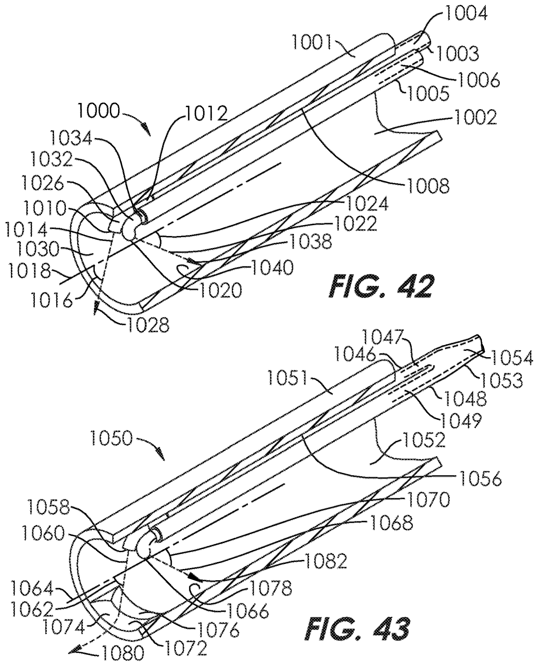

FIG. 42 illustrates an aspiration catheter 1000 including a shaft 1001 having an aspiration lumen 1002, a first supply tube 1003 having a first supply lumen 1004 and a second supply tube 1005 having a second supply lumen 1006. The first supply tube 1003 and second supply tube 1005 are secured to an inner wall 1008 of the shaft 1001. The first supply lumen 1004 is configured to carry pressurized fluid 912, which may include saline, lytic (thrombolytic) agents, contrast agents, or other agents. The pressurized fluid 912 exits a first orifice 1010 of the first supply lumen 1004 in a spray pattern 1014 that is directed at an oblique, distally-oriented angle 1016 with respect to a longitudinal axis 1018 of the aspiration catheter 1000. The second supply lumen 1005 is configured to carry pressurized fluid 912, which may include saline, lytic (thrombolytic) agents, contrast agents, or other agents. The pressurized fluid 912 exits a second orifice 1020 of the second supply lumen 1006 in a spray pattern 1022 that is directed at an oblique, proximally-oriented angle 1024 with respect to the longitudinal axis 1018 of the aspiration catheter 1000. The agent or agents may be undiluted or may be diluted (e.g., with saline).

A first curved hollow tip extension 1026 includes an outer diameter at its proximal end 1012 that is inserted within the first supply lumen 1004 of the first supply tube 1003. The curve of the first curved hollow tip extension 1026 aims the spray pattern 1014 that exits the first orifice 1010 in the oblique, distally-oriented angle 1016 such that a substantially distally-oriented flow 1028 is directed, or oriented, outside the open distal end 1030 of the aspiration lumen 1002. A second curved hollow tip extension 1032 includes an outer diameter at its proximal end 1034 that is inserted within the second supply lumen 1006 of the second supply tube 1005. The curve of the second curved hollow tip extension 1032 aims the spray pattern 1022 that exits the second orifice 1020 in the oblique, proximally-oriented angle 1024 such that a substantially proximally- oriented flow 1038 is oriented towards an inner wall surface 1040 the aspiration lumen 1002. The application and adjustment of a negative pressure on a proximal end of the aspiration lumen 1002 may be used to adjust the extent of aspiration (e.g., of thrombus or blood) and the extent of delivery of an agent distally through the first orifice 1010.

FIG. 43 illustrates an aspiration catheter 1050 including a shaft 1051 having an aspiration lumen 1052, and a first supply tube 1053 having a first supply lumen 1054. The first supply tube 1053 bifurcates into a first tubular branch 1046 having a first branch lumen 1047 and a second tubular branch 1048 having a second branch lumen 1049. The first tubular branch 1046 and second tubular branch 1048 are secured to an inner wall 1056 of the shaft 1051. The first supply lumen 1054, first tubular branch 1046, and second tubular branch 1048 are configured to carry pressurized fluid 912, which may include saline, lytic (thrombolytic) agents, contrast agents, or other agents. The pressurized fluid 912 exits a first orifice 1058 of the first branch lumen 1047 in a spray pattern 1060 that is directed at an oblique, distally-oriented angle 1062 with respect to a longitudinal axis 1064 of the aspiration catheter 1050. The pressurized fluid 912 exits a second orifice 1066 of the second branch lumen 1049 in a spray pattern 1068 that is directed at an oblique, proximally-oriented angle 1070 with respect to the longitudinal axis 1064 of the aspiration catheter 1050. The agent or agents may be undiluted or may be diluted (e.g., with saline). One or more deflection members 1072 having one or more ramps 1074, 1076 (e.g., forward ramp 1074 and reverse ramp 1076) may be carried on an inner wall 1078 of the aspiration lumen 1052 for deflecting one or both spray patterns 1060, 1068 to produce a distally-oriented flow 1080 and/or proximally-oriented flow 1082. In other embodiments, the forward ramp 1074 and/or reverse ramp 1076 may simply be projections of the inner wall 1078, or may be formed by a deflection of the shaft 1001.

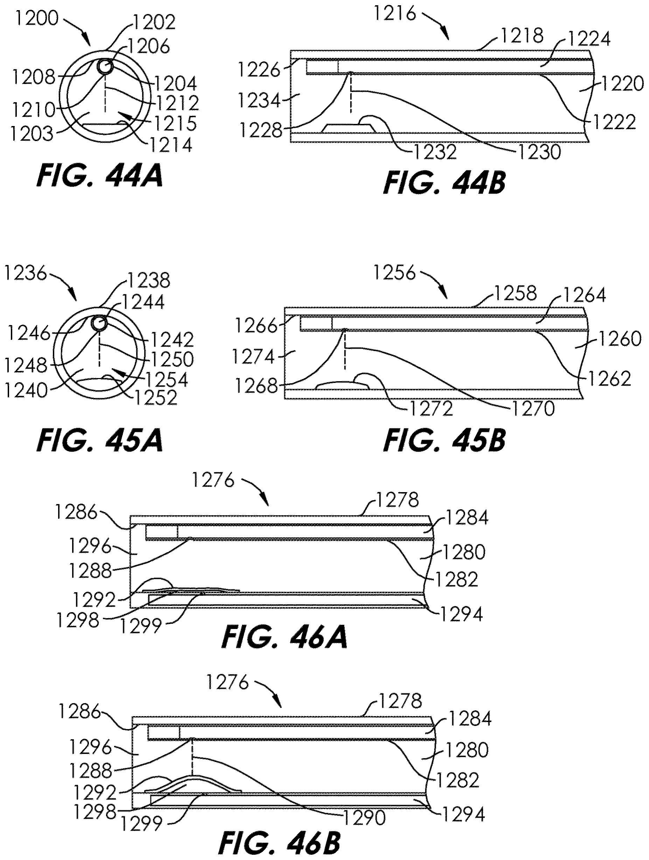

FIG. 44A illustrates a catheter 1200 having a shaft 1202 having a lumen 1203 and a supply tube 1204 having a supply lumen 1206. The supply tube 1204 is secured to an inner wall 1208 of the shaft 1202 and includes an orifice 1210 configured for directing pressurized fluid to exit in a spray pattern 1212, which may form a jet. The spray pattern 1212 is directed against an opposing deflection member 1214 which may either be a separate component secured to the inner wall 1208 of the shaft 1202, or may be a formed portion of the shaft 1202. The lumen 1204 is a guidewire lumen configured for allowing the catheter 1200 to track over the guidewire (not shown). In use, the catheter 1200 is operated as an infusion catheter, and the guidewire may be retracted proximally to the orifice 1210 and deflection member 1214 so that they are able to function with less potential interference. In some cases, the guidewire may be removed entirely. In other embodiments, the lumen 1204 may be an aspiration lumen, configured for aspiration of material such as thrombus or other emboli. The lumen may alternatively have other purposes, for example as a conduit for larger volume injections or infusions. The deflection member 1214 has a flat surface extending transversely, or radially and is configured to deflect the spray pattern 1212. For example, the deflection member 1214 may be configured to deflect the spray pattern 1212 so that at least some of an agent carried by the spray pattern 1212 is urged out of the distal opening 1215 of the lumen 1204.

FIG. 44B illustrates a catheter 1216 including a shaft 1218 having a lumen 1220 and a supply tube 1222 having a supply lumen 1224. The supply tube 1222 is secured to an inner wall 1226 of the shaft 1218 and includes an orifice 1228 configured for directing pressurized fluid to exit in a spray pattern 1230, which may form a jet. The spray pattern 1230 is directed against an opposing deflection member 1232 which may either be a separate component secured to the inner wall 1226 of the shaft 1218, or may be a formed portion of the shaft 1218. The lumen 1220, like the lumen 1203 of the catheter 1200 of FIG. 44A, may be a guidewire lumen and/or an aspiration lumen, or may have other purposes. The deflection member 1232 has a flat surface extending longitudinally, or axially, and is configured to deflect the spray pattern 1230. For example, the deflection member 1232 may be configured to deflect the spray pattern 1230 so that at least some of an agent carried by the spray pattern 1230 is urged out of the distal opening 1234 of the lumen 1220.

FIG. 45A illustrates a catheter 1236 having a shaft 1238 having a lumen 1240 and a supply tube 1242 having a supply lumen 1244. The supply tube 1242 is secured to an inner wall 1246 of the shaft 1238 and includes an orifice 1248 configured for directing pressurized fluid to exit in a spray pattern 1250, which may form a jet. The spray pattern 1250 is directed against an opposing deflection member 1252 which may either be a separate component secured to the inner wall 1246 of the shaft 1238, or may be a formed portion of the shaft 1238. The lumen 1240 is a guidewire lumen configured for allowing the catheter 1236 to track over the guidewire (not shown). In use, the catheter 1236 is operated as an infusion catheter, and the guidewire may be retracted proximally to the orifice 1248 and deflection member 1252 so that they are able to function with less potential interference. In some cases, the guidewire may be removed entirely. In other embodiments, the lumen 1240 may be an aspiration lumen, configured for aspiration of material such as thrombus or other emboli. The lumen may alternatively have other purposes, for example as a conduit for larger volume injections or infusions. The deflection member 1252 has a convex surface when viewed from an end view, and is configured to deflect the spray pattern 1250. For example, the deflection member 1252 may be configured to deflect the spray pattern 1250 so that at least some of an agent carried by the spray pattern 1250 is urged out of the distal opening 1254 of the lumen 1240.

FIG. 45B illustrates a catheter 1256 including a shaft 1258 having a lumen 1260 and a supply tube 1262 having a supply lumen 1264. The supply tube 1262 is secured to an inner wall 1266 of the shaft 1258 and includes an orifice 1268 configured for directing pressurized fluid to exit in a spray pattern 1270, which may form a jet. The spray pattern 1270 is directed against an opposing deflection member 1272 which may either be a separate component secured to the inner wall 1266 of the shaft 1258, or may be a formed portion of the shaft 1258. The lumen 1260 may be a guidewire lumen and/or an aspiration lumen, or may have other purposes. The deflection member 1272 has a convex surface when viewed from the side, and is configured to deflect the spray pattern 1270. For example, the deflection member 1272 may be configured to deflect the spray pattern 1270 so that at least some of an agent carried by the spray pattern 1270 is urged out of the distal opening 1274 of the lumen 1260.

FIGS. 46A and 46B illustrate a catheter 1276 having a shaft 1278 having a lumen 1280 and a supply tube 1282 having a supply lumen 1284. The supply tube 1282 is secured to an inner wall 1286 of the shaft 1278 and includes an orifice 1288 configured for directing pressurized fluid to exit in a spray pattern 1290, which may form a jet. The spray pattern 1290 is directed against an opposing adjustable deflection member 1292 having at least two states, a first state (FIG. 46A) and a second state (FIG. 46B). In the embodiment shown, the adjustable deflection member 1292 comprises a balloon secured to the inner wall 1286 of the shaft 1278 such that it may be inflated or deflated via a fluid passage 1294 within or carried by the shaft 1278. An inflation device with or without a volume measurement device, pressure sensor, and/or pressure gauge may be coupled to a proximal end of the fluid passage 1294, to thus aid in the inflation or deflation of the balloon. The lumen 1280 is a guidewire lumen, configured for allowing the catheter 1276 to track over the guidewire (not shown). In use, the catheter 1276 is operated as an infusion catheter, and the guidewire may be retracted proximally to the orifice 1288 and adjustable deflection member 1292 so that they are able to function with less potential interference. In some cases, the guidewire may be removed entirely. In other embodiments, the lumen 1280 may be an aspiration lumen, configured for aspiration of material such as thrombus or other emboli. The lumen may alternatively have other purposes, for example as a conduit for larger volume injections or infusions.

The adjustable deflection member 1292, in at least one of its two or more states, is configured to deflect the spray pattern 1290. For example, the adjustable deflection member 1292 may be configured to deflect the spray pattern 1290 so that at least some of an agent carried by the spray pattern 1290 is urged out of the distal opening 1296 of the lumen 1280. In a first state displayed in FIG. 46A, the adjustable deflection member 1292 is deflated, or in other words, its interior volume 1298 is substantially empty. This first state may be desired if, for example, passing the catheter 1276 over a guidewire that extends through the lumen 1280, or if aspirating through the lumen 1280 (with or without the guidewire in place). In another version of the first state, a vacuum (negative pressure) may additionally be placed and held on the fluid passage 1294 (e.g., from an evacuated syringe or evacuated locking syringe on the proximal end of the fluid passage 1294) to minimize the profile of the deflated adjustable deflection member 1292 and thus maximize the cross-sectional area of the lumen 1280 in this area. In a second state displayed in FIG. 46B, fluid has been injected through the fluid passage 1294 (e.g., by a syringe or other type of inflation device) and into the interior volume 1298 of the adjustable deflection member 1292 through an aperture 1299 between the fluid passage 1294 and the interior volume 1298. The adjustable deflection member 1292 in its second state is configured to deflect the spray pattern 1290 in a desired direction, such as at least partially out through the distal opening 1296 of the lumen 1280. The shape of the inflated adjustable deflection member 1292 is depicted in FIG. 46B as having a convex nature, but in other embodiments, the balloon or other structure constituting the adjustable deflection member 1292 may be fabricated to form one or more linear ramps, or other shapes. In addition, there may be several different shapes or sizes that may be achieved by adjusting the adjustable deflection member 1292 into several different states, by injecting different volumes of fluid into the interior volume 1298. During fabrication, the shape of the adjustable deflection member 1292 may be heat formed by use of one or more molds or fixtures. An additional state may even be possible, wherein the adjustable deflection member 1292 in inflated enough to substantially or completely block off the lumen 1280, or to partially or completely block the orifice 1288. This additional state may be desired, for example, in cases during which an embolus is aspirated into the catheter, and it is desired to maintain the embolus within the catheter 1276 securely, while removing the catheter 1276 from the patient.

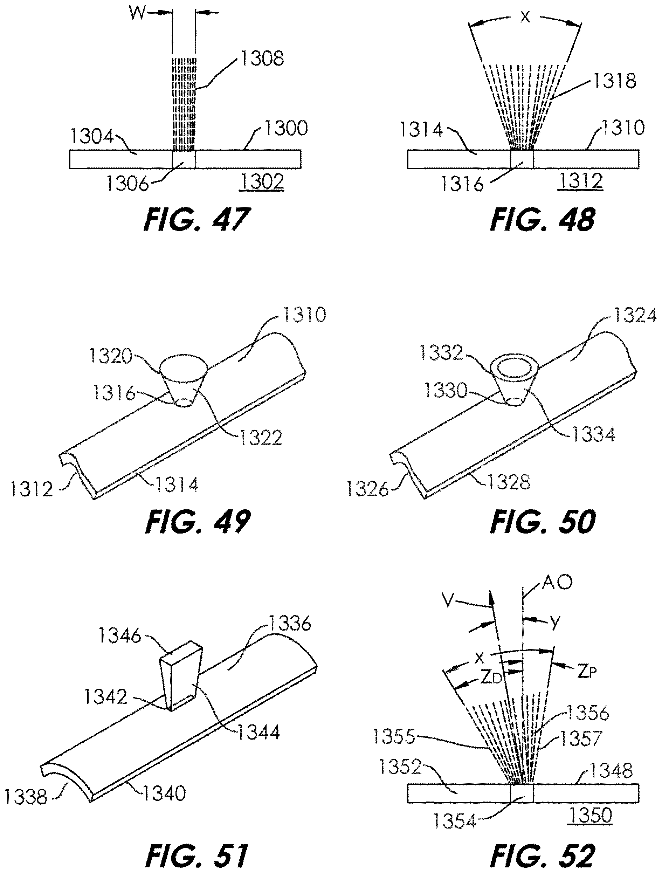

FIG. 47 illustrates a supply tube 1300 having a lumen 1302, a wall 1304, and an orifice 1306 through the wall 1304. A spray pattern 1308 exiting the orifice 1306, emanating from pressurized fluid within the lumen 1302, has a substantially solid or straight stream, wherein the width (or diameter) W of the stream does not significantly increase. FIG. 48 illustrates a supply tube 1310 having a lumen 1312, a wall 1314, and an orifice 1316 through the wall 1314. A spray pattern 1318 exiting the orifice 1316, emanating from pressurized fluid within the lumen 1312, has a divergent stream having an included angle x. FIG. 49 illustrates a three-dimensional depiction of a spray pattern 1320 having a divergent stream, which thus gives the spray pattern 1320 a conical shape 1322.

FIG. 50 illustrates a supply tube 1324 having a lumen 1326, a wall 1328, and an orifice 1330 through the wall 1328. A spray pattern 1332 exiting the orifice 1330, emanating from pressurized fluid within the lumen 1326, has a stream having a hollow conical shape 1334. FIG. 51 illustrates a supply tube 1336 having a lumen 1338, a wall 1340, and a rectangular orifice 1342 through the wall 1340. A spray pattern 1344 exiting the rectangular orifice 1342, emanating from pressurized fluid within the lumen 1338, has a stream having a divergent wedge shape 1346.

FIG. 52 illustrates a supply tube 1348 having a lumen 1350, a wall 1352, and an orifice 1354 through the wall 1352. A spray pattern 1356 exiting the orifice 1354, emanating from pressurized fluid within the lumen 1350, has a directional vector V that is angled at an angle y with respect to an axis AO of the orifice 1354. The directional vector represents a central portion of the spray pattern 1356. The spray pattern 1356 diverges and has an included angle x. The spray pattern has a distal-most extremity 1355 and a proximal-most extremity 1357. The distal-most extremity 1355 forms an angle ZD with the axis AO of the orifice 1354 and the proximal-most extremity 1357 forms an angle zp with the axis AO of the orifice 1354. In other embodiments, the spray pattern 1356 may have a shape similar to any of the spray patterns 1308, 1318, 1320, 1332, 1344 of FIGS. 47-51, or any other shape.

Any of the shapes of the spray patterns 1308, 1318, 1320, 1332, 1344, 1356 may be tailored by modifying the structure of the orifice in the wall of the supply tube (transverse dimension, diameter, length or wall thickness, angle, taper angle, cross-sectional shape), which facilitates the spray pattern(s) interfacing with the interior wall surface 916, 1040, 1078 or deflection elements/members 929, 940, 956, 1072, 1214, 1232, 1252, 1272, 1292 to create a number of different flow shapes, including substantially distally-oriented flow and/or substantially proximally-oriented flow. The spray patterns 1308, 1318, 1320, 1332, 1344, 1356 may be tailored to comprise a jet, a stream, a mist, or other spray physical characteristics. The spray patterns 1308, 1318, 1320, 1332, 1344, 1356 may convertible between any of these different modes or shapes with the aid of varying the pressure of the pressurized fluid.

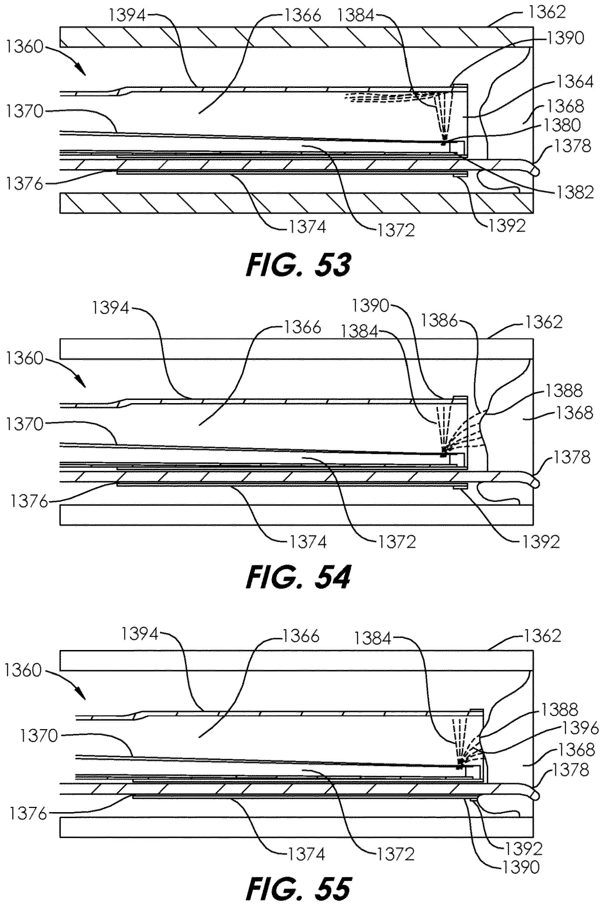

FIG. 53 illustrates an aspiration catheter 1360 which has been inserted into a blood vessel 1362 (artery, vein, etc.) and advanced such that the open distal end 1364 of the aspiration lumen 1366 is adjacent a thrombus/clot 1368. The aspiration catheter 1360 also includes a supply tube 1370 having a supply lumen 1372, and a guiding tube 1374 having a guidewire lumen 1376 configured for tracking over a guidewire 1378. A dilute or nondilute contrast media is pressurized by syringe, pump or other means through the supply lumen 1372 such that it exits the orifice 1380 at the distal end 1382 of the supply lumen 1372. A jet spray 1384 may include a distal component and/or a proximal component. The distal component 1386 (FIG. 54) may be a substantially distally-oriented component, and may at least partially exit the open distal end 1364 of the aspiration lumen 1366. The distal component 1386, as it fills a volume around the thrombus/clot 1368 (FIG. 54), may be viewed under radiography or fluoroscopy to identify a boundary 1388 of the thrombus/clot 1368. If the boundary 1388 is located within a desired proximity to the open distal end 1364 the aspiration lumen 1366 of the aspiration catheter 1360, the user may desire to inject or pump (e.g., with syringe or pump), using a high pressure, through the supply lumen 1372, to start or to continue a thrombolysis procedure. In some cases, the user may use the dilute or nondilute contrast media to perform the thrombolysis procedure. In some cases, the dilute or non-dilute contrast media may be combined or mixed with a lytic agent. In other cases, the user may replace the dilute or non-dilute contrast media with saline or a lytic agent, for example, by priming the supply lumen. If instead the boundary 1388 is located distal to the open distal end 1364 of the aspiration lumen 1366 of the aspiration catheter 1360 by more than a desired amount, the user may choose to advance the aspiration catheter 1360 until the open distal end 1364 is within the desired proximity to the boundary 1388 of the thrombus/clot 1368. In some cases, the desired proximity may be when the open distal end 1364 is flush with the boundary 1388 of the thrombus/clot 1368. In some cases, the desired proximity may be when the open distal end 1364 is about one mm from the boundary 1388 of the thrombus/clot 1368. In some cases, the desired proximity may be when the open distal end 1364 is about five mm from the boundary 1388 of the thrombus/clot 1368. Once the user advances the aspiration catheter 1360 such that the open distal end 1364 is within the desired proximity of the boundary 1688 of the thrombus/clot 1368, the user may start or continue the thrombolysis procedure.

FIG. 55 illustrates a method in which a user continually or temporarily injects or "puffs" small amounts 1396 of contrast agent (or contrast agent mixtures as described), in order to continually delineate the boundary 1388 of the thrombus/clot 1368, and the proximity of the open distal end 1364 of the aspiration lumen 1366 of the aspiration catheter 1360. In any of the embodiments presented herein, the distal end 1390 of the aspiration catheter 1360 may comprise a radiopaque marker or marker band 1392. In some embodiments, the catheter tubing 1394 may be radiopaque tubing, comprising radiopaque materials, including, but not limited to barium-sulfate, tantalum oxide, or titanium oxide.

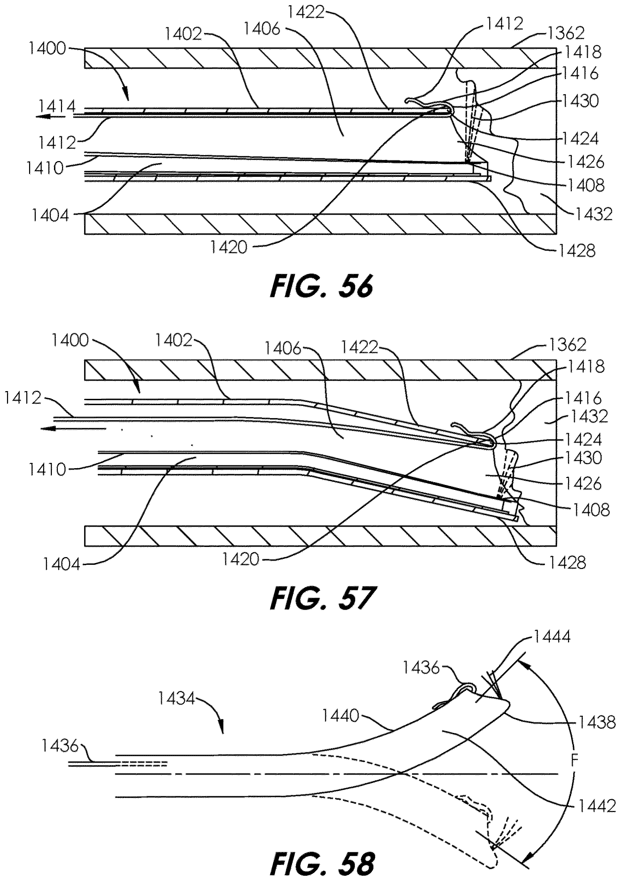

FIG. 56 illustrates a catheter system 1400 comprising a catheter 1402 having a supply lumen 1404, and lumen 1406. A wall 1410 surrounding the supply lumen 1404 includes an orifice 1408. A mandrel 1412 having a proximal end 1414 and a distal end 1416 extends through the lumen 1406. The distal end 1416 may have a curved portion 1418 (or hook portion) that includes a concavity 1420 for engaging a wall 1422 of the catheter 1402. The mandrel 1412 may be configured for insertion through the lumen 1406 such that the concavity 1420 engages the distal end 1424 of the wall 1422 (e.g., at the open distal end 1426) in a manner that traction (arrow, FIG. 57) may be placed by a user on the mandrel 1412, thereby pulling the distal end 1428 of the catheter 1402 in a proximal direction. This traction, coupled with the column strength of the catheter 1402, causes the distal end 1428 of the catheter 1402 to flex, as shown in FIG. 57. In some cases, the amount of flexure may be controlled by a particular force applied on the proximal end 1414 of the mandrel 1412 (e.g., by hand, or by a grasping tool which is connected to the proximal end 1414 by a collet or other lock), such that the jet of fluid 1430 exiting the orifice 1408 is steered such that it impinges on an adjacent structure (such as a thrombus/clot 1432). In some embodiments, the lumen 1406 may serve as an aspiration lumen, according to other embodiments described herein, and may also be used to aspirate at least some of the thrombus 1432. In this embodiment, the mandrel 1412 may also be used to disengage the lumen 1406 from a thrombus 1432, in cases where the thrombus 1432 becomes engaged, via vacuum, with the open distal end 1426 of the lumen 1406. Contrast media may be added to the fluid being delivered through the supply lumen 1404, in order to better visualize the location and status of the thrombus 1432. Contrast media may even be delivered through the lumen 1406, if the lumen 1406 is not actively being used to aspirate. A user may flex the distal end 1428 of the catheter 1402 back and forth such that the jet of fluid 1430 disrupts various areas/regions of the thrombus 1432. Additionally, the user applies a vacuum to the lumen 1406 to remove disrupted/macerated thrombus from the blood vessel 1362. A more thorough and efficient removal of the thrombus 1432 is thus possible.

FIG. 58 illustrates a catheter system 1434 having most of the characteristics of the catheter system 1400 of FIGS. 56 and 57, but with an additional preformed shape. A mandrel 1436 is configured to flex the distal end 1438 of the catheter 1440, but the distal end 1438 of the catheter 1440 additionally has a preformed curve 1442. Thus, a large flexure angle F range is possible, allowing the jet 1444 itself to strike a thrombus with many different possible trajectories.

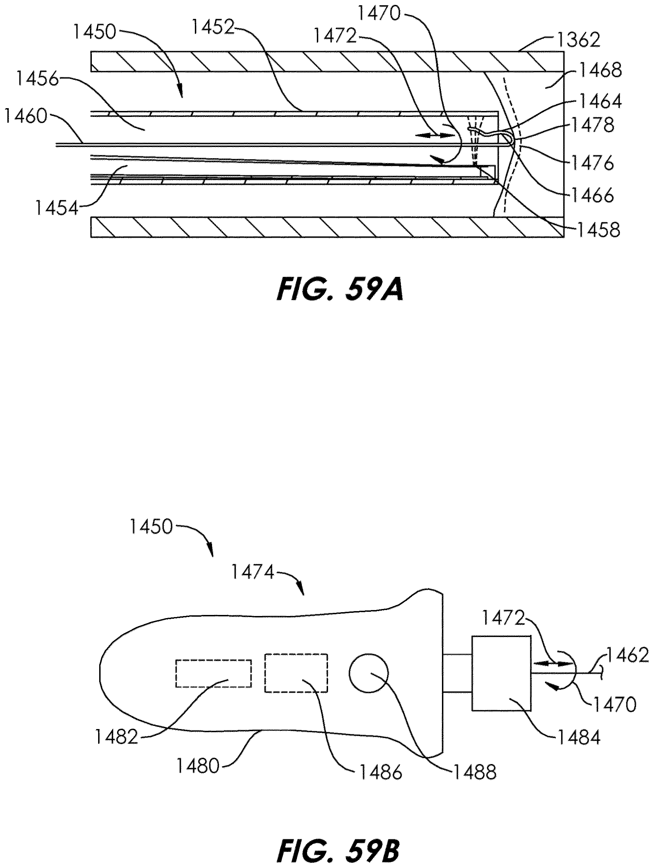

FIGS. 59A and 59B illustrate an aspiration system 1450 comprising an aspiration catheter 1452 having a supply lumen 1454, an aspiration lumen 1456 and an orifice 1458 communicating between the supply lumen 1454 and the aspiration lumen 1456, and a mandrel 1460 having a proximal end 1462 and a distal end 1464, the distal end 1464 including an enlarged portion 1466. The enlarged portion 1466 of the mandrel 1460 may include a hook (e.g., shepherd's crook), a curve, or other structure which is effective in disrupting a thrombus 1468 when the mandrel 1460 (and thus the enlarged portion 1466) is made to rotate 1470 and/or to longitudinally translate 1472. The mandrel 1460 may be inserted through the aspiration lumen 1456 of the aspiration catheter 1452 and may be rotated by attaching the proximal end 1462 of the mandrel 1460 to a rotation device 1474. The rotation device 1474 may also translate the mandrel 1460 back-and- forth longitudinally. The rotation device 1474 may include comprise such devices as a SPINR™ device marketed by Merit Medical Systems, Inc., (South Jordan, Utah, USA) or a FireBow™ device marketed by Vesatek, LLC (Irvine, California, USA). The enlarged portion 1466 may be used to disrupt a fibrous and/or calcified cap 1476 at one end of a thrombus 1468 by applying a disruptive force through rotation and/or cyclic longitudinal displacement. A convex or blunt portion 1478 of the enlarged portion 1466 may form an atraumatic end to the mandrel 1460. The rotation device 1474 comprises a handle 1480, a motor 1482, a rotatable chuck or lock 1484, and a transmission 1486 that is configured to couple movement from the motor into movement (e.g., rotation and/or longitudinal translation) of the rotatable chuck or lock 1484. The transmission 1486 may in some embodiments include gearing. A switch 1488 may be pressed by a user while the user holds the handle 1480, to turn the rotation/movement on or off. In some embodiments, the mandrel 1460 may also be usable in the manner of the mandrel 1412 of FIGS. 56 and 57 or the mandrel 1436 of FIG. 58.

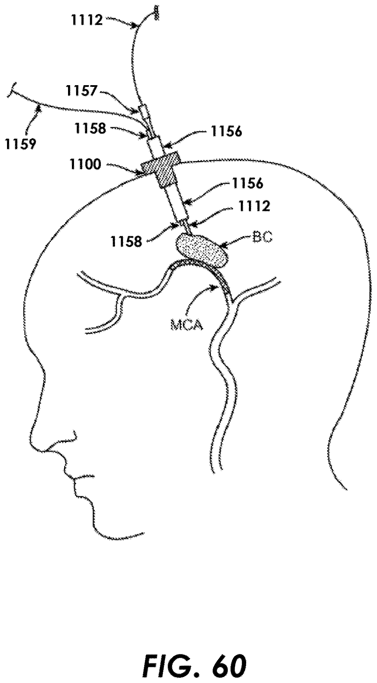

FIG. 60 illustrates as system for removing intracranial thrombus or intracranial hematoma (illustrated simply as BC-blood clots) through a window, aperture, or hole in the cranium of a patient. The window, aperture, or hole may be made by any suitable device, including, but not limited to a hand drill having a burr or other cutting element. Referring to FIG. 60, a trocar 1156, for example a four-channel trocar, can be introduced through an introducer 1100 close to the treatment area where blood clots BC are located. A visualization device 1158 such as a scope device, including but not limited to the NeuroPen (Medtronic Inc.) or the Epic Microvision (Codman, J&J Company, Piscataway, N.J.), may be introduced in the visualization channel of the trocar 1 156, and an ultrasound device 1112 may be introduced into the working channel of the trocar 1156. The ultrasound device 1112 may transmit, for example, at frequencies between about 1 kHz and about 20 MHz, and may be configured to disrupt or break up the blood clot BC.

FIG. 60 shows a cross sectional view of a human skull and brain, showing an introducer 1100 placed through the aperture in the skull. The trocar device 1156 is placed through the introducer 1100 and positioned within the treatment area where blood clots BC are located. The middle cerebral artery MCA is also shown. Often, the trocar 1156 can be introduced directly into the aperture in the skull without use of the introducer 1100. A visualization device 1158 may be introduced through the visualization channel of the trocar 1156. The visualization device 1158 is connected to a monitor (not shown) through a cable 1159. Some visualization devices (such as scopes) have an ocular element that can be used for visualization instead of a monitor. An ultrasound device 1112 having a handle 1157 is introduced through the working channel of the trocar 1156. Before the procedure, the physician directs the trocar 1156 under the visualization device 1158 to the location of the blood clots BC, and then positions the distal end of the ultrasound device 1112 inside the blood clots and activates ultrasound energy delivery. The physician has the ability to simultaneously observe the field of therapy with a visualization device 1158 while the therapeutic device 1112 dissolves and aspirates blood clots from the patients head. Blood clots maybe aspirated through an irrigation or overflow channel, which is analogous to the aspiration lumens of the aspiration catheters described herein. Also, blood clots may be aspirated through the ultrasound device 1112. Suitable systems for removing intracranial thrombus or intracranial hematoma are described by Nita in U.S. Patent Application Publication No. 2012/0330196, published December 27, 2012, and titled Method and Apparatus for Removing Blood Clots and Tissue from the Patients Head.

To further improve the ability to dissolve blood clots BC, delivery of one or more pharmacologic agents or microbubbles or nanobubbles to the clot location may be helpful. Such pharmacologic agents, microbubbles or nanobubbles can be delivered directly or in mixture with a conventional saline to the treatment location.

Cerebral temperature has been recognized as a strong factor in ischemic brain damage. Clinical evidence has shown that hypothermia ameliorates brain damage. Also, a therapeutic cooling to between 30°C or 35°C that includes the patient head or a whole body (systemic cooling) may reduce ischemic brain damage; reduce intracranial pressure and edema after ICH. Focused cranial cooling can be achieved with a simple method of placing ice or cold gel packs around the head or neck. Systemic cooling maybe be done by infusing ice-cold saline using intravenous (IV) approach.

Any of the embodiments described herein may be used conjunction with the Apollo™ System (Penumbra, Inc., Alameda, CA, USA).

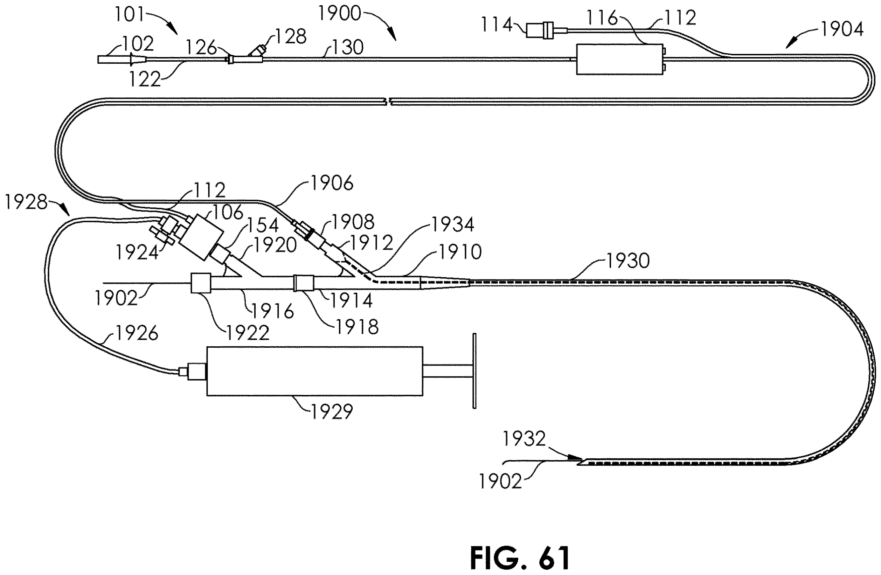

A system for aspirating thrombus 1900 is illustrated in FIG. 61, and comprises an aspiration catheter 1930, a tubing set 1904 configured for injection of a fluid at high pressure through at least a portion of the aspiration catheter 1930, and a vacuum set 1928, configured to couple a vacuum source 1929 to the aspiration catheter 1930. The aspiration catheter 1930 includes a y-connector 1910 having a female luer 1912 hydraulically coupled to its high-pressure injection lumen 1934 and a female luer 1914 hydraulically coupled to its aspiration lumen 1932. The high-pressure injection lumen 1934 may have similar characteristics to the high-pressure injection lumen 36 of the catheter 16 of FIG. 3. The aspiration lumen 1932 may have similar characteristics to the aspiration lumen 38 of the catheter 16 of FIG 3. The tubing set 1904 may be coupled to a fluid source and a pump, for example, the fluid source 20 and pump 26 of FIG. 1. The tubing set 1904 includes an injection tube 1906 connected to a male luer 1908, which may be removably coupled to the female luer 1912 of the y-connector 1910 of the aspiration catheter 1930. The aspiration lumen 1932 of the aspiration catheter 1930 also serves as a guidewire lumen, for the placement of a guidewire 1902. The aspiration lumen/guidewire lumen 1932 extends the entire length of the aspiration catheter 1930, providing an "over-the- wire" system which can be delivered or tracked over the guidewire 1902. A y-connector 1916 having a Touhy-Borst 1922 is attached to the y-connector 1910 of the aspiration catheter 1930 via its distal male luer 1918 which is hydraulically coupled to the female luer 1914 of the y-connector 1910. The Touhy-Borst 1922 may be adjusted an appropriate amount to create a seal over the guidewire 1902. The Touhy-Borst 1922 may in some cases even be adjusted so that a slow, steady drip of blood (e.g., Heparinized blood or non-Heparinized blood) occurs out through the Touhy-Borst 1922. This may be done in order to minimize any stagnation of blood within the aspiration lumen 1932. The Touhy- Borst 1922 may alternatively be replaced by any other type of seal that is configured to permanently, adjustably, or removably seal around a guidewire 1902. The vacuum set 1928 includes a luer fitting 154 (for example, a male luer) that is configured to attach to a female luer 1920 of the y-connector 1916. The vacuum set 1928 includes a pressure transducer 106 having an internal passage which is carried in line with a stopcock 1924. Proximal to the stopcock 1924, a vacuum line 1926 is configured to connect to the vacuum source 1929 (via a connector or by direct attachment). Signals from the pressure transducer 106 are carried by a cable 112 (e.g., to the circuit board 304 of the pump base 200 (FIG. 17). In alternate embodiments, the male luer 1918 and female luer 1914 may be replaced by two connectors/connections that are permanently attached to each other, or the y-connector 1910 and y-connector 1916 may be integrally constructed. Other disposable components 101 are similar to those described in the system for aspirating thrombus 100 of FIG. 4.

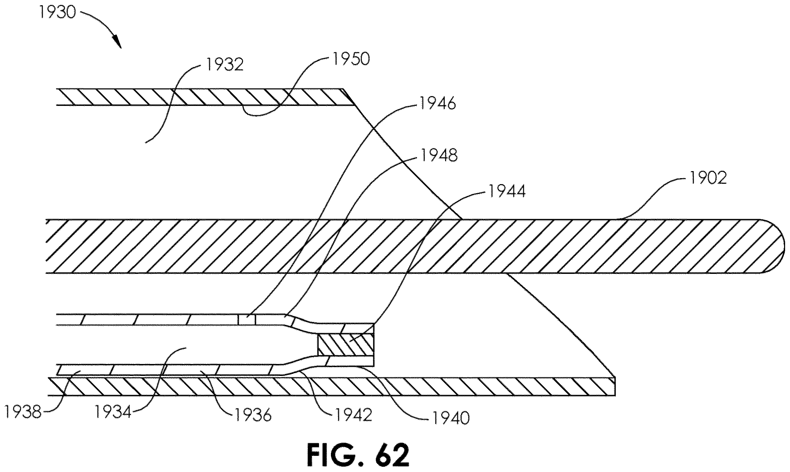

FIG. 62 illustrates the distal end of the aspiration catheter 1930 and the guidewire 1902. The guidewire 1902 is free to be moved distally or proximally in the longitudinal direction, or to be rotated within the aspiration lumen/guidewire lumen 1932. The distal end of the guidewire 1902 may be shapeable, for example, to create a "J"-tip for selectability of vessels or through stenoses or obstructions. The high-pressure injection lumen 1934 is contained within a tube 1936 having a large diameter portion 1938 and a small diameter portion 1940. The small diameter portion 1940 may transition from the large diameter portion 1938 via a neckdown or tapered portion 1942. The small diameter portion 1940 is blocked using a blocking material 1944, which may include a polymer, adhesive, or epoxy adhered to the internal walls of the small diameter portion 1940. Alternatively, the small diameter portion 1940 may be crimped, tied off, sealed, or otherwise occluded, without the use of a blocking material 1944. An orifice 1946 in a wall 1948 of the tube 1936 is configured to create a jet from high pressure fluid injected through the high-pressure injection lumen 1934. The jet exiting the high-pressure injection lumen 1934 and entering the aspiration lumen 1932 may be configured to impinge on an inner wall 1950 of the aspiration lumen/guidewire lumen 1932. Aspiration may be performed with the guidewire 1902 in place within the aspiration lumen/guidewire lumen 1932, or may be performed with the guidewire 1902 retracted proximally of the longitudinal location of the orifice 1946. In cases where the guidewire is left in place (as shown in FIG. 62), during aspiration the guidewire 1902 may be rotated so that it does not significantly impede the jetting through the orifice 1946, or in some cases, the jet itself may be sufficient to force the guidewire 1902 into a position that does not impede the jetting against the inner wall 1950.

Returning to FIG. 61, the stopcock 1924 may be manipulated by the user (physician, technician, etc.) to turn the system 1900 on and off. The pressure transducer 106 sends its signals via the cable 112 to pump, such as the pump base 200 (FIG. 12). Circuitry, e.g., contained in the circuit board 304 of the pump base 200 (FIG. 17), such as a microprocessor or microcontroller, may be configured or configurable (e.g., programmable), such that a change in pressure to a particular pressure value, or a change in pressure having a particular slope of pressure change over time initiates the pump to start or stop. The user, who may be wearing sterile gloves, is thus able to turn the system on and off, without requiring the help of any external (e.g., non-sterile, non-scrubbed) personnel, simplifying and speeding up the procedure. In alternate embodiments, an extension tube may be placed between the stopcock 1924 and the pressure transducer 106. Though a one-way stopcock is generally illustrated in FIG. 61, other types of stopcocks may be used. In alternate embodiments, the stopcock 1924 may be replaced with other types of valves having on and off positions. In some embodiments, the circuitry (e.g. circuit board 304) is configured to provide a delay between the receipt of the signal from the pressure transducer 106/cable 112 and a signal commanding initiation of the pumping action of the pump base 200. The purpose of the delay may be so that the vacuum applied initially engages a thrombus without any injection of fluid, and then, after the delay, allows the injection on fluid (e.g., to macerate the thrombus) once the thrombus is engaged, and in position to be macerated. In some embodiments, the circuitry is configured to allow a delay of between about 0.01 second and about 1.00 second. In some embodiments, the circuitry is configured to allow a delay of between about 0.10 second and about 0.25 second.

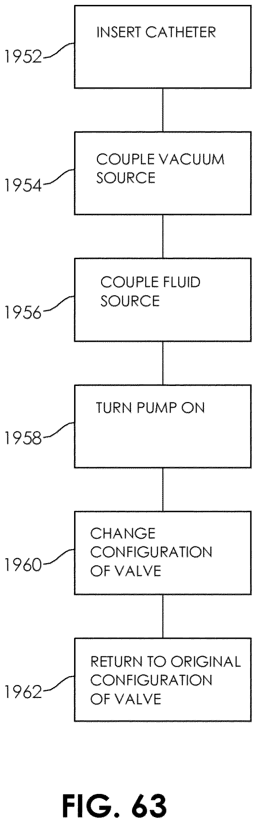

A representative method for using the system for aspirating thrombus is presented in FIG. 63. In step 1952, a user inserts a distal portion of the aspiration catheter 1930 into a subjects vasculature, for example, in a target area near or adjacent a thrombus. The user may choose to perform step 1952 after connecting the aspiration catheter 1930 to other components. In step 1954, the user couples a vacuum source 1929 to the aspiration lumen 1932 of the aspiration catheter 1930 by coupling the luer fitting 154 to the female luer 1920 of the y-connector 1916 and the vacuum line 1926 to the vacuum source 1929. The connection may be a luer connection in the case of a syringe, or a friction fitting, or a spike, or other type of connection. In some cases, the user may start with the stopcock 1924 in a closed position between the vacuum source 1929 and the pressure sensor 106. In step 1956, the user couples the supply lumen 1934 of the aspiration catheter 1930 to the fluid source 20 and pump base 200 by coupling the male luer 1908 of the tubing set 1904 to the female luer 1912 of the y-connector 1910 of the aspiration catheter 1930. In step 1958, the user may turn on the pump base 200, for example, by pressing an "ON" button. Step 1958 may be optional, for example, in an embodiment of a pump base 200 that is configured to automatically sense the attachment of the aspiration catheter 1930, or components such as the cassette 116 and/or the connector 114. Some examples include proximity sensors, RFID chips, a resisitor having a particular value, or a switch carried on one or more connectors.

In step 1960, the user changes the position or configuration of the valve of the stopcock 1924. For example, the user may turn the stopcock 1924 from the off position to the on position. With the stopcock 1924 in the off position, the pressure sensor 106 is blocked from being able to sense the internal pressure (e.g., negative pressure) of the vacuum source 1929, and thus does not sense pressures that are below a particular pressure threshold programmed into the circuitry (e.g., circuit board 304). The circuitry is configured (or configurable) to not allow the motor 302 of the pump base 200 to operate when this condition is sensed, so that no pressurized fluid is forced through the supply lumen 1934 and into the aspiration lumen 1932. By assuring that the motor 302 does not cause the pumping of pressurized fluid if the vacuum source 1929 is not actively causing aspiration through the aspiration lumen 1932, the disruption of thrombus within the patients vasculature is avoided. Disruption of the thrombus without aspiration could potentially create thromboemboli that could migrate or be circulated to portions of the vasculature and body where they could cause damage (occlusion, stroke, myocardial infarction, etc.). When the user opens the stopcock 1924, as in step 1960, and a pressure below a particular pressure threshold is sensed by the pressure sensor 106, the control circuitry initiates the motor 302 to force pressurized fluid through the supply lumen 1934 and into the aspiration lumen 1932, causing thrombus to be safely aspirated through the aspiration catheter 1930. The user may choose to move the aspiration catheter 1930 in the blood vessel (distally or proximally or rotating it), while aspirating.

In step 1962, the user returns the position of the stopcock 1924 to its original position. For example, if the stopcock 1924 was turned to its on position in step 1960, then the stopcock 1924 is turned to its off position in step 1962. As an example, after the user turns the stopcock 1924 to its on position in step 1960, and uses the system 1900 to aspirate thrombus, the user may desire to terminate the aspiration, and does so by turning the stopcock 1924 to its off position in step 1962. When the user closes the stopcock 1924, as in step 1962, and a pressure at or above a particular pressure threshold is sensed by the pressure sensor 106, the control circuitry stops the motor 302 to stop pressurized fluid from being pumped through the supply lumen 1934 and into the aspiration lumen 1932, causing the aspiration of thrombus to be safely terminated. The user is able, thus, to control when aspiration occurs by simply turning the stopcock 1924 on and off. The stopcock 1924 functions as an electric switch via the pressure measurement by the pressure sensor 106 and the control by the circuit board 304. This allows a user, who has likely "scrubbed" and is operating in a sterile field, to avoid any switches (on the pump base 200 or other) that may either be non-sterile and/or remote or out of reach. The user does not have to shout voice commands to other medical personnel, which would not have the same one-to-one effect. Thus, the user is able to rapidly and immediately stop and start aspiration, to best respond to critical events. For example, when the user is aspirating thrombus with the aspiration catheter 1930, and suddenly sees (e.g., via fluoroscopy) that something in the blood vessel has changed, the user can immediately turn the stopcock 1924 to the off position and stop aspiration. The user may move back and forth between steps 1960 and 1962, while moving the aspiration catheter 1930 or stopping the aspiration catheter 1930, to optimize the aspiration procedure.

In an alternative embodiment, the pressure sensor 106 may be coupled to the vacuum source 1929, but not the aspiration catheter 1930, in order to be used in an analogous manner of an on/off switch. For example, the pump base 200 of the system for aspirating thrombus 100 or the saline pump drive unit 400 of the piston pump system 300 may be operated for pumping a drug 730 through the supply lumen 708 of the aspiration catheter 700, while no aspiration is being performed through the aspiration lumen 710. Distal to the pressure sensor 106, a plug or closed stopcock (or other closed valve) may be placed, while the cable 112 extending from the pressure sensor 106 is electrically coupled to the pump base 200 or piston pump system 300. Thus, signals from the pressure sensor 106 may be used to turn the pump base 200 or piston pump system 300 on or off by the turning the vacuum source 1929 on or off (or by connecting or disconnecting the vacuum source 1929 or otherwise adjusting the vacuum source 1929). This is done even though the vacuum source 1929 is not connected to the aspiration lumen 710 of the aspiration catheter 700. Thus, automatic injection of the drug 730 may be initiated or ended by manipulation of the vacuum source 1929 alone (e.g., switch, power, etc.). In yet another embodiment, the aspiration catheter may be replaced by another catheter that does not even have an aspiration lumen 710, but does have a supply lumen 708. In this embodiment, if connected as described above, would still allow automatic injection of the drug 730 by manipulation of the vacuum source 1929.

In any one of the embodiments in which the pump base 200 or the saline pump drive unit 400 may be used to deliver a drug 730, a precision delivery of the drug 730 is achieved, which is an improvement over standard gravity-fed infusion systems, having somewhat limited precision.

Alternative embodiments are contemplated, wherein either the system for aspirating thrombus 100 or the piston pump system 300 includes a standard on/off power switch that can be used to initiate or suspend pumping. The switch may be carried on the system for aspirating thrombus 100 or the piston pump system 300 itself, for example, on the pump base 200 or on the saline pump drive unit 400. Alternatively, the switch may be remote from the pump base 200 or the saline pump drive unit 400, and may even be supplied sterile or sterilizable, so that it can be maintained on a sterile field on or in the vicinity of the patient. The separate switch may in some embodiments include a vacuum switch valve 540 or the vacuum sensing method described above in order to control its operation (on/off). In some embodiments, the switch may be used to control other parameters than on and off, for example it may control the speed of the pump motor, or may control certain safety features. In the embodiment in which the switch controls the speed of the pump motor, there may be particular embodiments, in which the switch includes a potentiometer for allowing the adjustment of a changeable electrical resistance. Though the aspiration catheter 1930 of FIGS. 61-62 is shown as an over-the-wire catheter (with guidewire 1902 internal to the aspiration catheter 1930 substantially the entire length of the aspiration catheter 1930), alternatively, the aspiration catheter 1930 may be a single operator exchange catheter with a short guidewire lumen (similar to the guidewire tube 132 of the aspiration catheter 118 of FIG. 8).

Another embodiment of a system for aspirating thrombus 2000 is illustrated in FIG. 64. The system for aspirating thrombus 2000 includes, three major components: the pump base 200 of FIG. 12, an aspiration catheter 2018, and a tubing set 2003. The aspiration catheter 2018 and the tubing set 2003 represent disposable components 2001, and the pump base 200 is a reusable component. It is not necessary to sterilize the pump base 200 as it is kept in a non-sterile field or area during use. The aspiration catheter 2018 and the tubing set 2003 may each be supplied sterile, after sterilization by ethylene oxide gas, electron beam, gamma, or other sterilization methods. The aspiration catheter 2018 may be packaged and supplied separately from the tubing set 2003, or the aspiration catheter 2018 and the tubing set 2003 may be packaged together and supplied together. Alternatively, the aspiration catheter 2018 and tubing set 2003 may be packaged separately, but supplied together (i.e., bundled). The aspiration catheter 2018 and tubing set 2003 share many of the same features as the aspiration catheter 118 and tubing set 103 of FIG. 4 and the aspiration catheter 818 and tubing set 803 of FIG. 21. The aspiration catheter 2018 has a distal end 2020 and includes an over-the-wire guidewire lumen/aspiration lumen 2032 extending between a distal tip 2036, and a proximal end 2019 comprising a y-connector 2010. The catheter shaft 2042 of the aspiration catheter 2018 is connected to the y-connector 2010 via a protective strain relief 2056. In other embodiments, the catheter shaft 2042 may be attached to the y-connector 2010 with a luer fitting. The y-connector 2010 comprises a first female luer 2055 which communicates with a catheter supply lumen 2093 (FIG. 65), and a second female luer 2051 which communicates with the guidewire lumen/aspiration lumen 2032.

A spike 2002 for coupling to a fluid source 20 (FIG. 1) allows fluid to enter through extension tubing 2022 and flow into a supply tube 2030. An optional injection port 2028 allows injection of materials or removal of air, as described in relation to previous embodiments. A cassette 2016 is used in conjunction with the pump base 200, and is similar in structure and function to the cassette 116 in FIGS. 15 and 16 and cassette 816 in FIGS. 21 and 23. Fluid is pumped into the injection tube 2052 from action of the cassette 2016 as applied by the pump base 200. A male luer 2054, coupled to the distal end of the injection tube 2052, is configured to attach to the female luer 2055 of the y- connector 2010.

Accessories 2057 are illustrated that are intended for applying a vacuum source 22, such as a syringe 2049 having a plunger 2067 and a barrel 2099, to the aspiration lumen 2032 of the catheter 2018. The syringe 2049 is attached to a vacuum line 2008 via the luer 2065 of the syringe 2049. A stopcock 2047 may be used to maintain the vacuum, or, the plunger 2067 may be a locking variety of plunger that is configured to be locked in the retracted (vacuum) position. A male luer 2053 at the end of the vacuum line 2008 may be detachably secured to the female luer 2051 of the y-connector 2010 of the aspiration catheter 2018. As shown in more detail in FIG. 66, a pressure sensor 2006 is secured inside an internal cavity 2097 of the y-connector 2010 proximal to the female luer 2055 and the female luer 2051. A valve 2095, for example a Touhy-Borst, at the proximal end of the y-connector 2010 allows hemostasis of the guidewire lumen/aspiration lumen 2032 around a guidewire 2091. In other embodiments, the valve 2095 may comprise a longitudinally spring-loaded seal. The guidewire 2091 may be inserted entirely through the guidewire lumen/aspiration lumen 2032. Signals from the pressure sensor 2006 are carried through a cable 2012 to a connector 2014. The connector 2014 is plugged into the socket 308 (FIG. 12) of the pump base 200. Pressure related signals may be processed by the circuit board 304 of the pump base 200. The pressure transducer 2006 may be powered from the pump base 200, via the cable 2012. The accessories 2057 may also be supplied sterile to the user. In some embodiments, the pressure sensor 2006 may comprise a sensor that is utilized in the single use LD20 Liquid Flow Sensor manufactured by Sensirion AG of Stafa, Switzerland.

As an alternative to the on/off switching function of the stopcock 1924 of the system for aspirating thrombus 1900 of FIG. 61, a foot pedal 2021 is configured to operate a pinch valve 2023 for occluding or opening the vacuum line 2008. The foot pedal 2021 comprises a base 1025 and a pedal 2027 and is configured to be placed in a non-sterile area, such as on the floor, under the procedure table/bed. The user steps on the pedal 2027 causing a signal to be sent along a cable 2029 which is connected via a plug 2041 to an input jack 2037 in the pump 200. The vacuum line 2008 extends through a portion of the pump 200. The circuit board 304 of the pump (FIG. 17) may include a controller configured to receive one or more signals indicating on or off from the foot pedal 2021. The controller of the circuit board 304 may be configured to cause an actuator 2031 carried by the pump 200 to move longitudinally to compress and occlude the vacuum line 2008 between an actuator head 2033 attached to the actuator 2031 and an anvil 2035, also carried by the pump 200. By stepping on the pedal 2027, the user is able to thus occlude the vacuum line 2008, stopping the application of a negative pressure. Also, by stepping on the pedal 2027, the user may cause the opposite action, wherein the actuator head 2033 opens the vacuum line 2008, by moving away from the anvil 2035. The anvil 2035 may have a flat (planar) shape, or a U-shape (e.g., semi -cylindrical), or a V-shape (e.g., a V-block) where it contacts the tubing of the vacuum line 2008. Furthermore, the actuator head 2033 may have a flat (planar) shape, or a U-shape (e.g., semi-cylindrical), or a V-shape (e.g., a V-block) where it contacts the vacuum line 2008. The foot pedal 2021 may operate by alternately causing the actuator 2031 to move in a first direction and a second, opposite direction, respectively, with alternate hits on the pedal 2027. In some embodiments, as the pedal 2027 of the foot pedal 2021 is depressed, the controller may be configured to open the pinch valve 2023. The pressure transducer 2006 thus senses a negative pressure and sends a signal, causing the controller to start the motor 302 of the pump 200. As the effect via the electronics is substantially immediate, the motor 302 starts pumping almost immediately after the pedal 2027 is depressed. As the pedal 2027 of the foot pedal 2021 is released, the controller then causes the pinch valve 2023 to close. The pressure transducer 2006 thus senses that no negative pressure is present and causes the motor 302 of the pump 200 to shut off. Again, the effect via the electronics is substantially immediate, and thus the motor 302 stops pumping almost immediately after the pedal 2027 is depressed. During sterile procedures, the main interventionalist is usually "scrubbed" such that the hands only touch items in the sterile field. However, the feet/shoes/shoe covers are not in the sterile field. Thus again, a single user may operate a switch (via the pedal 2027) while also manipulating the catheter 2018 and guidewire 2091. However, this time, it is the sterile field hands and non-sterile field feet that are used. Alternatively, the foot pedal 2021 may comprise two pedals, one for occlude and one for open. In an alternative foot pedal embodiment, the pedal 2027 may operate a pneumatic line to cause a pressure activated valve or a cuff to occlude and open the vacuum line 2008, for example, by forcing the actuator head 2033 to move. In another alternative embodiment, the pedal 2027 may turn, slide, or otherwise move a mechanical element, such as a flexible pull cable or push rod that is coupled to the actuator 2031, to move the actuator head 2033. The cable 2029 may be supplied sterile and connected to the base 2025 prior to a procedure. The occlusion and opening of the vacuum line 2008 thus acts as a on and off switch for the pump 200 (via the pressure sensor 2006), as described in relation to FIG. 61. The on/off function may thus be performed by a user whose hands can focus on manipulating sterile catheters, guidewires, and accessories, and whose foot can turn the pump on and off in a non-sterile environment. This allows a single user to control the entire operation or the majority of operation of the system for aspirating thrombus 2000. This can be an advantage both in terms of a rapid, synchronized procedure, but is also helpful in laboratories where additional assistants are not available. The actuator 2031 and anvil 2035 may be controlled to compress the vacuum line 2008 with a particular force, and the actuator 2031 may be controlled to move at a particular speed, either when compressing or when removing compression. Speed and force control allows appropriate response time, but may also be able to add durability to the vacuum line 2008, for example, by not overcompressing.

A particular configuration for a system for aspirating thrombus 1600 is illustrated in FIG. 77, and comprises a pump 1602, a vacuum line 1606, and a pressure sensor 1608 having a cable 1604 for connecting to the pump 1602 and carrying signals from the pressure sensor 1608. A pinch valve 1610 is operable by a foot pedal (not shown, but similar to the foot pedal 2021 of the system for aspirating thrombus 2000 in FIG. 64). The foot pedal 2021 may communicate with the pinch valve 1610 via a wired connection through the pump 1602 or may communicate with the pinch valve 1610 wirelessly. The pinch valve 1610 extends from the pump 1602 and includes a pinch valve housing 1609 having an opening 1611 which is configured to hold a portion of the vacuum line 1606. Internal to the housing 1609 are components similar to the actuator head 2033, actuator 2031, and anvil 2035 of the pinch valve 2023 of FIG. 64, which are configured to compress an external portion of the tubing of the vacuum line 1606 when the foot pedal 2021 is depressed. The foot pedal 2021 may then be depressed a second time to release the compression on (decompress) the vacuum line 1606. The compression of the vacuum line 1606 may be configured to be a complete occlusion of the tubing, thus isolating the vacuum source 22 from the pressure sensor 1608. An input port 1612 to the pressure sensor 1608 may include a septum 1614 for adding or removing fluid within the vacuum line 1606 (e.g., via a hypodermic needle), or alternatively may include a luer connector and valve. The pressure sensor 1608 is thus configured to reside in a non-sterile field, and is capable of detecting the presence of vacuum (negative pressure) or the lack of vacuum when the foot pedal is depressed by the foot of a user. For example, with the pinch valve 1610 closed via a signal (or resultant mechanical action) from foot pressure on the foot pedal, and thus no vacuum applied within the vacuum line 1606, fluid (such as saline) may be injected (proximal to distal) through the aspiration lumen of an aspiration catheter connected to the vacuum line 1606, and into the blood vessel of a patient. The pump 1602 may be configured (via an internal controller) to not pump saline when the lack of vacuum in the vacuum line 1606 is determined. Additionally, if vacuum is present, but is suddenly lost, the pump 1602 will shut down. As seen in FIG. 77, the pinch valve 1610 is located between the vacuum source 22 and the pressure sensor 1608, thus when the pinch valve 1610 shuts off the aspiration catheter 2018 from the vacuum source 22, the pressure sensor 1608 is still able to sense the condition within the aspiration lumen 2032 of the aspiration catheter 2018. In most cases, after the pinch valve 1610 is caused to close, the negative pressure within the aspiration lumen 2032 will rise toward the ambient pressure rather quickly. This change will be sensed by the pressure sensor 1608. However, in cases in which a piece of thrombus causes a temporary or permanent clog in the aspiration lumen 2032, the pressure sensor 1608 is able to sense these occurrences. For example, a large moving thrombus will delay the time that the internal pressure of the aspiration lumen 2032 rises to ambient after the pinch valve 1610 is closed. A complete occlusion of the aspiration lumen 2032 by a thrombus may cause at least some level of negative pressure to remain in the aspiration lumen. Each of these potential occurrences can be identified by the pressure measured by the pressure sensor 1608. The controller may be configured to error or indicate that there is a temporary or permanent clog in the aspiration lumen 2032, for example, with a display, or a visual, audible, or tactile warning or alarm. The user may respond to this indication by removing and unclogging the aspiration catheter 2018, e.g., by moving a guidewire back and forth, or may determine that the aspiration catheter 2018 needs to be replaced. Thus, the ability of the pressure sensor 1608 to monitor aspiration lumen pressure, regardless of whether the pinch valve 1610 is open or closed, offers an important safety control, as well as a general diagnostic of the state of the system (catheter flow status, etc.). Another general advantage of using a pinch valve 2023, 1610 is that blood only contacts the internal diameter of the vacuum line 2008, 1606, and thus is not forced within interstices of rotatable valves or other moving parts that otherwise could begin to stick or foul with biological material. The vacuum line 2008, 1606 is simply compressed an uncompressed, allowing a robust and durable design. The internal volume of the vacuum line 2008, 1606 easily maintains sterility. And, as the pinch valve 2023, 1610 is isolated from blood/thrombus, it is reusable. The co-location of two or more of the vacuum source 22, the pinch valve, the pump 1602 and the push button 1607 may also be an advantage because it allows a quick assessment by an attending physician or medical personnel in a quick glance, for example, if otherwise focused on catheter manipulation in the sterile field.

An additional advantage supplied by the pinch valve 1610 is that the controller may be configured to cause the pump to operate whenever the pinch valve is in the open condition. Thus, there will always be at least some jet-induced maceration of thrombus while a vacuum is being applied to the aspiration lumen 2032. This minimizes or prevents aspiration lumen clogging which could occur if vacuum is being applied to a large portion of thrombus without any maceration (breaking into smaller pieces).

As an alternative or in addition to the foot pedal 2021, a push button 1607 may be provided on the pump 1062, or in a remote component. In a first embodiment, the push button 1607 may simply allow manual opening and closing of the pinch valve 1610 on the vacuum line 1606. A first push to compress the vacuum line 1606 and isolate the pressure sensor 1608 from the vacuum source 22, and a second push to decompress the vacuum line 1606. Alternatively, the push button 1607 may act as a reset button, and be configured to always open the pinch valve 1610 (when it is closed), or to make no change if the pinch valve 1610 is already open. In an embodiment having both the foot pedal 2021 and the push button 1067, with the push button 1607 configured as a reset button, activation of the foot pedal 2021 toggles the pinch valve 1610 open and closed, while activation of the push button 1607 always places or maintains the pinch valve 1610 in the open position. The push button 1607 may be a mechanical (doorbell) type button, or may be a touch switch (e.g., capacitive, resistive, or piezo), or in some embodiment my even be a toggle or rocker switch.

Returning to FIG. 64, the plug 2041 contains an identification component 2043, which may be read by the circuitry (e.g., circuit board 304) coupled to the input jack 2037 of the pump 200. In some embodiments, the identification component 2043 comprises a resistor having a particular value. When the plug 2041 is connected to the input jack 2037, the circuitry of the input jack 2037 sends a current through the resistor, resulting in the pump 200 being electronically placed into a "foot pedal" mode, wherein the foot pedal 2021 can be used to control the operation of the pinch valve 1610. Alternatively, when the plug 2041 is detached from the input jack 2037, and the circuitry is not able to identify the resistor, the pump 200 is placed in a "manual" mode, wherein the pump is controllable only by buttons 232 (FIG. 12). In other embodiments, instead of a resistor, the identification component 2043 may comprise an RFID (radio-frequency identification) chip, which is read by the circuitry when the plug 2041 is connected to the input jack 2037. In other embodiments, a proximity sensor, such as a Hall-effect device, may be utilized to determine whether the plug 2041 is or is not connected to the input jack 2037.

In should be noted that in certain embodiments, the pinch valve 2023, 1610 and the foot pedal 2021 may be incorporated for on/off operation of the pinch valve 2023, 1610 on the vacuum line 2008, 1606, without utilizing the pressure sensor 2006, 1608. In fact, in some embodiments, the pressure sensor 2006, 1608 may even be absent from the system for aspirating thrombus 2000, 1600, the foot pedal 2021 being used as a predominant control means.

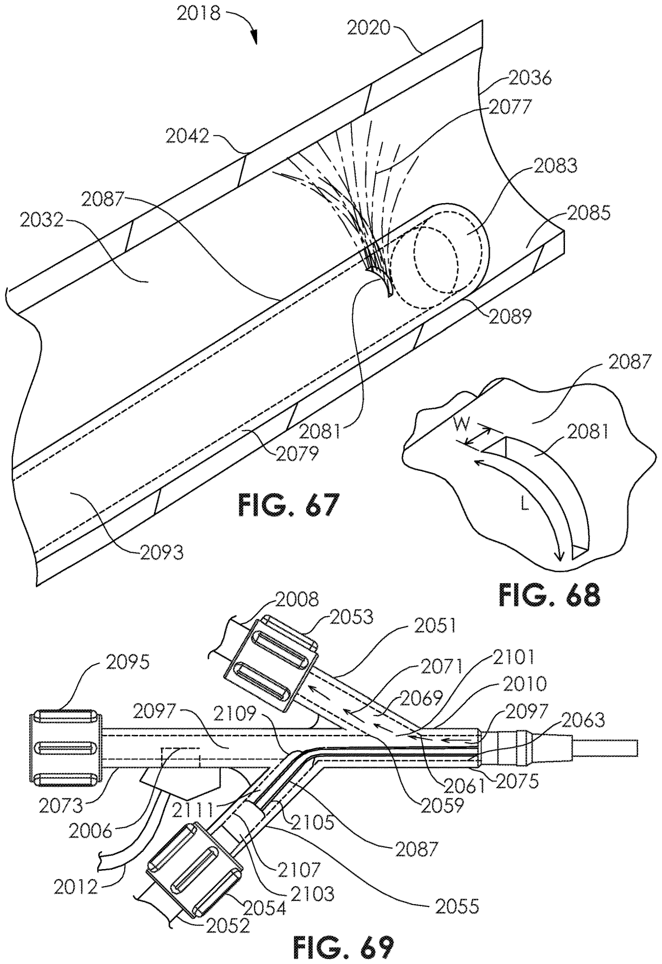

Turning to FIG. 65, a supply tube 2087, which contains the catheter supply lumen 2093, freely and coaxially extends within the over-the-wire guidewire lumen/aspiration lumen 2032. A distal end 2089 of the supply tube 2087 is secured to an interior wall 2085 of the guidewire lumen/aspiration lumen 2032 of the catheter shaft 2042 by adhesive, epoxy, hot melt, thermal bonding, or other securement modalities. A plug 2083 is secured within the catheter supply lumen 2093 at the distal end 2089 of the supply tube 2087. The plug 2083 blocks the exit of pressurized fluid, and thus the pressurized fluid is forced to exit through an orifice 2081 in the wall 2079 of the supply tube 2087. The free, coaxial relationship between the supply tube 2087 and the catheter shaft 2042 along their respective lengths, allows for improved flexibility. In some embodiments, in which a stiffer proximal end of the aspiration catheter 2018 is desired (e.g., for pushability or even torquability), the supply tube 2087 may be secured to the interior wall 2085 of the guidewire lumen/aspiration lumen 2032 of the catheter shaft 2042 along a proximal portion of the aspiration catheter 2018, but not along a distal portion. This may be appropriate if, for example, the proximal portion of the aspiration catheter 2018 is not required to track through tortuous vasculature, but the distal portion is required to track through tortuous vasculature. The free, substantially unconnected, coaxial relationship between the supply tube 2087 and the catheter shaft 2042 along their respective lengths, may also be utilized to optimize flow through the guidewire lumen/aspiration lumen 2032, as the supply tube 2087 is capable of moving out of the way due to the forces of flow (e.g., of thrombus/saline) over its external surface, such that the remaining inner luminal space of the guidewire lumen/aspiration lumen 2032 self- optimizes, moving toward the lowest energy condition (least fluid resistance) or toward the largest cross-sectional space condition (e.g., for accommodating and passing pieces of thrombus). [0200] In FIG. 67, the distal end 2089 of the supply tube 2087 is shown in relation to the distal tip 2036. The orifice 2081 is a circumferential slit in the wall 2079 of the supply tube 2087 having a width W and an arc length L (FIG. 68). In catheters having a diameter of between about 3 French and about 14 French, or between about 5 French and about 10 French, or about 8 French, the width W of the slit may range between about 0.0005 inch and about 0.0025 inch, or between about 0.0010 inch and about 0.0020 inch, and the arc length L may range between about 0.002 inch and about 0.015 inch, or between about 0.004 inch and about 0.012 inch, or between about 0.005 inch and about 0.010 inch.

Pressurization of fluid (e.g., saline) by the pump base 200/cassette 2016 combination and through the catheter supply lumen 2093 and out the orifice 2081 may form a spray pattern 2077, whose shape is at least partially controlled by the dimensions of the orifice 2081, as well as by the wall thickness of the wall 2079, the viscosity of the fluid or slurry being aspirated and the flow characteristics (e.g., flow rate) of the fluid or slurry being aspirated. The spray pattern 2077 caused by the circumferential slit orifice 2081 is particularly effective at cutting or disrupting portions of thrombus within a significant sector of the interior wall 2085 of the guidewire lumen/aspiration lumen 2032.

Turning to FIG. 69, the y-connector 2010 having a distal end 2075 and a proximal end 2073 is shown in use during a thrombus aspiration procedure. The structure of the y-connector 2010 is particular in order to optimize the flow 2071 of the fluid or slurry being aspirated, for example, to minimize turbulence, maximize flow rate, and/or minimize pressure head loss. The second female luer 2051 (sideport) is closer to the distal end 2075 of the y-connector 2010 than is the first female luer 2055 (sideport). The y- connector 2010 has an internal cavity 2097 having an inner surface 2063 The second female luer 2051 has an interior space 2069 and an opening 2101 which communicates with the internal cavity 2097 of the y-connector 2010, the opening 2101 having a distal extreme 2061 and a proximal extreme 2059. The opening 2101 of the second female luer 2051 is the first significant discontinuity or interruption in the inner surface 2063 of the internal cavity 2097 of the y-connector 2010 when moving from the distal end 2075 to the proximal end 2073. Thus, flow of the aspirant (aspirated fluid/slurry) is efficiently diverted from the internal cavity 2097 to the interior space 2069 of the second female luer 2051 before it is able to significantly touch or interface with other portions of the interior of the y-connector 2010. For example, the first female luer 2055 (sideport) has an interior space 2103, much of which is filled with the proximal portion 2105 of the supply tube 2087, and bonding material 2111 (e.g., adhesive, epoxy, hot melt) which secures the proximal portion 2105 of the supply tube 2087 to the interior wall 2107 of the first female luer 2055. A projection 2109 of the bonding material 2111 and/or proximal portion 2105 of the supply tube 2087 into the internal cavity 2097 of the y-connector 2010 is the second significant discontinuity or interruption in the inner surface 2063 of the internal cavity 2097 of the y-connector 2010 when moving from the distal end 2075 to the proximal end 2073. Because the second female luer 2051 (sideport) is closer to the distal end 2075 of the y-connector 1010 than is the first female luer 2055 (sideport), the flow 2071 of the fluid or slurry being aspirated avoids contact with the second discontinuity/interruption. The internal cavity 2097, the interior space 2069, and the interior space 2103 may each have a circular cross-section having a cylindrical-shaped inner surface. Alternatively, each or all may have a non-circular cross-section (e.g., elliptical). In addition to the second female luer 2051 (sideport) being closer to the distal end 2075 of the y-connector 2010, the inner diameter of the interior space 2069 can be made large enough that it is not flow limiting. The length of the interior space 2069 can also be made short enough that it is not flow limiting.

In use, the first female luer 2055 of the system for aspirating thrombus 2000 is coupled to a fluid source 20 and the second female luer 2051 is coupled to a vacuum source (e.g., syringe 2049) by a user or by an assistant. The cassette 2016 is then coupled to the pump base 200 as described herein. The pump base 200 is then operated such that fluid from the fluid source 20 is injected through the supply lumen 2093 and through the orifice 2081 into the aspiration lumen 2032. The pump base 200 is manipulated or commanded in order to adjust the settings on the pump base 200. For example, the pump base 200 may be operated such that an input pressure of the supply lumen 2093 is between about 650 pounds per square inch and about 1200 pounds per square inch. The pump base 200 may be operated such that an input pressure of the supply lumen 2093 is between about 650 pounds per square inch and about 1000 pounds per square inch. The pump base 200 may be operated such that an input pressure of the supply lumen 1093 is between about 800 pounds per square inch and about 1000 pounds per square inch. In addition, the pulsatility of the pump may be adjusted, such that the frequency of injection pulses is increased or decreased. A total flow rate of between about 25 milliliters per minute and about 35 milliliters per minute may be utilized, or between about 28 milliliters per minute and about 33 milliliters per minute, or between about 30 milliliters per minute and about 32 milliliters per minute.

FIG. 70 illustrates an aspiration catheter 2018 A with a y-connector 2200 having a distal end 2202 and a proximal end 2204 shown in use during a thrombus aspiration procedure. The aspiration catheter 2018 A is similar to the aspiration catheter 2018 described in relation to FIG. 64. The structure of the y-connector 2200, as in the y- connector 2010 of FIG. 69, is particular in order to optimize the flow 2071 of the fluid or slurry being aspirated, for example, to minimize turbulence, maximize flow rate, and/or minimize pressure head loss. The second female luer 2206 (sideport) is closer to the distal end 2202 of the y-connector 2200 than is the first female luer 2208 (sideport). The y-connector 2200 has an internal cavity 2210 having an inner surface 2212. The second female luer 2206 has an interior space 2214 and an opening 2216 which communicates with the internal cavity 2210 of the y-connector 2200, the opening 2216 having a distal extreme 2218 and a proximal extreme 2220. The opening 2216 of the second female luer 2206 is the first significant discontinuity or interruption in the inner surface 2212 of the internal cavity 2210 of the y-connector 2200 when moving from the distal end 2202 to the proximal end 2204. Thus, flow of the aspirant (aspirated fluid/slurry) is efficiently diverted from the internal cavity 2210 to the interior space 2214 of the second female luer 2206 before it is able to significantly touch or interface with other portions of the interior of the y-connector 2200. For example, the first female luer 2208 (sideport) has an interior space 2222, much of which is filled with the proximal portion 2224 of the supply tube 2226, and bonding material 2228 (e.g., adhesive, epoxy, hot melt) which secures the proximal portion 2224 of the supply tube 2226 to the interior wall 2230 of the first female luer 2208. A projection 2232 of the bonding material 2228 and/or proximal portion 2224 of the supply tube 2226 into the internal cavity 2210 of the y-connector 2200 is the second significant discontinuity or interruption in the inner surface 2212 of the internal cavity 2210 of the y-connector 2200 when moving from the distal end 2202 to the proximal end 2204. Because the second female luer 2206 (sideport) is closer to the distal end 2202 of the y-connector 2200 than is the first female luer 2208 (sideport), the flow 2071 of the fluid or slurry being aspirated avoids contact with the second discontinuity/interruption. The internal cavity 2210, the interior space 2214, and the interior space 2222 may each have a circular cross-section having a cylindrical-shaped inner surface. Alternatively, each or all may have a non-circular cross-section (e.g., elliptical).

A sensor connector 2234 and a valved connector 2236 are connected in series to the y-connector 2200. A male luer 2238 at the distal end 2240 of the sensor connector 2234 is connected to a female luer 2242 at the proximal end 2204 of the y-connector 2200. A male luer 2244 at the distal end 2246 of the valved connector 2236 is connected to a female luer 2248 at the proximal end 2250 of the sensor connector 2234. The sensor connector 2234 has an inner bore 2252, and includes a pressure sensor 2006 within the inner bore 2252. Signals from the pressure sensor 2006 are carried through a cable 2012, as described in earlier embodiments herein. A valve 2254, for example a Touhy-Borst, at the proximal end 2235 of the valved connector 2236 allows hemostasis of the guidewire lumen/aspiration lumen 2032 around a guidewire 2091. In other embodiments, the valve 2254 may comprise a spring-loaded seal. The guidewire 2091 may be inserted entirely through the guidewire lumen/aspiration lumen 2032, passing also through a bore 2256 in the valved connector 2236, the inner bore 2252 in the sensor connector 2234, and the internal cavity 2210 in the y-connector 2200. With this configuration, the sensor connector 2234 and/or the valved connector 2236 may be easily replaced, if necessary, while maintaining the aspiration catheter 2018 A in position, for example, within a blood vessel.

FIG. 71 illustrates an aspiration catheter 2018B with a y-connector 2300 having a distal end 2302 and a proximal end 2304 shown in use during a thrombus aspiration procedure. The aspiration catheter 2018B is similar to the aspiration catheter 2018 described in relation to FIG. 64. The structure of the y-connector 2300, as in the y- connector 2010 of FIG. 69, is particular in order to optimize the flow 2071 of the fluid or slurry being aspirated, for example, to minimize turbulence, maximize flow rate, and/or minimize pressure head loss. The second female luer 2306 (sideport) is closer to the distal end 2302 of the y-connector 2300 than is the first female luer 2308 (sideport). The y- connector 2300 has an internal cavity 2310 having an inner surface 2312. The second female luer 2306 has an interior space 2314 and an opening 2316 which communicates with the internal cavity 2310 of the y-connector 2300, the opening 2316 having a distal extreme 2318 and a proximal extreme 2320. The opening 2316 of the second female luer 2306 is the first significant discontinuity or interruption in the inner surface 2312 of the internal cavity 2310 of the y-connector 2300 when moving from the distal end 2302 to the proximal end 2304. Thus, flow of the aspirant (aspirated fluid/slurry) is efficiently diverted from the internal cavity 2310 to the interior space 2314 of the second female luer 2306 before it is able to significantly touch or interface with other portions of the interior of the y-connector 2300. For example, the first female luer 2308 (sideport) has an interior space 2322, much of which is filled with the proximal portion 2324 of the supply tube 2326, and bonding material 2328 (e.g., adhesive, epoxy, hot melt) which secures the proximal portion 2324 of the supply tube 2326 to the interior wall 2330 of the first female luer 2308. A projection 2332 of the bonding material 2328 and/or proximal portion 2324 of the supply tube 2326 into the internal cavity 2310 of the y-connector 2300 is the second significant discontinuity or interruption in the inner surface 2312 of the internal cavity 2310 of the y-connector 2300 when moving from the distal end 2302 to the proximal end 2304. Because the second female luer 2306 (sideport) is closer to the distal end 2302 of the y-connector 2300 than is the first female luer 2308 (sideport), the flow 2071 of the fluid or slurry being aspirated avoids contact with the second discontinuity/interruption. The internal cavity 2310, the interior space 2314, and the interior space 2322 may each have a circular cross-section having a cylindrical-shaped inner surface. Alternatively, each or all may have a non-circular cross-section (e.g., elliptical).

A sensor connector 2334 and a valved connector 2336 are connected to the y- connector 2300. A male luer 2338 at the distal end 2340 of the valved connector 2336 is connected to a female luer 2342 at the proximal end 2304 of the y-connector 2300. A male luer 2344 at the distal end 2346 of the sensor connector 2334 is connected to a female luer 2348 at an intermediate portion 2350 of the valved connector 2336. The sensor connector 2334 has an inner bore 2352, and includes a pressure sensor 2006 within the inner bore 2352. Signals from the pressure sensor 2006 are carried through a cable 2012, as described in earlier embodiments herein. A valve 2354, for example a Touhy- Borst, at the proximal end 2335 of the valved connector 2336 allows hemostasis of the guidewire lumen/aspiration lumen 2032 around a guidewire 2091. In other embodiments, the valve 2354 may comprise a spring-loaded seal. The guidewire 2091 may be inserted entirely through the guidewire lumen/aspiration lumen 2032, passing also through a bore 2356 in the valved connector 2336, and the internal cavity 2310 in the y-connector 2300. With this configuration, the sensor connector 2334 and/or the valved connector 2336 may be easily replaced, if necessary, while maintaining the aspiration cateter 2018B in position, for example, within a blood vessel. The sensor connector 2334 additionally includes an inlet 2358, which may be used to inject fluid, such as saline or contrast media or a mixture of the two. The inlet 2358 may comprise a female luer configured for coupling a male luer of a syringe. In some embodiments, the inlet 2358 may comprise a rubber septum 2360, configured for repeatable penetration of the needle of a syringe therethrough. In alternative embodiments, the pressure sensor 2006 may be placed at a number of different alternate locations.

FIGS. 72-74 illustrate a system for aspirating thrombus 2400. The system for aspirating thrombus 2400 includes many similarities to and uses several components of the system for aspirating thrombus 1900 of FIG. 61, including the aspiration catheter 1930, y-connector 1910, having a female luer 1912 hydraulically coupled to the high pressure injection lumen 1934 (FIG. 62) and a female luer 1914 hydraulically coupled to the aspiration lumen 1932 (FIG. 62), an additional y-connector 1916 having a male luer 1918 and female luer 1920 and a touhy-borst 1922, an injection tube 1906 having a male luer 1908, a pressure transducer 106 electrically-connected to a cable 112, and a vacuum line 1926 having a luer fitting 2455. The luer fitting 2455 in the system for aspirating thrombus 2400 of FIGS. 72-74 is a male luer connector, through in alternate embodiments, may be another type of connector. The aspiration catheter 1930 has been inserted through a guiding catheter 2450 having a hemostasis valve 2452 configured for sealing around the shaft 2454 of the aspiration catheter 1930. Fluid (e.g., saline) may be injected through the interior lumen 2456 of the guiding catheter 2450, and around the shaft 2454 of the aspiration catheter 1930 by attaching a syringe or pump to the luer connection 2458 of an extension tube 2460 while the stopcock 2462 coupled to the luer connection 2458 is in an open condition. The stopcock 2462 is shown, however, in a closed condition in FIGS. 72-74. A guidewire 1902 can be used in conjunction with the system for aspirating thrombus 2400.

A new feature in the system for aspirating thrombus 2400 of FIGS. 72-74 is a four- way stopcock 2402 connected between the vacuum line 1926, the pressure transducer 106, and the aspiration catheter 1930. The four-way stopcock 2402 includes a male luer 2404, that is fluidly coupled to the female luer 1920 of the y-connector 1916, a female luer 2406, that is fluidly coupled to the (male) luer fitting 2455 of the vacuum line 1926, and a female luer 2408, that is fluidly coupled to a male luer 2457 of the pressure transducer 106. The four- way stopcock 2402 includes a main housing 2410 having three inlets/outlets 2412, 2414, 2416, and a rotatable valve body 2418 which is rotated via a projection 2420. In FIG. 72, the valve body 2418 is in an "aspiration" position, which allows fluid communication between all of the pressure transducer 106, the vacuum line 1926 (and thus, the syringe 2049), and the aspiration lumen 1932 of the aspiration catheter 1930. The distal end 1997 of the aspiration catheter 1930 is shown within a blood vessel 1999 having a thrombus 1995. The projection 2420 points in the direction of the "closed" side of the valve body 2418, and thus, in FIG. 72 is not closing off any of the three inlets/outlets 2412, 2414, 2416. A controller, for example, carried on the circuit board 304 of FIG. 17, is configured to receive signals from the pressure transducer 106 to obtain information as to the pressure sensed by the pressure transducer 106. The controller is further configured to stop operation of the pump 200 if the signals received from the pressure transducer 106 indicate that the pressure transducer is not communicating with a negative pressure ("vacuum"), with an intention to assure that injection of fluid occurs through the high-pressure injection lumen 1934 only when the vacuum source is actively causing the aspiration lumen 1932 to aspirate thrombus 1995. For example, in FIG. 73, the user has turned the valve body 2418 to an "off position in relation to the vacuum line 1926 and syringe 2049. The inlets/outlets 2412, 2414 are open and the inlet/outlet 2416 is closed. Thus, the pressure transducer 106 does not measure the negative pressure of the vacuum line 1926, and instead measures the pressure near the proximal end of the aspiration lumen 1932 (actually the pressure adjacent the interior of the female luer 1920 of the y-connector 1916). After the valve body 2418 is turned to this particular "off position, the pressure measured by the pressure transducer 106 will increase, causing the controller to stop the operation of the pump 200. The intention is to insure not to inject into the blood vessel or disrupt thrombus in the blood vessel when not aspiration is actively being performed.

However, there are instances in which it may be desired to perform a power pulse or power injection with the pump 200, without an active vacuum applied on the aspiration lumen 1932 of the aspiration catheter 1930. The four-way stopcock 2402 allows the pressure transducer 106 (and thus, the controller) to be "tricked" without having to reprogram or reconfigure the controller. Turning to FIG. 74, the valve body 2418 has been turned by the user to a "power inject' position, wherein the pressure transducer 106 is in fluid communication with the vacuum line 1926 (and vacuum source/syringe 2049), but the aspiration lumen 1932 of the aspiration catheter 1930 is not in fluid communication with the vacuum line 1926 (or vacuum source/syringe 2049), or the pressure transducer 106. The inlet/outlet 2412 is closed, isolating the aspiration lumen 1932 from the pressure transducer 106 and the vacuum line 1926. The inlets/outlets 2414/2416 are open, and only allow fluid communication between the pressure transducer 106 and the vacuum line 1926. Thus, still allowing (via the controller) injection of fluid through the high-pressure injection lumen 1934, and out the orifice 1946 (FIG. 62) of the high-pressure injection lumen 1934 and out the distal orifice of the aspiration lumen 1932 into the blood vessel 1999. The power pulse or power injection may be used to deliver a forceful disturbance to thrombus 1995 in the blood vessel 1999, or may be used to increase the amount of lower viscosity fluid (e.g., saline) 1993 around the thrombus 1995 (to aid in subsequent aspiration attempts). Contrast media may also be added to the injection fluid or may be used as the injection fluid, so that the power pulse increases the radi opacity within the blood vessel 1999, in the vicinity of the thrombus 1995.

In certain situations, aspiration of thrombus 1995 may become difficult through the aspiration lumen 1932 of the aspiration catheter 1930 during an aspiration procedure. One reason that may cause this is if the thrombus 1995 surrounding the distal end 1997 of the aspiration catheter 1930 is of a substantially high viscosity, thus making it difficult for the thrombus 1995 to begin flowing into the distal opening and through the aspiration lumen 1932 of the aspiration catheter 1930. In these situations, a syringe, or a second pump may be attached to the luer connection 2458 of the extension tube 2460 (with the stopcock 2062 in the open position) and saline (and/or contrast media) may be injected through the interior lumen 2456 of the guiding catheter 2450. The outer diameter of the aspiration catheter 1930 is sized sufficiently smaller than the inner diameter of the interior lumen 2456 of the guiding catheter 2450 such that a hand injection is possible without straining. The use of at least some contrast media allows for a visual diagnostic of the catheter flow status (e.g., aspiration) as well as the thrombus location and even thrombus shape or contour. The distal end 2451 of the guiding catheter may be moved (if needed) to place it in sufficient proximity with the distal end 1997 of the aspiration catheter 1930 and/or the target portion of thrombus 1995 to be aspirated. By injecting a bolus of fluid having substantially the viscosity of water or saline, or at least a fluid whose viscosity is on the same order as that of water or saline, or even blood, the initiation of aspiration at the target thrombus site and entry into the aspiration lumen 1932 of the aspiration catheter 1930 is facilitated. This may happen because the overall (bulk) viscosity is lowered. Once the somewhat diluted thrombus 1991 begins to flow through the aspiration catheter with the application of the pump to the high-pressure injection lumen 1934, the aspiration procedure tends to continue, as it is now in a dynamic state, instead of an initially static state. The procedure may be continually or continuously assessed using the pressure sensor 106, or by visualization with angiography/fluoroscopy. In some embodiments, a second pump (not shown) may be attached to the luer connection 2458 of the extension tube 2460, and may be triggered by a controller which is carried on either the first pump 200, 400 or on the pressure sensor 106. The second pump may be turned on or turned off based on a threshold pressure measured by the pressure sensor 106 that is met or crossed.

Alternatively, saline, contrast, or other fluids may even be injected in a retrograde fashion, by turning the valve body 2418 of the four-way stopcock 2402 to the particular "off position of FIG. 73. This will stop the action of the pump 200, if the pump has not already been stopped by other means. A luer cap 2422 on the pressure transducer 106 may now be removed and the fluid may be injected (e.g., with a syringe) retrograde through the access luer 2424 of the pressure transducer 106 and through the aspiration lumen 1932 from proximal end to distal end and out the distal opening of the aspiration lumen 1932 into the blood vessel. The valve body 2418 may be used as a sterile on/off switch to perform a number of different sub-procedures within the aspiration/thrombectomy procedure.

FIG. 75 illustrates the system for aspirating thrombus 2400 further comprising a guiding catheter 2426 (or long sheath) configured for placing the aspiration catheter 1930 therethrough. A blood vessel 2428 includes thrombus 2430 therein. The guiding catheter 2426 includes a tubular shaft 2434, a distal opening 2436, and a proximal end 2438 having a valve 2440 with a sideport extension tube 2442 having a luer 2444 for injection and a stopcock 2446. The luer 2444 of the extension tube 2442 may be fluidly coupled to sources of fluids, such as a saline bottle or bag, or a contrast media bottle or bag. In use, as shown in FIG. 75, the distal end 1997 of the aspiration catheter 1930 has been extended out of the distal opening 2436 of the guiding catheter 2426 in order to perform a thrombectomy procedure according to embodiments described herein. If during the procedure, a user determines that aspiration of the thrombus 2430 through the aspiration lumen 1932 of the aspiration catheter 1930 is not occurring at a desired thrombus aspiration rate (flow rate, mass removal rate, etc.), the user grasps the guiding catheter 2426 by the tubular shaft 2434 and spins the guiding catheter 2426 (arrow) within the blood vessel 2428 to increase the thrombus aspiration rate. The circumferential shear caused by the rotating cylindrical surface area of the tubular shaft 2434 of the guiding catheter 2426 serves to disrupt or macerate the thrombus, particularly in the area near the distal opening 2436. The status of the aspiration of thrombus may be determined by data received from the pressure transducer 106, or by visual evidence (amount of thrombus seen passing through clear tubing or connectors). The guiding catheter 2426 may be spun around axis 2427 while the aspiration catheter 1930 has fluid injected through the high-pressure injection lumen 1934 and/or while the vacuum source (syringe 2049) is applied to the aspiration lumen 1932. Alternatively, the pump 200 and or vacuum source may be temporarily stopped while the rotation of the guiding catheter 2426 is performed. As shown in FIG. 75, the extension tube 2442 and luer 2444 have been decoupled from any fluid sources in order to facilitate the untethered rotatability of the guiding catheter 2426. The inner material of the valve 2440 is typically low friction and allows a smooth and sealed rotation over the shaft of the aspiration catheter 1930. Alternatively, instead of the valve 2440, the guiding catheter may have a proximal luer connector and may be attached to a rotatable y-connector or other rotatable (swivel) connector, which also allows rotation of the guiding catheter 2426.

FIG. 76 illustrates an aspiration catheter 1518 with a y-connector 1500 having a distal end 1502 and a proximal end 1504 configured for use in a thrombus aspiration procedure. The y-connector 1500 includes a first female luer 1508 (sideport) which allows injection through the interior of a supply tube 1526 of the aspiration catheter 1518. The first female luer 1508 (sideport) has an interior space 1522, much of which is filled with the proximal portion 1524 of the supply tube 1526 and bonding material 1528 (e.g., adhesive, epoxy, hot melt) which secures the proximal portion 1524 of the supply tube 1526 to the interior wall 1530 of the first female luer 1508. A projection 1532 of the bonding material 1528 and/or proximal portion 1524 of the supply tube 1526 into the internal cavity 1510 of the y-connector 1500 may cause a discontinuity or interruption in the inner surface 1512 of the internal cavity 1510 of the y-connector 1500. The internal cavity 1510 and the interior space 1522 may each have a circular cross-section having a cylindrical-shaped inner surface. Alternatively, each or all may have a non-circular cross- section (e.g., elliptical). The female luer 1508 is configured for attaching a male luer 2054 of an injection tube 2052 so that pressurized saline may be injected through the supply tube 1526. The proximal end 1504 of the y-connector 1500 includes a female luer 1542.

A combined connector 1536 includes a male luer 1538 at its distal end 1540 which is configured to be connected to the female luer 1542 of the y-connector 1500. The combined connector 1536 includes a first inner bore 1556 and a second inner bore 1552 which are co-communicating, the second inner bore 1552 including a pressure sensor 2006 communicating therein. Signals from the pressure sensor 2006 are carried through a cable 2012, as described in earlier embodiments herein. A valve 1554, for example a Touhy-Borst, at the proximal end 1535 of the combined connector 1536 allows hemostasis of the guidewire lumen/aspiration lumen 2032 around a guidewire 2091. In other embodiments, the valve 1554 may comprise a spring-loaded seal. The guidewire 2091 may be inserted entirely through the guidewire lumen/aspiration lumen 2032, passing also through the bore 1556 in the combined connector 1536, and the internal cavity 1510 in the y-connector 1500. The combined connector 1536 further comprises a female luer 1506 configured for attaching the male luer 2053 of the vacuum line 2008. With this configuration, the combined connector 1536 may be easily replaced, if necessary, while maintaining the aspiration catheter 1518 in position, for example, within a blood vessel. The combined connector 1536 additionally includes an inlet 1558, which may be used to inject fluid, such as saline or contrast media or a mixture of the two, for example, when aspiration is not actively occurring. The inlet 1558 may comprise a female luer configured for coupling a male luer of a syringe. In some embodiments, the inlet 1558 may comprise a rubber septum 1560, configured for repeatable penetration of the needle of a syringe therethrough. During aspiration, a vacuum is applied to the female luer 1506 and the pressure is measured by the pressure sensor 2006.

FIG. 78 illustrates a connector 1620 for use in subsequently presented embodiments of aspiration catheters, including the aspiration catheter 1616 of FIG. 79. The connector 1620 comprises a molded, cast or otherwise formed body which may comprise a rigid polymer, such as polycarbonate, acrylic, polyester-polycarbonate blend, or acrylonitrile-butadiene-styrene (ABS). The connector 1620 includes a main body 1619 including a proximal female luer 1634 having male threads 1635. The connector 1620 further includes a first sideport 1622 having a female luer 1621 and male threads 1637. The connector 1620 further includes a second, dual -use sideport 1628 having a female luer 1629 and male threads 1639. The sideport 1628 also includes a barbed fitting 1633, thus allowing either attachment of a male luer to the female luer 1629 (or if threaded, to the female luer 1629 and threads 1639) or a frictional fitting (tubular inner diameter) to the barbed fitting 1633. The barbed fitting 1633 may be particularly useful for frictionally attaching tubing from a vacuum line. The multipurpose utility of the sideport 1628 allows it to be easily used with a variety of commercially available vacuum sources, including, but not limited to, syringes, VacLok® syringes, vacuum pumps, house vacuum lines, vacuum canisters, or vacuum bottles. When the sideport 1628 is used for infusion, the female luer 1629 would be commonly used, but the barbed fitting 1633 also allows for alternative infusion connections.

FIG. 79 illustrates an aspiration catheter 1616 comprising a shaft 1618 and a connector 1620. The connector 1620 includes a female luer sideport 1622 which allows injection through the interior of a supply tube 1623 of the aspiration catheter 1616 via a fluid supply line 1624 having a male luer 1626. The male luer 1626 is connected to the female luer 1621 of the sideport 1622. The connector 1620 includes a barbed fitting sideport 1628 which is configured for attachment of a vacuum line 1630 having a plastic or elastomeric tubular end 1632 configured for sealingly forcing over the barbed fitting 1628. The tubular end may comprise Tygon®, PVC, or silicone or other appropriate materials. The connector 1620 further includes a proximal female luer 1634, which is shown capped off by a luer cap 1636. If the aspiration catheter 1616 is used without a guidewire, the luer cap 1636 may be left in place over the luer 1643. If a guidewire is used, the luer cap 1636 may be removed from the luer 1634, and the guidewire inserted through the interior of the connector 1620 and through the aspiration lumen of the aspiration catheter 1616. Alternatively, with the luer cap 1636 removed, an additional pump and injection tube having a male luer may be attached to the female luer 1634 of the connector 1620, to access the aspiration lumen of the aspiration catheter 1616. The pump may be used to apply a positive pressure and force saline distally, for example, when vacuum is not being significantly applied or applied at all to the aspiration lumen via the vacuum line 1630. The purpose of the injection of fluid through the additional pump may be for potential declogging of the aspiration lumen. Declogging may be particularly helpful in venous cases, where any distal emboli of clog material (e.g., clot) ejected from the aspiration lumen into the vein is of lesser concern than in a procedure located in a coronary or cerebral artery. In some cases, the fluid injected to unblock the aspiration lumen may be alternated with the injection of contrast media (undiluted or dilute) to aid in the visualization of the target area, e.g., via fluoroscopy. The injection of fluid through the aspiration lumen may also aid in lowering the bulk viscosity of the thrombus and blood surrounding the thrombus (e.g., at the target site) to aid in its subsequent aspiration into the aspiration lumen of the aspiration catheter 1616.

FIG. 80 illustrates an aspiration catheter 1638 comprising a shaft 1640 and a connector 1642. The connector 1642 includes a female luer sideport 1644 which allows injection through the interior of a supply tube 1647 of the aspiration catheter 1638 via a fluid supply line 1646 having a male luer 1648. The connector 1642 includes a barbed fitting 1650 (sideport) which is configured for attachment of a vacuum line 1652 having a plastic or elastomeric tubular end 1654 configured for sealingly forcing over the barbed fitting 1650. In some embodiments, the barbed fitting 1650 may also include a female luer. The connector 1642 further includes a Touhy-Borst valve 1656 which may be sealed (closed) is a guidewire is not used, and may be opened to allow the passage of a guidewire through the connector 1642 and the aspiration lumen of the aspiration catheter 1638, and may be sealed over the guidewire. The Touhy-Borst valve 1656 may include a distal male luer 1657 configured to secure to a female luer 1659 at the proximal end of the connector 1642. In alternate embodiments, the Touhy-Borst valve 1656 may be permanently connected or formed on the connector 1642. As an alternative to the foot switch 2021 operated actuation (FIG. 64) of the pinch valve 1610 (FIG. 77), the vacuum line 1652 (FIG. 80) can be manually clamped and undamped in order to close and open the vacuum line 1652. The manual clamping would be performed at a location on the vacuum line that is between the vacuum source 22 and the pressure sensor 1608 (FIG. 77), and, if there is a pinch valve 1610 along the vacuum line 1652, the pinch valve 1610 would have to be open to allow this manual clamping functionality. Alternatively, instead of a manual clamp, the vacuum line 1652 may be removed by pulling the elastomeric tubular end 1654 from the barbed fitting 1650, and capping off or otherwise occluding the lumen of the vacuum line 1652.

FIG. 81 illustrates an aspiration catheter 1658 comprising a shaft 1660 and a connector 1662. The connector 1662 includes a female luer sideport 1664 which allows injection through the interior of a supply tube 1667 of the aspiration catheter 1658 via a fluid supply line 1666 having a male luer 1668. The connector 1662 includes a barbed fitting 1670 (sideport) which is configured for attachment of a vacuum line 1672 having a plastic or elastomeric tubular end 1674 configured for sealingly forcing over the barbed fitting 1670. In some embodiments, the barbed fitting 1670 may also include a female luer. The connector 1662 further includes a proximal female luer 1676. A y-connector 1678 includes a male luer 1680 which is attached to the female luer 1676 of the connector 1662. The y-connector 1678 further includes a Touhy-Borst valve 1682 which may be sealed (closed) is a guidewire is not used, and may be opened to allow the passage of a guidewire through the y-connector 1678, the connector 1662, and the aspiration lumen of the aspiration catheter 1658, and may be sealed over the guidewire. The y-connector 1678 further includes a female luer sideport 1684 to which a syringe or other implement may be connected, for example to inject fluids or drugs. An additional pump and injection tube having a male luer may be attached to the luer sideport 1684 of the y-connector 1678, to access the aspiration lumen of the aspiration catheter 1658. The pump may be used to apply a positive pressure and force saline distally, for example, when vacuum is not being significantly applied or applied at all to the aspiration lumen via the vacuum line 1672. The purpose of the injection of fluid through the additional pump may be for potential declogging of the aspiration lumen. Declogging may be particularly helpful in venous cases, where any distal emboli of clog material (e.g., clot) ejected from the aspiration lumen into the vein is of lesser concern than in a procedure located in a coronary or cerebral artery. In some cases, the fluid injected to unblock the aspiration lumen may be alternated with the injection of contrast media (undiluted or dilute) to aid in the visualization of the target area, e.g., via fluoroscopy. The injection of fluid through the aspiration lumen may also aid in lowering the bulk viscosity of the thrombus and blood surrounding the thrombus (e.g., at the target site) to aid in its subsequent aspiration into the aspiration lumen of the aspiration catheter 1658. Injection of contrast or saline, or other fluid, may be performed by a pump, or by hand/syringe injection, via attachment to the female luer sideport 1684. Alternatively, the y-connector 1678 may be removed by detaching the male luer 1680 from the female luer 1676 of the connector 1662, and then attaching the pump or syringe to the female luer 1676 and injecting.

In some cases, parts or all of the devices described herein may be doped with, made of, coated with, or otherwise include a radiopaque material. Radiopaque materials are understood to be materials capable of producing a relatively bright image on a fluoroscopy screen or another imaging technique during a medical procedure. Some examples of radiopaque materials can include, but are not limited to, gold, platinum, palladium, tantalum, tungsten alloy, polymer material loaded with a radiopaque filler, and the like. One or more hydrophilic or hydrophobic lubricious coatings may be used in order to improve trackability of the aspiration catheter 118 through the blood vessels.

In some instances, a degree of MRI compatibility may be imparted into parts of the devices described herein. For example, to enhance compatibility with Magnetic Resonance Imaging (MRI) machines, it may be desirable to make various portions of the devices described herein from materials that do not substantially distort MRI images or cause substantial artifacts (gaps in the images). Some ferromagnetic materials, for example, may not be suitable as they may create artifacts in an MRI image. In some cases, the devices described herein may include materials that the MRI machine can image. Some materials that exhibit these characteristics include, for example, tungsten, cobalt-chromium-molybdenum alloys (e.g., UNS: R30003 such as ELGILOY®, PHYNOX®, and the like), nickel-cobalt-chromium-molybdenum alloys (e.g., UNS: R30035 such as MP35-N® and the like), nitinol, and the like, and others.

In some instances, some of the devices described herein may include a coating such as a lubricious coating or a hydrophilic coating. Hydrophobic coatings such as fluoropolymers provide a dry lubricity. Lubricious coatings improve steerability and improve lesion crossing capability. Suitable lubricious polymers are well known in the art and may include silicone and the like, hydrophilic polymers such as high-density polyethylene (HDPE), polytetrafluoroethylene (PTFE), polyarylene oxides, polyvinylpyrrolidones, polyvinylalcohols, hydroxy alkyl cellulosics, algins, saccharides, caprolactones, and the like, and mixtures and combinations thereof. Hydrophilic polymers may be blended among themselves or with formulated amounts of water insoluble compounds (including some polymers) to yield coatings with suitable lubricity, bonding, and solubility.

It should be understood that this disclosure is, in many respects, only illustrative. Changes may be made in details, particularly in matters of shape, size, and arrangement of steps without exceeding the scope of the disclosure. The scope of the disclosure is, of course, defined in the language in which the appended claims are expressed.

While embodiments of the present disclosure have been shown and described, various modifications may be made without departing from the scope of the present disclosure as defined by the appended claims. Embodiments of the present disclosure are contemplated to have utility in a variety of blood vessels, including but not limited to coronary arteries, carotid arteries, intracranial/cerebral arteries, inferior and superior vena cavae and other veins (for example, in cases of deep venous thrombosis or pulmonary embolism), peripheral arteries, shunts, grafts, vascular defects, and chambers of the heart. This includes, but is not limited to, any vessel having a diameter of bout two mm or greater. An aspiration catheter 118 outer diameter of about seven French or less is contemplated for many of the applications, though in certain applications, it may be larger. In some embodiments, an aspiration catheter 118 diameter of about six French or less is contemplated. Embodiments of the present disclosure may even be used in non-vascular applications, for example body lumens or cavities having material accumulations that need to be macerated and/or removed.

It is contemplated that various combinations or subcombinations of the specific features and aspects of the embodiments disclosed above may be made and still fall within one or more of the embodiments. Further, the disclosure herein of any particular feature, aspect, method, property, characteristic, quality, attribute, element, or the like in connection with an embodiment can be used in all other embodiments set forth herein. Accordingly, it should be understood that various features and aspects of the disclosed embodiments can be combined with or substituted for one another in order to form varying modes of the disclosed embodiments. Thus, it is intended that the scope of the present disclosure herein disclosed should not be limited by the particular disclosed embodiments described above. Moreover, while the present disclosure is susceptible to various modifications, and alternative forms, specific examples thereof have been shown in the drawings and are herein described in detail. It should be understood, however, that the present disclosure is not to be limited to the particular forms or methods disclosed, but to the contrary, the present disclosure is to cover all modifications, equivalents, and alternatives falling within the scope of the various embodiments described and the appended claims. Any methods disclosed herein need not be performed in the order recited. The methods disclosed herein include certain actions taken by a practitioner, however, they can also include any third-party instruction of those actions, either expressly or by implication.

The ranges disclosed herein also encompass any and all overlap, sub-ranges, and combinations thereof. Language such as "up to," "at least," "greater than," "less than," "between," and the like includes the number recited. Numbers preceded by a term such as "approximately", "about", and "substantially" as used herein include the recited numbers (e.g., about 10%= 10%), and also represent an amount close to the stated amount that still performs a desired function or achieves a desired result. For example, the terms "approximately", "about", and "substantially" may refer to an amount that is within less than 10% of, within less than 5% of, within less than 1% of, within less than 0.196 of, and within less than 0.01% of the stated amount.

## Claims

1. A system for aspirating thrombus (2000), comprising:
   an aspiration catheter (2018) comprising:

   an elongate shaft (2042) configured for placement within a blood vessel of a subject;
   a supply lumen (2093) and an aspiration lumen (2032) each extending within the shaft (2042) and separated by a wall (2079), wherein the wall (2079) forms a supply tube (2087) forming the supply lumen (2093) and an interior wall (2085) of the elongate shaft (2042) forms the aspiration lumen (2032), the supply lumen (2093) having a proximal end and a distal end, and the aspiration lumen (2032) having a proximal end (2019) and an open distal end (2036), a portion of the supply tube (2087) being configured to move within the aspiration lumen (2032) due to flow force exerted over an external surface of the supply tube (2087) so that a remaining inner luminal space of the aspiration lumen (2032) self-optimizes for flow of aspirated thrombus; and
   a circumferentially-extending slit (2081) in the wall (2079) at or near the distal end of the supply lumen (2093), the slit (2081) being configured to allow the injection of pressurized fluid into the aspiration lumen (2032) at or near the distal end of the aspiration lumen (2032) when the pressurized fluid is pumped through the supply lumen (2093).

2. The system of claim 1, wherein the slit has a width (W) oriented in the direction of the longitudinal axis of the aspiration catheter (2018) and an arc length (L) oriented circumferentially on an outer surface of the tubular structure (2087).

3. The system of claim 2, wherein the width (W) is between about 25.4 μm (0.0010 inch) and about 63.5 μm (0.0025 inch).

4. The system of claim 3, wherein the width (W) is between about 38.1 μm (0.0015 inch) and about 50.8 μm (0.0020 inch).

5. The system of claim 2, wherein the arc length (L) is between about 50.8 μm (0.002 inch) and about 254 μm (0.010 inch).

6. The system of claim 5, wherein the arc length (L) is between about 101.6 μm (0.004 inch) and about 203.2 μm (0.008 inch).

7. The system of claim 6, wherein the arc length (L) is between about 127 μm (0.005 inch) and about 177.8 μm (0.007 inch).

8. The system of claim 1, further comprising a connector (2010) hydraulically coupled to the proximal end of the aspiration lumen (2032), the connector (2010) having an interior cavity (2087), a proximal end (2073) and a distal end (2075).

9. The system of claim 1, wherein the slit (2081) creates a spray pattern (2077) of the pressurized fluid on a sector of an interior wall (2085) of the aspiration lumen (2032).

10. The system of claim 9, wherein the sector of the interior wall (2085) comprises portions of the interior wall opposite to the slit (2081) and proximal and distal to the slit (2081).

11. The system of claim 10, wherein the supply tube (2087) is disposed within the aspiration lumen (2032).

12. The system of claim 10, wherein the distal end of the supply lumen (2093) is proximal the distal end (2036) of the aspiration lumen (2032).

13. The system of claim 10, further comprising a plug (2083) secured within the supply lumen distal the slit (2081).

## Patentansprüche

1. Ein System zum Absaugen von Thromben (2000), das Folgendes umfasst:
einen Absaugkatheter (2018), der Folgendes umfasst:

einen langgestreckten Schaft (2042), der zur Einführung in ein Blutgefäß eines Patienten ausgelegt ist;
ein Versorgungslumen (2093) und ein Aspirationslumen (2032), die sich jeweils innerhalb des Schafts (2042) erstrecken und durch eine Wand (2079) getrennt sind, wobei die Wand (2079) eine Versorgungsröhre (2087) bildet, die das Versorgungslumen (2093) bildet, und eine Innenwand (2085) des länglichen Schafts (2042) das Aspirationslumen (2032) bildet, wobei das Versorgungslumen (2093) ein proximales Ende und ein distales Ende aufweist, und das Aspirationslumen (2032) ein proximales Ende (2019) und ein offenes distales Ende (2036) aufweist, wobei ein Teil der eine Versorgungsröhre (2087) so gestaltet ist, dass er sich aufgrund der über eine äußere Oberfläche der Versorgungsröhre (2087) ausgeübten Strömungskraft innerhalb des Aspirationslumens (2032) bewegt, so dass ein verbleibender innerer Lumenraum des Aspirationslumens (2032) sich selbst für den Fluss des aspirierten Thrombus optimiert; und
einen sich in Umfangsrichtung erstreckenden Schlitz (2081) in der Wand (2079) am oder in der Nähe des distalen Endes des Versorgungslumens (2093), wobei der Schlitz (2081) so gestaltet ist, dass er die Injektion von unter Druck stehendem Fluid in das Aspirationslumen (2032) am oder in der Nähe des distalen Endes des Aspirationslumens (2032) ermöglicht, wenn das unter Druck stehende Fluid durch das Versorgungslumen (2093) gepumpt wird.

2. Das System nach Anspruch 1, wobei der Schlitz eine Breite (W) hat, die in Richtung der Längsachse des Aspirationskatheters (2018) orientiert ist, und eine Bogenlänge (L), die in Umfangsrichtung auf einer Außenfläche der röhrenförmigen Struktur (2087) orientiert ist.

3. Das System nach Anspruch 2, wobei die Breite (W) zwischen etwa 25,4 $\mu$m (0,0010 Zoll) und etwa 63,5 $\mu$m (0,0025 Zoll) liegt.

4. Das System nach Anspruch 3, wobei die Breite (W) zwischen etwa 38,1 $\mu$m (0,0015 Zoll) und etwa 50,8 $\mu$m (0,0020 Zoll) liegt.

5. Das System nach Anspruch 2, wobei die Bogenlänge (L) zwischen etwa 50,8 $\mu$m (0,002 Zoll) und etwa 254 $\mu$m (0,010 Zoll) liegt.

6. Das System nach Anspruch 5, wobei die Bogenlänge (L) zwischen etwa 101,6 $\mu$m (0,004 Zoll) und etwa 203,2 $\mu$m (0,008 Zoll) liegt.

7. Das System nach Anspruch 6, wobei die Bogenlänge (L) zwischen etwa 127 $\mu$m (0,005 Zoll) und etwa 177,8 $\mu$m (0,007 Zoll) liegt.

8. Das System nach Anspruch 1 umfasst ferner ein Verbindungsstück (2010), das hydraulisch mit dem proximalen Ende des Ansauglumens (2032) verbunden ist, wobei das Verbindungsstück (2010) einen inneren Hohlraum (2087), ein proximales Ende (2073) und ein distales Ende (2075) aufweist.

9. Das System nach Anspruch 1, wobei der Schlitz (2081) ein Sprühmuster (2077) der unter Druck stehenden Flüssigkeit auf einem Sektor einer Innenwand (2085) des Aspirationslumens (2032) erzeugt.

10. Das System nach Anspruch 9, wobei der Sektor der Innenwand (2085) Teile der Innenwand gegenüber dem Schlitz (2081) und proximal und distal des Schlitzes (2081) umfasst.

11. Das System nach Anspruch 10, wobei die Versorgungsröhre (2087) innerhalb des Ansauglumens (2032) angeordnet ist.

12. Das System nach Anspruch 10, wobei das distale Ende des Versorgungslumens (2093) proximal des distalen Endes (2036) des Aspirationslumens (2032) liegt.

13. Das System nach Anspruch 10 umfasst ferner einen Stopfen (2083), der in dem Versorgungslumen distal des Schlitzes (2081) befestigt ist.

**Revendications**

1. Un système d'aspiration de thrombus (2000), comprenant :
un cathéter d'aspiration (2018) comprenant :

une tige allongée (2042) configurée pour être placée dans un vaisseau sanguin d'un sujet ;
une lumière d'alimentation (2093) et une lumière d'aspiration (2032) s'étendant chacune à l'intérieur de la tige (2042) et séparées par une paroi (2079), dans laquelle la paroi (2079) forme un tube d'alimentation (2087) formant la lumière d'alimentation (2093) et une paroi intérieure (2085) de la tige allongée (2042) formant la lumière d'aspiration (2032), la lumière d'alimentation (2093) comportant une extrémité proximale et une extrémité distale, et la lumière d'aspiration (2032) comportant une extrémité proximale (2019) et une extrémité distale ouverte (2036), une partie du tube d'alimentation (2087) étant configurée de manière à se déplacer à l'intérieur de la lumière d'aspiration (2032) en raison de la force d'écoulement exercée sur une surface externe du tube d'alimentation (2087) de telle sorte qu'un espace luminal interne restant de la lumière d'aspiration (2032) s'auto-optimise pour l'écoulement du thrombus aspiré ; et une fente (2081) s'étendant de manière circonférentielle dans la paroi (2079) au niveau ou à proximité de l'extrémité distale de la lumière d'alimentation (2093), la fente (2081) étant configurée pour permettre l'injection d'un fluide sous pression dans la lumière d'aspiration (2032) au niveau ou à proximité de l'extrémité distale de la lumière d'aspiration (2032) lorsque le fluide sous pression est pompé à travers la lumière d'alimentation (2093).

2. Le système selon la revendication 1, dans lequel la fente a une largeur (I) orientée dans la direction de l'axe longitudinal du cathéter d'aspiration (2018) et une longueur d'arc (L) orientée de manière circonférentielle sur une surface extérieure de la structure tubulaire (2087).

3. Le système selon la revendication 2, dans lequel la largeur (1) est comprise entre environ 25,4 $\mu$m (0,0010 pouce) et environ 63,5 $\mu$m (0,0025 pouce).

4. Le système selon la revendication 3, dans lequel la largeur (1) est comprise entre environ 38,1 $\mu$m (0,0015 pouce) et environ 50,8 $\mu$m (0,0020 pouce).

5. Le système selon la revendication 2, dans lequel la longueur de l'arc (L) est comprise entre environ 50,8 $\mu$m (0,002 pouce) et environ 254 $\mu$m (0,010 pouce).

6. Le système selon la revendication 5, dans lequel la longueur de l'arc (L) est comprise entre environ 101,6 $\mu$m (6,004 pouces) et environ 203,2 $\mu$m (6,008 pouces).

7. Le système selon la revendication 6, dans lequel la longueur de l'arc (L) est comprise entre environ 127 $\mu$m (0,005 pouce) et environ 177,8 $\mu$m (0,007 pouce).

8. Le système selon la revendication 1, comprenant en outre un connecteur (2010) couplé hydrauliquement à l'extrémité proximale de la lumière d'aspiration (2032), le connecteur (2010) comportant une cavité intérieure (2087), une extrémité proximale (2073) et une extrémité distale (2075).

9. Le système selon la revendication 1, dans lequel la fente (2081) crée un modèle de pulvérisation (2077) du fluide sous pression sur un secteur d'une paroi intérieure (2085) de la lumière d'aspiration (2032).

10. Le système selon la revendication 9, dans lequel le secteur de la paroi intérieure (2085) comprend des parties de la paroi intérieure opposées à la fente (2081) et proximales et distales par rapport à la fente (2081).

11. Le système selon la revendication 10, dans lequel le tube d'alimentation (2087) est disposé à l'intérieur de la lumière d'aspiration (2032).

12. Le système selon la revendication 10, dans lequel l'extrémité distale de la lumière d'alimentation (2093) est disposée de manière proximale par rapport à l'extrémité distale (2036) de la lumière d'aspiration (2032).

13. Le système selon la revendication 10, comprenant en outre un bouchon (2083) fixé à l'intérieur de la lumière d'alimentation de manière distale par rapport à la fente (2081).

FIG.1

FIG. 2

EP 4 056 128 B1

FIG. 3

FIG. 4

FIG. 5

FIG. 8

FIG. 6

FIG. 7

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

FIG. 22

FIG. 23

EP 4 056 128 B1

FIG. 24

FIG. 25

FIG. 26

FIG. 27

FIG. 29

FIG. 31

FIG. 28

FIG. 30

FIG. 32

FIG.33

FIG. 34

FIG. 35

EP 4 056 128 B1

FIG. 36

FIG. 37

FIG. 38

FIG. 39

**FIG. 40**

**FIG. 41**

FIG. 42

FIG. 43

**FIG. 44A**

**FIG. 44B**

**FIG. 45A**

**FIG. 45B**

**FIG. 46A**

**FIG. 46B**

FIG. 47

FIG. 48

FIG. 49

FIG. 50

FIG. 51

FIG. 52

*FIG. 53*

*FIG. 54*

*FIG. 55*

**FIG. 56**

**FIG. 57**

**FIG. 58**

**FIG. 59A**

**FIG. 59B**

FIG. 60

**FIG. 61**

**FIG. 62**

```
              ┌─────────────────────┐
   1952 ──────│  INSERT CATHETER    │
              └─────────────────────┘
                         │
              ┌─────────────────────┐
              │  COUPLE VACUUM      │
   1954 ──────│  SOURCE             │
              └─────────────────────┘
                         │
              ┌─────────────────────┐
              │  COUPLE FLUID       │
   1956 ──────│  SOURCE             │
              └─────────────────────┘
                         │
              ┌─────────────────────┐
              │  TURN PUMP ON       │
   1958 ──────│                     │
              └─────────────────────┘
                         │
              ┌─────────────────────┐
              │  CHANGE             │
              │  CONFIGURATION      │
   1960 ──────│  OF VALVE           │
              └─────────────────────┘
                         │
              ┌─────────────────────┐
              │  RETURN TO ORIGINAL │
              │  CONFIGURATION      │
   1962 ──────│  OF VALVE           │
              └─────────────────────┘
```

# FIG. 63

**FIG. 64**

**FIG. 65**

**FIG. 66**

FIG. 67

FIG. 68

FIG. 69

FIG. 70

FIG. 71

**FIG. 72**

EP 4 056 128 B1

FIG. 73

**FIG. 74**

EP 4 056 128 B1

FIG. 75

**FIG. 76**

**FIG. 77**

**FIG. 78**

FIG. 79

FIG. 80

FIG. 81

## REFERENCES CITED IN THE DESCRIPTION

**Patent documents cited in the description**

- US 2015327875 A1
- US 2006229550 A1
- US 20120330196